# EUROPEAN PATENT SPECIFICATION

(11) **EP 4 405 043 B1**
(45) Date of publication and mention of the grant of the patent: **16.09.2026**
(21) Application number: 22786801.5
(22) Date of filing: 22.09.2022
(51) Int. Cl.: A61P 15/00, A61P 25/00, A61P 25/08, A61P 25/16, A61P 25/18, A61P 25/22, A61P 25/24, A61P 25/28, A61P 29/00, C07D 487/04

(54) **BENZODIAZEPINE DERIVATIVES AS GABA A GAMMA1 PAM**
BENZODIAZEPINDERIVATE ALS GABA A GAMMA1 PAM
DÉRIVÉS DE BENZODIAZÉPINE EN TANT QUE PAM GAMMA1 GABA A

(30) Priority: 24.09.2021 EP 21198723
(43) Date of publication of application: 31.07.2024
(73) Proprietor: F. Hoffmann-La Roche AG, 4070 Basel (CH)
(72) Inventor: CECERE, Giuseppe, 4070 Basel (CH); GOBBI, Luca, 4070 Basel (CH); HANLON, Steven Paul, 4070 Basel (CH); HERNANDEZ, Maria-Clemencia, 4070 Basel (CH); HUMM, Roland, 4070 Basel (CH); KOBLET, Andreas, 4070 Basel (CH); OLIVARES MORALES, Andrés Miguel, 4070 Basel (CH); RUNTZ-SCHMITT, Valerie, 4070 Basel (CH)
(74) Representative: Graham Watt & Co
(86) International application number: PCT/EP2022/076323
(87) International publication number: WO 2023/046817

(56) References cited:
- LEE S ET AL: "Axial chirality and affinity at the GABA"A receptor of pyrimido[1,2-a][1,4]benzodiazepines and related compounds", BIOORGANIC, ELSEVIER, AMSTERDAM, NL, vol. 16, no. 21, 1 November 2008 (2008-11-01), pages 9519 - 9523, XP025545777, ISSN: 0968-0896, [retrieved on 20080917], DOI: 10.1016/J.BMC.2008.09.037

## Description

### Field of the Invention

The present invention relates to organic compounds useful for therapy or prophylaxis in a mammal, and in particular to new benzodiazepine derivatives that exhibit activity as GABAA γ1 receptor positive allosteric modulators (PAMs) and are thus useful for the treatment or prophylaxis of GABAA γ1 receptor related diseases or conditions.

### Background of the Invention

Receptors for the major inhibitory neurotransmitter, gamma-aminobutyric acid (GABA), are divided into two main classes: (1) GABA_{A} receptors, which are members of the ligand-gated ion channel superfamily and (2) GABA_{B} receptors, which are members of the G-protein linked receptor family. The GABA_{A} receptor complex which is a membrane-bound heteropentameric protein polymer is composed principally of α, β and γ subunits. GABA_{A} receptors are ligand-gated chloride channels and the principal mediators of inhibitory neurotransmission in the human brain.

There are 19 genes encoding for GABA_{A} receptor subunits that assemble as pentamers with the most common stoichiometry being two α, two β and one γ subunit. GABA_{A} subunit combinations give rise to functional, circuit, and behavioral specificity. GABA_{A} receptors containing the γ1 subunit (GABA_{A} γ1) are of particular interest due to their enriched expression in the limbic system and unique physiological and pharmacological properties. The GABA_{A} γ1 subunit-containing receptors, while less abundant (around 5-10 % of total expression of GABA_{A} receptors in the brain) than γ2 subunit-containing receptors exhibit an enriched brain mRNA and protein distribution in key brain areas such as extended amygdala (central, medial, and bed nucleus of the stria terminalis), lateral septum, hypothalamus, and pallidum/nigra. These structures form the interconnected core of a subcortical limbic circuit regulating motivated social and affective behaviors. In abnormal or disease conditions, hyper-recruitment of this circuit promotes anxiety, arousal, aggression, fear and defense while inhibiting foraging and social interactions.

Hyperactivity in limbic cortical regions (known to form a coordinated functional network with extended amygdala/ hypothalamus regions) which are key areas for processing of social and emotionally relevant stimuli, is the common hallmark of a variety of psychiatric, neurological, neurodevelopmental, neurodegenerative, mood, motivational and metabolic disorders. In such a disease state, and given the characteristic anatomical distribution of the γ1 subunit-containing GABA_{A} receptors, a GABA_{A} γ1 positive allosteric modulator (PAM) may be an effective treatment as a symptomatic or disease-modifying agent.

Multiple lines of evidence suggest that an imbalance between excitatory/inhibitory (E/I) neurotransmission arising from dysfunction of GABAergic signaling system, the main inhibitory neurotransmitter system in the brain, to be at the core of the pathogenesis a variety of CNS disorders. Given the distribution and function of GABA_{A} γ1 subunit-containing receptors in the CNS, they are very attractive targets for restoring levels of inhibition within key brain circuits and consequently the E/I balance in these conditions.

A CNS disorders of particular interest in the context of the present invention is autism spectrum disorder (ASD), including its core symptoms and associated comorbidities, such as anxiety and irritability, social anxiety disorder (social phobia) and generalized anxiety disorder. ASD is a complex, heterogeneous neurodevelopmental disorder characterized by impairments in two core domains: impairments in social interaction and communication, and presence of repetitive or restricted behaviors, interests, or activities (American Psychiatric Association 2013).

No approved pharmacological treatment exists for core symptoms of social deficits and restricted/repetitive behaviour of ASD, while only inadequate therapeutic options are available for most of ASD's affective and physiological co-morbidities. As a result, this disorder continues to be an area of high unmet medical need. Current approved treatments for associated symptoms of ASD are limited to the antipsychotics (Risperidone and Aripiprazole) indicated for the treatment of irritability associated with ASD symptoms. Emerging evidence suggests that the GABAergic system, the main inhibitory neurotransmitter system in the brain, plays a key role in the pathophysiology of ASD.

Both genetic and imaging studies using positron emission tomography study (PET) and magnetic resonance spectroscopy (MRS) suggest alterations in GABAergic signaling in ASD. The gene encoding GABA_{A} γ1, GABRG1, is located on chromosome 4 (mouse Chr.5) in a cluster with genes encoding α2, α4 and β1 GABA_{A} receptor subunits. Rare CNVs, including inversion of chromosome 4p12 disrupting GABRG1 have been observed in autistic siblings (Horike et al., 2006), as well as GABRG1 loss in one case of ADHD. Mutations in 4p12 gene cluster have been linked to increased risk of anxiety, substance abuse and eating disorders - providing a link between GABRG1/4p12 and affective dysfunction. MRS studies found altered GABA levels in ASD and in particular some recent studies showed reduced GABA and altered somatosensory function in children with ASD. In line with these observations, a reduced number of inhibitory interneurons were found from postmortem tissues of ASD and TS patients. Furthermore, reduced GABA synthesizing enzymes, glutamic acid decarboxylase (GAD) 65 and 67 were found in parietal and cerebellar cortices of patients with autism. Strong evidence in humans points to specific dysfunction in ASD of the limbic cortical regions known to form a coordinated functional network with GABA_{A} γ1 subunit-containing extended amygdala/ hypothalamus regions. These areas: Cortical/lateral amygdala, Insula, PFC, and Cingulate are recognized key for processing of social and emotionally relevant stimuli. While subcortical subnuclei that form specific partnerships with these areas, coordinating behavioural outcomes, are often difficult to study due to spatial resolution limitations, many lines of evidence point to hyper-recruitment of these cortical- to sub cortical connections in ASD. Moreover, recent high resolution studies provide a clear link between extended amygdala activity /functional connectivity and emotional state. Targeting such highly specified limbic subcortical regions, which exhibit substantial molecular and cellular diversity compared to the neocortex, will create a precision entry point for safe and specific therapeutic modulation of ASD-affected socio-affective circuits, while avoiding broad modulation of global brain state. Enhancement of GABA_{A} receptor activity by non-selective BZDs have been shown to ameliorate behavioral deficits in mouse models of ASD, however very narrow therapeutic margins were observed due to sedation mediated by the GABA_{A} α1γ2 subtype. These findings support the notion that rebalancing of GABAergic transmission *via* GABA_{A} γ1 receptors can improve symptoms in ASD without the side effects of non-selective benzodiazepines.

Lee et al., Bioorg. Med. Chem. 2008, 16, 9519-9523 discusses the axial chirality and affinity at the GABAA receptor of pyrimido[1,2-a][1,4]benzodiazepines and related compounds.

Compounds of the present invention are selective GABA_{A} γ1 receptor positive allosteric modulators (PAMs) that selectively enhance the function of γ1-containing GABA_{A} receptors by increasing GABAergic currents (influx of chloride) at a given concentration *(e.g.* EC₂₀) of gamma amino butyric acid (GABA). The compounds of the present invention have high PAM efficacy and binding selectivity for the γ1-containing subtypes (α5γ1, α2γ1, α1γ1) relative to the γ2-containing subtypes (e.g. α1γ2, α2γ2, α3γ2 and α5γ2). As such, compounds of the present invention are strongly differentiated from classical benzodiazepine drugs such as Alprazolam, Triazolam, Estazolam, and Midazolam, which are selective for the γ2-containing GABA_{A} subtypes and possess low affinity for the γ1-containing subtypes. Compatible with the γ1-subtypes brain distribution, selective GABA_{A} γ1 PAMs will restore GABAergic signaling in key brain regions (e.g. extended amygdala: central, medial, and bed nucleus of the stria terminalis, lateral septum, hypothalamus, and pallidum/nigra) without the side-effects of non-selective GABA_{A} modulators (e.g. benzodiazepines).

In view of the above, the selective GABA_{A} γ1 PAMs described herein and their pharmaceutically acceptable salts and esters are useful, alone or in combination with other drugs, as disease-modifying or as symptomatic agents for the treatment or prevention of acute neurological disorders, chronic neurological disorders and/or cognitive disorders, including autism spectrum disorders (ASD), Angelman syndrome, age-related cognitive decline, Rett syndrome, Prader-Willi syndrome, amyotrophic lateral sclerosis (ALS), fragile-X disorder, negative and/or cognitive symptoms associated with schizophrenia, tardive dyskinesia, anxiety, social anxiety disorder (social phobia), panic disorder, agoraphobia, generalized anxiety disorder, disruptive, impulse-control and conduct disorders, Tourette's syndrome (TS), obsessive-compulsive disorder (OCD), acute stress disorder, post-traumatic stress disorder (PTSD), attention deficit hyperactivity disorder (ADHD), sleep disorders, Parkinson's disease (PD), Huntington's chorea, Alzheimer's disease (AD), mild cognitive impairment (MCI), dementia, behavioral and psychological symptoms (BPS) in neurodegenerative conditions, multi-infarct dementia, agitation, psychosis, substance-induced psychotic disorder, aggression, eating disorders, depression, chronic apathy, anhedonia, chronic fatigue, seasonal affective disorder, postpartum depression, drowsiness, sexual dysfunction, bipolar disorders, epilepsy and pain.

### Summary of the Invention

In a first aspect, the present invention provides a compound of formula (I) 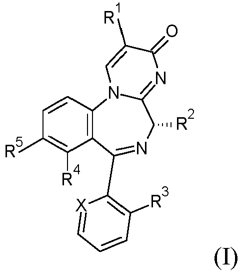 or a pharmaceutically acceptable salt thereof, wherein R¹ to R⁵ and X are as defined herein.

In one aspect, the present invention provides a process of manufacturing the compounds of formula (I) described herein, wherein said process is as described in any one of Schemes 1 to 5 herein.

In a further aspect, the present invention provides a compound of formula (I) as described herein, when manufactured according to the processes described herein.

In a further aspect, the present invention provides a compound of formula (I) as described herein, or a pharmaceutically acceptable salt thereof, for use as therapeutically active substance.

In a further aspect, the present invention provides a pharmaceutical composition comprising a compound of formula (I) as described herein, or a pharmaceutically acceptable salt thereof, and a therapeutically inert carrier.

In a further aspect, the present invention provides a compound of formula (I) as described herein, or a pharmaceutically acceptable salt thereof, for use in a method for treating or preventing acute neurological disorders, chronic neurological disorders and/or cognitive disorders in a subject.

### Detailed Description of the Invention

### Definitions

Features, integers, characteristics, compounds, chemical moieties or groups described in conjunction with a particular aspect, embodiment or example of the invention are to be understood to be applicable to any other aspect, embodiment or example described herein, unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

The term "alkyl" refers to a mono- or multivalent, e.g., a mono- or bivalent, linear or branched saturated hydrocarbon group of 1 to 6 carbon atoms ("C₁-C₆-alkyl"), e.g., 1, 2, 3, 4, 5, or 6 carbon atoms. In some embodiments, the alkyl group contains 1 to 3 carbon atoms, e.g., 1, 2 or 3 carbon atoms. Some non-limiting examples of alkyl include methyl, ethyl, propyl, 2-propyl (isopropyl), n-butyl, iso-butyl, sec-butyl, tert-butyl, and 2,2-dimethylpropyl. Particularly preferred, yet non-limiting examples of alkyl include methyl and ethyl.

The term "alkenyl" denotes a monovalent linear or branched hydrocarbon group of 2 to 6 carbon atoms with at least one double bond ("C₂-C₆-alkenyl"). In particular embodiments, alkenyl has 2 to 4 carbon atoms with at least one double bond. Examples of alkenyl include ethenyl, propenyl, prop-2-enyl, isopropenyl, n-butenyl and iso-butenyl. Particular alkenyl group is ethenyl.

The term "alkoxy" refers to an alkyl group, as previously defined, attached to the parent molecular moiety via an oxygen atom. Unless otherwise specified, the alkoxy group contains 1 to 6 carbon atoms ("C₁-C₆-alkoxy"). In some preferred embodiments, the alkoxy group contains contains 1 to 4 carbon atoms. In still other embodiments, the alkoxy group contains 1 to 3 carbon atoms. Some non-limiting examples of alkoxy groups include methoxy, ethoxy, n-propoxy, isopropoxy, n-butoxy, isobutoxy and tert-butoxy. A particularly preferred, yet non-limiting example of alkoxy is methoxy.

The term "halogen" or "halo" refers to fluoro (F), chloro (Cl), bromo (Br), or iodo (I). Preferably, the term "halogen" or "halo" refers to fluoro (F), chloro (Cl) or bromo (Br). Particularly preferred, yet non-limiting examples of "halogen" or "halo" are fluoro (F) and chloro (Cl).

The term "cycloalkyl" as used herein refers to a saturated or partly unsaturated monocyclic or bicyclic hydrocarbon group of 3 to 10 ring carbon atoms ("C₃-C₁₀-cycloalkyl"). In some preferred embodiments, the cycloalkyl group is a saturated monocyclic hydrocarbon group of 3 to 8 ring carbon atoms. "Bicyclic cycloalkyl" refers to cycloalkyl moieties consisting of two saturated carbocycles having two carbon atoms in common, i.e., the bridge separating the two rings is either a single bond or a chain of one or two ring atoms, and to spirocyclic moieties, i.e., the two rings are connected via one common ring atom. Preferably, the cycloalkyl group is a saturated monocyclic hydrocarbon group of 3 to 6 ring carbon atoms, e.g., of 3, 4, 5 or 6 carbon atoms. Some non-limiting examples of cycloalkyl include cyclopropyl, cyclobutyl, cyclopentyl, cyclohexyl, cycloheptyl, and spiro[2.3]hexan-5-yl.

The term "aminoalkyl" refers to an alkyl group, wherein at least one of the hydrogen atoms of the alkyl group has been replaced by an amino group. Preferably, "aminoalkyl" refers to an alkyl group wherein 1, 2 or 3 hydrogen atoms of the alkyl group have been replaced by an amino group. Preferred, yet non-limiting examples of aminoalkyl are aminomethyl and 1-aminoethyl.

The term "hydroxy" refers to an -OH group.

The term "amino" refers to an -NH₂ group.

The term "cyano" refers to a -CN (nitrile) group.

The term "carbamoyl" refers to a -C(O)NH₂ group.

The term "haloalkyl" refers to an alkyl group, wherein at least one of the hydrogen atoms of the alkyl group has been replaced by a halogen atom, preferably fluoro. Preferably, "haloalkyl" refers to an alkyl group wherein 1, 2 or 3 hydrogen atoms of the alkyl group have been replaced by a halogen atom, most preferably fluoro. Non-limiting examples of haloalkyl are fluoromethyl, difluoromethyl, trifluoromethyl, trifluoroethyl, 2-fluoroethyl, and 2,2-difluoroethyl. A particularly preferred, yet non-limiting example of haloalkyl is trifluoromethyl.

The term "alkoxyalkyl" refers to an alkyl group, wherein at least one of the hydrogen atoms of the alkyl group has been replaced by an alkoxy group. Preferably, "alkoxyalkyl" refers to an alkyl group wherein 1, 2 or 3 hydrogen atoms of the alkyl group have been replaced by an alkoxy group. Most preferably, "alkoxyalkyl" refers to an alkyl group wherein 1 hydrogen atom of the alkyl group has been replaced by an alkoxy group. A preferred, yet non-limiting example of alkoxyalkyl is 2-methoxyethyl.

The term "alkoxyalkenyl" refers to an alkenyl group, wherein at least one of the hydrogen atoms of the alkenyl group has been replaced by an alkoxy group. Preferably, "alkoxyalkenyl" refers to an alkenyl group wherein 1, 2 or 3 hydrogen atoms of the alkenyl group have been replaced by an alkoxy group. Most preferably, "alkoxyalkenyl" refers to an alkenyl group wherein 1 hydrogen atom of the alkenyl group has been replaced by an alkoxy group. A preferred, yet non-limiting example of alkoxyalkenyl is 2-ethoxyvinyl.

The term "haloalkoxy" refers to an alkoxy group, wherein at least one of the hydrogen atoms of the alkoxy group has been replaced by a halogen atom, preferably fluoro. Preferably, "haloalkoxy" refers to an alkoxy group wherein 1, 2 or 3 hydrogen atoms of the alkoxy group have been replaced by a halogen atom, most preferably fluoro. Particularly preferred, yet non-limiting examples of haloalkoxy are fluoromethoxy (FCH₂O-), difluoromethoxy (F₂CHO-), and trifluoromethoxy (F₃CO-).

The term "hydroxyalkyl" refers to an alkyl group, wherein at least one of the hydrogen atoms of the alkyl group has been replaced by a hydroxy group. Preferably, "hydroxyalkyl" refers to an alkyl group wherein 1, 2 or 3 hydrogen atoms, most preferably 1 hydrogen atom of the alkyl group have been replaced by a hydroxy group. Preferred, yet non-limiting examples of hydroxyalkyl are hydroxymethyl, hydroxyethyl (e.g. 2-hydroxyethyl), and 3-hydroxy-3-methyl-butyl.

The term "pharmaceutically acceptable salt" refers to those salts which retain the biological effectiveness and properties of the free bases or free acids, which are not biologically or otherwise undesirable. The salts are formed with inorganic acids such as hydrochloric acid, hydrobromic acid, sulfuric acid, nitric acid, phosphoric acid and the like, in particular hydrochloric acid, and organic acids such as formic acid, acetic acid, trifluoroacetic acid, propionic acid, glycolic acid, pyruvic acid, oxalic acid, maleic acid, malonic acid, succinic acid, fumaric acid, tartaric acid, lactic acid, citric acid, benzoic acid, cinnamic acid, mandelic acid, methanesulfonic acid, ethanesulfonic acid, p-toluenesulfonic acid, salicylic acid, N-acetylcystein and the like. In addition these salts may be prepared by addition of an inorganic base or an organic base to the free acid. Salts derived from an inorganic base include, but are not limited to, the sodium, potassium, lithium, ammonium, calcium, magnesium salts and the like. Salts derived from organic bases include, but are not limited to salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, lysine, arginine, N-ethylpiperidine, piperidine, polyimine resins and the like. Particular pharmaceutically acceptable salts of compounds of formula (I) are hydrochlorides, fumarates, formates, lactates (in particular derived from L-(+)-lactic acid), tartrates (in particular derived from L-(+)-tartaric acid) and trifluoroacetates.

The compounds of formula (I) can contain several asymmetric centers and can be present in the form of optically pure enantiomers, mixtures of enantiomers such as, for example, racemates, optically pure diastereioisomers, mixtures of diastereoisomers, diastereoisomeric racemates or mixtures of diastereoisomeric racemates.

According to the Cahn-Ingold-Prelog Convention, the asymmetric carbon atom can be of the "R" or "S" configuration.

The term "treatment" as used herein includes: (1) inhibiting the state, disorder or condition (e.g. arresting, reducing or delaying the development of the disease, or a relapse thereof in case of maintenance treatment, of at least one clinical or subclinical symptom thereof); and/or (2) relieving the condition (i.e., causing regression of the state, disorder or condition or at least one of its clinical or subclinical symptoms). The benefit to a patient to be treated is either statistically significant or at least perceptible to the patient or to the physician. However, it will be appreciated that when a medicament is administered to a patient to treat a disease, the outcome may not always be effective treatment.

The term "prophylaxis" or "prevention" as used herein includes: preventing or delaying the appearance of clinical symptoms of the state, disorder or condition developing in a subject and especially a human that may be afflicted with or predisposed to the state, disorder or condition but does not yet experience or display clinical or subclinical symptoms of the state, disorder or condition.

The term "subject" as used herein includes both humans and non-humans and includes but is not limited to humans, non-human primates, canines, felines, murines, bovines, equines, and porcines. In a particularly preferred embodiment, the term "subject" refers to humans.

The term "protecting group" (PG) denotes a group which selectively blocks a reactive site in a multifunctional compound such that a chemical reaction can be carried out selectively at another unprotected reactive site in the meaning conventionally associated with it in synthetic chemistry. Protecting groups can be removed at the appropriate point. Exemplary protecting groups are amino-protecting groups, carboxy-protecting groups or hydroxy-protecting groups. Particular protecting groups are the tert-butoxycarbonyl (Boc), benzyloxycarbonyl (Cbz), fluorenylmethoxycarbonyl (Fmoc) and benzyl (Bn) groups. Further particular protecting groups are the tert-butoxycarbonyl (Boc) and the fluorenylmethoxycarbonyl (Fmoc) groups. More particular protecting group is the *tert-*butoxycarbonyl (Boc) group.

The abbreviation uM means microMolar and is equivalent to the symbol µM.

The abbreviation uL means microliter and is equivalent to the symbol µL.

The abbreviation ug means microgram and is equivalent to the symbol µg.

### Compounds of the Invention

In a first aspect, the present invention provides a compound of formula (I) 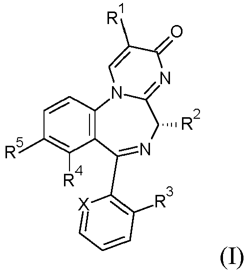 or a pharmaceutically acceptable salt thereof, wherein:
- X: is C-R⁶ or N;
- R¹: is selected from hydrogen, halogen, cyano, C₁-C₆-alkyl, halo-C₁-C₆-alkyl, hydroxy-C₁-C₆-alkyl, amino-C₁-C₆-alkyl, C₁-C₆-alkoxy-C₁-C₆-alkyl, C₁-C₆-alkoxy-C₂-C₆-alkenyl, C₁-C₆-alkoxy, halo-C₁-C₆-alkoxy, carbamoyl, C₁-C₆-alkyl-NH-C(O)-, (C₁-C₆-alkyl)₂N-C(O)-, and C₃-C₁₀-cycloalkyl-NH-C(O)-;
- R²: is selected from hydrogen, C₁-C₆-alkyl, halo-C₁-C₆-alkyl, C₁-C₆-alkoxy, and halo-C₁-C₆-alkoxy;
- R³ and R⁶: are each independently selected from hydrogen, C₁-C₆-alkyl, halo-C₁-C₆-alkyl, C₁-C₆-alkoxy, halo-C₁-C₆-alkoxy, halogen and cyano;
- R⁴: is selected from chloro and bromo; and
- R⁵: is selected from hydrogen, C₁-C₆-alkyl, halo-C₁-C₆-alkyl, C₁-C₆-alkoxy, halo-C₁-C₆-alkoxy, halogen and cyano.

In a particularly preferred embodiment, the present invention provides a compound of formula (I) as described herein, or a pharmaceutically acceptable salt thereof, wherein X is C-R⁶.

In one embodiment, the present invention provides a compound of formula (I) as described herein, wherein R¹ is selected from hydrogen, halogen, C₁-C₆-alkyl, hydroxy-C₁-C₆-alkyl, C₁-C₆-alkoxy-C₁-C₆-alkyl, C₁-C₆-alkoxy-C₂-C₆-alkenyl, C₁-C₆-alkyl-NH-C(O)-, and C₃-C₁₀-cycloalkyl-NH-C(O)-.

In a preferred embodiment, the present invention provides a compound of formula (I) as described herein, or a pharmaceutically acceptable salt thereof, wherein R¹ is selected from C₁-C₆-alkyl and C₁-C₆-alkyl-NH-C(O)-.

In a particularly preferred embodiment, the present invention provides a compound of formula (I) as described herein, or a pharmaceutically acceptable salt thereof, wherein R¹ is selected from methyl and methyl-NH-C(O)-.

In one embodiment, the present invention provides a compound of formula (I) as described herein, or a pharmaceutically acceptable salt thereof, wherein R² is selected from hydrogen and C₁-C₆-alkyl.

In a preferred embodiment, the present invention provides a compound of formula (I) as described herein, or a pharmaceutically acceptable salt thereof, wherein R² is C₁-C₆-alkyl.

In a particularly preferred embodiment, the present invention provides a compound of formula (I) as described herein, or a pharmaceutically acceptable salt thereof, wherein R² is methyl.

In a preferred embodiment, the present invention provides a compound of formula (I) as described herein, or a pharmaceutically acceptable salt thereof, wherein R³ is halogen.

In a particularly preferred embodiment, the present invention provides a compound of formula (I) as described herein, or a pharmaceutically acceptable salt thereof, wherein R³ is fluoro.

In a particularly preferred embodiment, the present invention provides a compound of formula (I) as described herein, or a pharmaceutically acceptable salt thereof, wherein R⁴ is chloro.

In one embodiment, the present invention provides a compound of formula (I) as described herein, or a pharmaceutically acceptable salt thereof, wherein R⁵ is selected from halogen, C₁-C₆-alkyl and halo-C₁-C₆-alkyl.

In a preferred embodiment, the present invention provides a compound of formula (I) as described herein, or a pharmaceutically acceptable salt thereof, wherein R⁵ is halo-C₁-C₆-alkyl.

In a particularly preferred embodiment, the present invention provides a compound of formula (I) as described herein, or a pharmaceutically acceptable salt thereof, wherein R⁵ is CF₃.

In one embodiment, the present invention provides a compound of formula (I) as described herein, or a pharmaceutically acceptable salt thereof, wherein R⁶ is selected from hydrogen and halogen.

In a preferred embodiment, the present invention provides a compound of formula (I) as described herein, or a pharmaceutically acceptable salt thereof, wherein R⁶ is halogen.

In a particularly preferred embodiment, the present invention provides a compound of formula (I) as described herein, or a pharmaceutically acceptable salt thereof, wherein R⁶ is fluoro.

In one embodiment, the present invention provides a compound of formula (I) as described herein, or a pharmaceutically acceptable salt thereof, wherein:
- R¹: is selected from hydrogen, halogen, C₁-C₆-alkyl, hydroxy-C₁-C₆-alkyl, C₁-C₆-alkoxy-C₁-C₆-alkyl, C₁-C₆-alkoxy-C₂-C₆-alkenyl, C₁-C₆-alkyl-NH-C(O)-, and C₃-C₁₀-cycloalkyl-NH-C(O)-;
- R²: is selected from hydrogen and C₁-C₆-alkyl;
- R³: is halogen;
- R⁵: is selected from halogen, C₁-C₆-alkyl and halo-C₁-C₆-alkyl; and
- R⁶: is selected from hydrogen and halogen.

In a preferred embodiment, the present invention provides a compound of formula (I) as described herein, or a pharmaceutically acceptable salt thereof, wherein:
- R¹: is selected from C₁-C₆-alkyl and C₁-C₆-alkyl-NH-C(O)-;
- R²: is C₁-C₆-alkyl;
- R³ and R⁶: are both halogen;
- R⁴: is chloro; and
- R⁵: is halo-C₁-C₆-alkyl.

In a particularly preferred embodiment, the present invention provides a compound of formula (I) as described herein, or a pharmaceutically acceptable salt thereof, wherein:
- R¹: is selected from methyl and methyl-NH-C(O)-;
- R²: is methyl;
- R³ and R⁶: are both fluoro;
- R⁴: is chloro; and
- R⁵: is CF₃.

In one embodiment, the present invention provides a compound of formula (I) as described herein, or a pharmaceutically acceptable salt thereof, wherein said compound of formula (I) is selected from:
8,9-dichloro-7-(2-fluorophenyl)-5H-pyrimido[1,2-a][1,4]benzodiazepin-3-one;
8,9-dichloro-7-(2,6-difluorophenyl)-5H-pyrimido[1,2-a][1,4]benzodiazepin-3-one;
8,9-dichloro-7-(3-fluoro-2-pyridyl)-5H-pyrimido[1,2-a][1,4]benzodiazepin-3-one;
(5S)-8,9-dichloro-7-(2,6-difluorophenyl)-5-methyl-5H-pyrimido[1,2-a][1,4]benzodiazepin-3-one;
2-bromo-8,9-dichloro-7-(2,6-difluorophenyl)-5H-pyrimido[1,2-a][1,4]benzodiazepin-3-one;
(5S)-8-bromo-9-chloro-7-(2,6-difluorophenyl)-5-methyl-5H-pyrimido[1,2-a][1,4]benzodiazepin-3-one;
(5S)-8,9-dichloro-7-(2,6-difluorophenyl)-2,5-dimethyl-5H-pyrimido[1,2-a][1,4]benzodiazepin-3-one;
(5S)-8-chloro-7-(2,6-difluorophenyl)-2,5,9-trimethyl-5H-pyrimido[1,2-a][1,4]benzodiazepin-3-one;
(5S)-8-chloro-7-(2,6-difluorophenyl)-2,5-dimethyl-9-(trifluoromethyl)-5H-pyrimido[1,2-a][1,4]benzodiazepin-3-one;
(5S)-8-chloro-7-(2,6-difluorophenyl)-5-methyl-9-(trifluoromethyl)-5H-pyrimido[1,2-a][1,4]benzodiazepin-3-one;
(5S)-8-chloro-7-(2,6-difluorophenyl)-2-ethyl-5-methyl-9-(trifluoromethyl)-5H-pyrimido[1,2-a][1,4]benzodiazepin-3-one;
9-bromo-8-chloro-7-(2,6-difluorophenyl)-5H-pyrimido[1,2-a][1,4]benzodiazepin-3-one;
(5S)-9-bromo-8-chloro-7-(2,6-difluorophenyl)-5-methyl-5H-pyrimido[1,2-a][1,4]benzodiazepin-3-one;
(5S)-8,9-dichloro-7-(2,6-difluorophenyl)-2-ethyl-5-methyl-5H-pyrimido[1,2-a][1,4]benzodiazepin-3-one;
2,9-dibromo-8-chloro-7-(2,6-difluorophenyl)-5H-pyrimido[1,2-a][1,4]benzodiazepin-3-one;
(5S)-2,9-dibromo-8-chloro-7-(2,6-difluorophenyl)-5-methyl-5H-pyrimido[1,2-a][1,4]benzodiazepin-3-one;
8-bromo-9-chloro-7-(2,6-difluorophenyl)-5H-pyrimido[1,2-a][1,4]benzodiazepin-3-one;
(5S)-8-chloro-7-(2,6-difluorophenyl)-2-[(E)-2-ethoxyvinyl]-5-methyl-9-(trifluoromethyl)-5H-pyrimido[1,2-a][1,4]benzodiazepin-3-one;
(5S)-8,9-dichloro-7-(2,6-difluorophenyl)-2-[(E)-2-ethoxyvinyl]-5-methyl-5H-pyrimido[1,2-a][1,4]benzodiazepin-3-one;
(5S)-8-chloro-7-(2,6-difluorophenyl)-2-(2-ethoxyethyl)-5-methyl-9-(trifluoromethyl)-5H-pyrimido[1,2-a][1,4]benzodiazepin-3-one;
(5S)-8,9-dichloro-7-(2,6-difluorophenyl)-2-(2-ethoxyethyl)-5-methyl-5H-pyrimido[1,2-a][1,4]benzodiazepin-3-one;
8,9-dichloro-7-(2,6-difluorophenyl)-2-[(E)-2-ethoxyvinyl]-5H-pyrimido[1,2-a][1,4]benzodiazepin-3-one;
(5S)-2,8-dibromo-9-chloro-7-(2,6-difluorophenyl)-5-methyl-5H-pyrimido[1,2-a][1,4]benzodiazepin-3-one;
8,9-dichloro-7-(2,6-difluorophenyl)-2-(2-ethoxyethyl)-5H-pyrimido[1,2-a][1,4]benzodiazepin-3-one;
8-chloro-7-(2,6-difluorophenyl)-2-[(*E*)-2-ethoxyvinyl]-9-(trifluoromethyl)-5H-pyrimido[1,2-a][1,4]benzodiazepin-3-one;
8-chloro-7-(2,6-difluorophenyl)-2-(2-ethoxyethyl)-9-(trifluoromethyl)-5H-pyrimido[1,2-a][1,4]benzodiazepin-3-one;
(5*S*)-8-chloro-7-(2,6-difluorophenyl)-N,5-dimethyl-3-oxo-9-(trifluoromethyl)-5H-pyrimido[1,2-a][1,4]benzodiazepine-2-carboxamide;
(5S)-8-chloro-N-cyclopropyl-7-(2,6-difluorophenyl)-5-methyl-3-oxo-9-(trifluoromethyl)-5H-pyrimido[1,2-a][1,4]benzodiazepine-2-carboxamide; and
(5*S*)-8-chloro-7-(2,6-difluorophenyl)-2-(hydroxymethyl)-5-methyl-9-(trifluoromethyl)-5H-pyrimido[1,2-a][1,4]benzodiazepin-3-one.

In a preferred embodiment, the present invention provides a compound of formula (I) as described herein, or a pharmaceutically acceptable salt thereof, wherein said compound of formula (I) is selected from:
(5*S*)-8-chloro-7-(2,6-difluorophenyl)-2,5-dimethyl-9-(trifluoromethyl)-5H-pyrimido[1,2-a][1,4]benzodiazepin-3-one; and
(5S)-8-chloro-7-(2,6-difluorophenyl)-N,5-dimethyl-3-oxo-9-(trifluoromethyl)-5H-pyrimido[1,2-a][1,4]benzodiazepine-2-carboxamide.

In a particularly preferred embodiment, the present invention provides a compound of formula (I) as described herein, or a pharmaceutically acceptable salt thereof, wherein said compound of formula (I) is (5S)-8-chloro-7-(2,6-difluorophenyl)-2,5-dimethyl-9-(trifluoromethyl)-5H-pyrimido[1,2-a][1,4]benzodiazepin-3-one.

In a particularly preferred embodiment, the present invention provides a compound of formula (I) as described herein, or a pharmaceutically acceptable salt thereof, wherein said compound of formula (I) is (5S)-8-chloro-7-(2,6-difluorophenyl)-N,5-dimethyl-3-oxo-9-(trifluoromethyl)-5H-pyrimido[1,2-a][1,4]benzodiazepine-2-carboxamide.

In one embodiment, the present invention provides a compound of formula (I) as described herein, or a pharmaceutically acceptable salt thereof, wherein:
- R³: is halogen; and
- R⁶: is selected from hydrogen and halogen.

In a preferred embodiment, the present invention provides a compound of formula (I) as described herein, or a pharmaceutically acceptable salt thereof, wherein R³ and R⁶ are both halogen.

In a preferred embodiment, the present invention provides a compound of formula (I) as described herein, or a pharmaceutically acceptable salt thereof, wherein R³ and R⁶ are both fluoro.

In a preferred embodiment, the present invention provides a compound of formula (I) as described herein, or a pharmaceutically acceptable salt thereof, wherein:
- R⁴: is chloro; and
- R⁵: is halo-C₁-C₆-alkyl.

In a particularly preferred embodiment, the present invention provides a compound of formula (I) as described herein, or a pharmaceutically acceptable salt thereof, wherein:
- R⁴: is chloro; and
- R⁵: is CF₃.

In one embodiment, the present invention provides pharmaceutically acceptable salts of the compounds of formula (I) as described herein, especially pharmaceutically acceptable salts selected from hydrochlorides, fumarates, lactates (in particular derived from L-(+)-lactic acid), tartrates (in particular derived from L-(+)-tartaric acid) and trifluoroacetates.

In yet a further particular embodiment, the present invention provides compounds according to formula (I) as described herein (i.e., as "free bases" or "free acids", respectively).

In some embodiments, the compounds of formula (I) are isotopically-labeled by having one or more atoms therein replaced by an atom having a different atomic mass or mass number. Such isotopically-labeled (i.e., radiolabeled) compounds of formula (I) are considered to be within the scope of this disclosure. Examples of isotopes that can be incorporated into the compounds of formula (I) include isotopes of hydrogen, carbon, nitrogen, oxygen, phosphorous, sulfur, fluorine, chlorine, and iodine, such as, but not limited to, ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ³⁶Cl, ¹²³I, and ¹²⁵I, respectively. Certain isotopically-labeled compounds of formula (I), for example, those incorporating a radioactive isotope, are useful in drug and/or substrate tissue distribution studies. The radioactive isotopes tritium, i.e. ³H, and carbon-14, i.e., ¹⁴C, are particularly useful for this purpose in view of their ease of incorporation and ready means of detection. For example, a compound of formula (I) can be enriched with 1, 2, 5, 10, 25, 50, 75, 90, 95, or 99 percent of a given isotope.

Substitution with heavier isotopes such as deuterium, i.e. ²H, may afford certain therapeutic advantages resulting from greater metabolic stability, for example, increased *in vivo* half-life or reduced dosage requirements.

Substitution with positron emitting isotopes, such as ¹¹C, ¹⁸F, ¹⁵O and ¹³N, can be useful in Positron Emission Topography (PET) studies for examining substrate receptor occupancy. Isotopically-labeled compounds of formula (I) can generally be prepared by conventional techniques known to those skilled in the art or by processes analogous to those described in the Examples as set out below using an appropriate isotopically-labeled reagent in place of the non-labeled reagent previously employed.

### Processes of Manufacturing

Processes for the manufacture of the compound of formula (I) as described herein are also an object of the invention.

The preparation of compounds of formula (I) of the present invention may be carried out in sequential or convergent synthetic routes. Syntheses of the compounds of the invention are shown in the following schemes. The skills required for carrying out the reactions and purifications of the resulting products are known to those skilled in the art. The substituents and indices used in the following description of the processes have the significance given herein before and in the claims, unless indicated to the contrary. In more detail, the compounds of formula (I) can be manufactured by the methods given below, by the methods given in the examples or by analogous methods. Appropriate reaction conditions for the individual reaction steps are known to a person skilled in the art. Also, for reaction conditions described in literature affecting the described reactions see for example: Comprehensive Organic Transformations: A Guide to Functional Group Preparations, 3rd Edition, Richard C. Larock. John Wiley & Sons, New York, NY. 2018*).* It is convenient to carry out the reactions in the presence or absence of a solvent. There is no particular restriction on the nature of the solvent to be employed, provided that it has no adverse effect on the reaction or the reagents involved and that it can dissolve the reagents, at least to some extent. The described reactions can take place over a wide range of temperatures, and the precise reaction temperature is not critical to the invention. It is convenient to carry out the described reactions in a temperature range between -78 °C to reflux temperature. The time required for the reaction may also vary widely, depending on many factors, notably the reaction temperature and the nature of the reagents. However, a period of from 0.5 h to several days will usually suffice to yield the described intermediates and compounds. The reaction sequence is not limited to the one displayed in the schemes, however, depending on the starting materials and their respective reactivity the sequence of reaction steps can be freely altered. Starting materials are either commercially available or can be prepared by methods analogous to the methods given below, by methods described in references cited in the description or in the examples, or by methods known in the art.

The present compounds of formula (I) and their pharmaceutically acceptable salts can be prepared by the process described in Scheme 1.

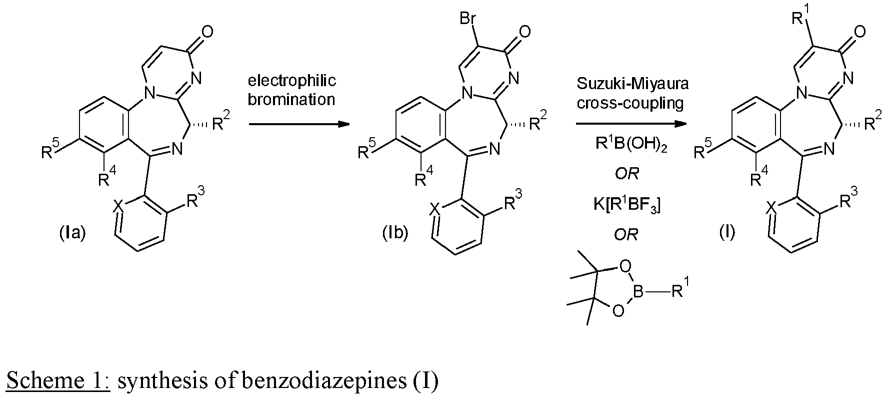

According to Scheme 1, benzodiazepine derivatives of formula (I) can be prepared in two steps starting from a pyrimido[1,2-a][1,4]benzodiazepin-3-one of formula (Ia). Following regioselective electrophilic bromination using N-bromosuccinimide, heteroaryl bromide intermediates (Ib) can be converted to final pyrimido[1,2-a][1,4]benzodiazepin-3-ones via a Suzuki-Miyaura reaction using boronic acids or boronic esters or potassium trifluoroborate.

Synthesis of pyrimido[1,2-a][1,4]benzodiazepin-3-ones of formula (Ia) can be accomplished in three steps by a process described below (Scheme 2). Building block lactames (II) are converted to corresponding thiolactames (III) using Lawesson's reagent or P₂S₅. Their reaction with ammonia yielded amidines of general formula (IV) which can undergo a regioselective thermal cyclisation under neutral conditions with activated alkynoates (V) *(e.g.* ethyl prop-2-ynoate) to form desired pyrimido[1,2-a][1,4]benzodiazepin-3-ones (Ia).

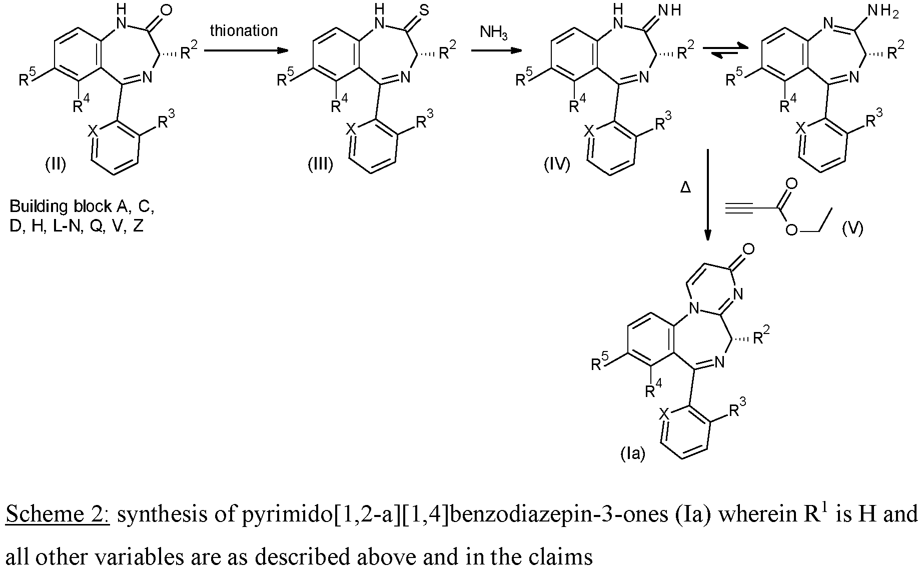

Syntheses of building blocks (A, C, D, H, N, Z) of formula (II) are highlighted in Scheme 3. Commercially available 2-amino-6-chlorobenzoic acid or 2-amino-6-bromobenzoic acid can be heated in acetic anhydride to form 5-chloro-2-methyl-3,1-benzoxazin-4-one and 5-bromo-2-methyl-3,1-benzoxazin-4-one, respectively. Grignard or organolithium reagents of formula (VI) (prepared by metalation reaction from corresponding aryl bromide or *via* kinetic deprotonation) can be reacted with benzoxazin-4-ones (electrophiles) at controlled temperatures to provide ketones of formula (VII). Following N-acetamide hydrolysis under acidic conditions (HCl), compounds of formula (VII) are converted into anilines of formula (VIII). Conveniently, at this junction, the halogen at R⁵ can be installed by treatment with N-chlorosuccinimide (NCS), N-bromosuccinimide (NBS) or *N-*iodosuccinimide (NIS) to yield intermediates of formula (IX). Final thermal cyclisation reaction with ethyl 2-aminoacetate hydrochloride in pyridine yields the desired benzodiazepines (II), presumably via formation of imine intermediate (X).

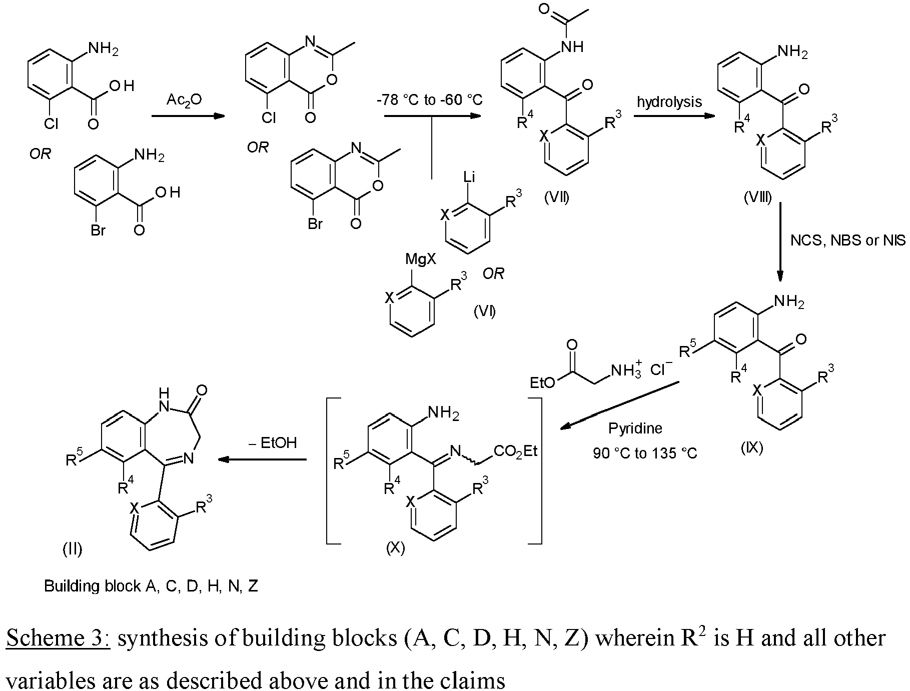

In further embodiments of the invention, where R² is alkyl or substituted alkyl, an alternative process is envisaged and detailed in Scheme 4.

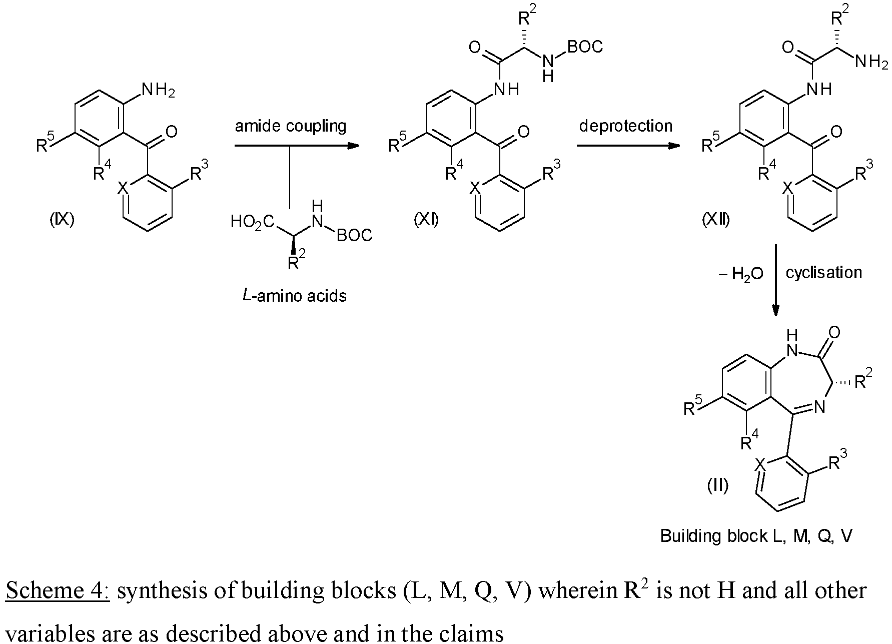

In such a case, compounds of formula (XI) can be prepared by amide coupling reaction between anilines (IX) and N-Boc protected *L*-amino acids upon exposure to phosphoryl chloride (POCl₃), or by other methods known to those skilled in the art. Removal of N-Boc protecting group can be effected with mineral acids *(e.g.* HCl) or organic acids *(e.g.* trifluoroacetic acid) to yield amines of formula (XII). Final intramolecular condensation reaction promoted by acidic media (e.g. silica or acetic acid or citric acid) and heat (80-110 °C) provides the desired benzodiazepine building blocks (L, M, Q, V) of formula (II). Notably, in the processes described in Scheme 1, 2 and 4, racemization at the chiral center occurs to a different extent (20-100%) depending on specific reaction conditions adopted. As a result, chiral purification (e.g. by HPLC or SFC) of final derivatives of formula (I) is required to obtain final derivatives with enantiomeric excess (ee) above 97%.

Also an embodiment of the present invention is a process to prepare pyrimido[1,2-a][1,4]benzodiazepin-3-ones of formula (I) as defined above comprising the reaction of a compound of formula (Ib) with a boronic acid or boronic ester or potassium trifluoroborate in a solvent, particularly mixtures of 1,4-dioxane/water, and a base such potassium carbonate and at temperature between 60 °C temperature and reflux of solvent or mixture thereof (Scheme 5).

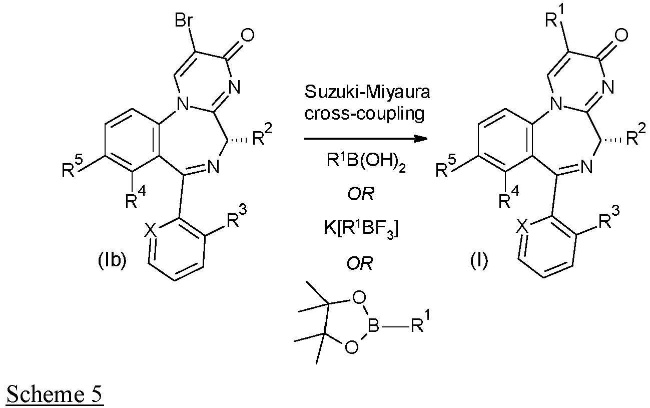

In one aspect, the present invention provides a process of manufacturing the compounds of formula (I) described herein, wherein said process is as described in any one of Schemes 1 to 5 above.

In a further aspect, the present invention provides a compound of formula (I) as described herein, or a pharmaceutically acceptable salt thereof, when manufactured according to the processes disclosed herein.

### Using the Compounds of the Invention

As explained in the background section and illustrated in the experimental section, the compounds of formula (I) and their pharmaceutically acceptable salts possess valuable pharmacological properties that make them useful for the treatment or prevention of diseases or condictions that are associated with the GABAA γ1 receptor.

In one aspect, the present invention provides a compound of formula (I) as described herein, or a pharmaceutically acceptable salt thereof, for use as therapeutically active substance.

In a further aspect, the present invention provides compounds for use in a method for treating or preventing acute neurological disorders, chronic neurological disorders and/or cognitive disorders in a subject, said method comprising administering an effective amount of a compound of formula (I) as described herein, or a pharmaceutically acceptable salt thereof, or a pharmaceutical composition described herein, to the subject.

In a further aspect, the present invention provides a compound of formula (I) as described herein, or a pharmaceutically acceptable salt thereof, or a pharmaceutical composition described herein, for use in a method for treating or preventing acute neurological disorders, chronic neurological disorders and/or cognitive disorders in a subject.

In a further aspect, the present invention provides the use of a compound of formula (I) as described herein, or a pharmaceutically acceptable salt thereof, for the manufacture of a medicament for the treatment of prevention of acute neurological disorders, chronic neurological disorders and/or cognitive disorders.

In one embodiment, said acute neurological disorders, chronic neurological disorders and/or cognitive disorders are selected from autism spectrum disorders (ASD), Angelman syndrome, age-related cognitive decline, Rett syndrome, Prader-Willi syndrome, amyotrophic lateral sclerosis (ALS), fragile-X disorder, negative and/or cognitive symptoms associated with schizophrenia, tardive dyskinesia, anxiety, social anxiety disorder (social phobia), panic disorder, agoraphobia, generalized anxiety disorder, disruptive, impulse-control and conduct disorders, Tourette's syndrome (TS), obsessive-compulsive disorder (OCD), acute stress disorder, post-traumatic stress disorder (PTSD), attention deficit hyperactivity disorder (ADHD), sleep disorders, Parkinson's disease (PD), Huntington's chorea, Alzheimer's disease (AD), mild cognitive impairment (MCI), dementia, behavioral and psychological symptoms (BPS) in neurodegenerative conditions, multi-infarct dementia, agitation, psychosis, substance-induced psychotic disorder, aggression, eating disorders, depression, chronic apathy, anhedonia, chronic fatigue, seasonal affective disorder, postpartum depression, drowsiness, sexual dysfunction, bipolar disorders, epilepsy and pain.

In one embodiment, said acute neurological disorders, chronic neurological disorders and/or cognitive disorders are selected from Alzheimer's disease, mild cognitive impairment (MCI), age-related cognitive decline, negative and/or cognitive symptoms associated with schizophrenia, bipolar disorders, autism spectrum disorder (ASD), Angelman syndrome, Rett syndrome, Prader-Willi syndrome, epilepsy, post-traumatic stress disorder (PTSD), amyotrophic lateral sclerosis (ALS), and fragile-X disorder.

In a preferred embodiment, said acute neurological disorders, chronic neurological disorders and/or cognitive disorders are selected from autism spectrum disorder (ASD), Angelman syndrome, Alzheimer's disease, negative and/or cognitive symptoms associated with schizophrenia and post-traumatic stress disorder (PTSD).

In a preferred embodiment, said acute neurological disorders, chronic neurological disorders and/or cognitive disorders are selected from autism spectrum disorder (ASD), Rett syndrome, Angelman syndrome, post-traumatic stress disorder and fragile-X disorder.

In a preferred embodiment, said acute neurological disorders, chronic neurological disorders and/or cognitive disorders are selected from autism spectrum disorder (ASD), Rett syndrome, post-traumatic stress disorder and fragile-X disorder.

In a particularly preferred embodiment, said acute neurological disorders, chronic neurological disorders and/or cognitive disorders are autism spectrum disorder (ASD).

In a particularly preferred embodiment, said acute neurological disorders, chronic neurological disorders and/or cognitive disorders are Angelman syndrome.

In a further particularly preferred embodiment, said acute neurological disorders, chronic neurological disorders and/or cognitive disorders are autism spectrum disorder (ASD), targeting core symptoms and associated comorbidities, such as anxiety and irritability, social anxiety disorder (social phobia) and generalized anxiety disorder.

In a further particularly preferred embodiment, said acute neurological disorders, chronic neurological disorders and/or cognitive disorders are selected from social anxiety disorder (social phobia) and generalized anxiety disorder.

### Pharmaceutical Compositions and Administration

In one aspect, the present invention provides pharmaceutical compositions comprising compounds of formula (I) or their pharmaceutically acceptable salts as defined herein and one or more pharmaceutically acceptable excipients. Exemplary pharmaceutical compositions are described in the Example section below.

In a further aspect, the present invention relates to pharmaceutical compositions comprising compounds of formula (I) or their pharmaceutically acceptable salts as defined above and one or more pharmaceutically acceptable excipients for the treatment or prevention of acute neurological disorders, chronic neurological disorders and/or cognitive disorders.

The compounds of formula (I) and their pharmaceutically acceptable salts can be used as medicaments (e.g. in the form of pharmaceutical preparations). The pharmaceutical preparations can be administered internally, such as orally (e.g. in the form of tablets, coated tablets, dragées, hard and soft gelatin capsules, solutions, emulsions or suspensions), nasally (e.g. in the form of nasal sprays) or rectally (e.g. in the form of suppositories). However, the administration can also be effected parentally, such as intramuscularly or intravenously (e.g. in the form of injection solutions or infusion solutions).

The compounds of formula (I) and their pharmaceutically acceptable salts can be processed with pharmaceutically inert, inorganic or organic excipients for the production of tablets, coated tablets, dragées and hard gelatin capsules. Lactose, corn starch or derivatives thereof, talc, stearic acid or its salts etc. can be used, for example, as such excipients for tablets, dragées and hard gelatin capsules.

Suitable excipients for soft gelatin capsules are, for example, vegetable oils, waxes, fats, semi-solid substances and liquid polyols, etc.

Suitable excipients for the production of solutions and syrups are, for example, water, polyols, saccharose, invert sugar, glucose, etc.

Suitable excipients for injection solutions are, for example, water, alcohols, polyols, glycerol, vegetable oils, etc.

Suitable excipients for suppositories are, for example, natural or hardened oils, waxes, fats, semi-solid or liquid polyols, etc.

Moreover, the pharmaceutical preparations can contain preservatives, solubilizers, viscosity-increasing substances, stabilizers, wetting agents, emulsifiers, sweeteners, colorants, flavorants, salts for varying the osmotic pressure, buffers, masking agents or antioxidants. They can also contain still other therapeutically valuable substances.

The dosage can vary in wide limits and will, of course, be fitted to the individual requirements in each particular case. In general, in the case of oral administration a daily dosage of about 0.1 mg to 20 mg per kg body weight, preferably about 0.5 mg to 4 mg per kg body weight (e.g. about 300 mg per person), divided into preferably 1-3 individual doses, which can consist, for example, of the same amounts, should be appropriate. It will, however, be clear that the upper limit given herein can be exceeded when this is shown to be indicated.

### Examples

The invention will be more fully understood by reference to the following examples. The claims should not, however, be construed as limited to the scope of the examples.

In case the preparative examples are obtained as a mixture of enantiomers, the pure enantiomers can be separated by methods described herein or by methods known to the man skilled in the art, such as e.g., chiral chromatography (e.g., chiral SFC) or crystallization.

All reaction examples and intermediates were prepared under an argon atmosphere if not specified otherwise.

### Building block A

### 7-bromo-6-chloro-5-(2,6-difluorophenyl)-1,3-dihydro-1,4-benzodiazepin-2-one

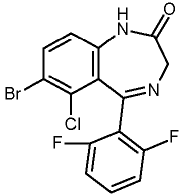

### a) 5-chloro-2-methyl-3,1-benzoxazin-4-one

A solution of 2-amino-6-chlorobenzoic acid (250 g, 1.46 mol) in acetic anhydride (1250 mL) was stirred at 140 °C for 2 h. The reaction mixture was concentrated *in vacuo.* The resulting crude residue was suspended in ethyl acetate (1000 mL), stirred for 30 min, filtered and dried *in vacuo* to afford the title compound (238 g, 84 %) as a grey solid. ¹H NMR (DMSO-*d₆*, 400 MHz): δ: 7.80 (app t, *J =* 8.0 Hz, 1H), 7.62 (d, *J =* 8.0 Hz, 1H), 7.49 (d, *J=* 7.6 Hz, 1H), 2.36 (s, 3H).

### b) N-[3-chloro-2-(2,6-difluorobenzoyl)phenyl]acetamide

To a solution of 5-chloro-2-methyl-3,1-benzoxazin-4-one (100 g, 511.2 mmol) and 2-bromo-1,3-difluorobenzene (118.4 g, 613.5 mmol) in tetrahydrofuran (1000 mL) was added dropwise *i*-PrMgCl·LiCl (1.3 m, 500 mL, 650 mmol) at -70 °C under nitrogen. The mixture was allowed to warm up to room temperature within 1 h, quenched with saturated aqueous ammonium chloride (1500 mL) and extracted with ethyl acetate (2 x 1500 mL). The organic phase was washed with brine (2000 mL), dried over sodium sulfate and concentrated *in vacuo.* The residue was suspended in ethyl acetate (150 mL). The resulting suspension was stirred at room temperature for 20 min, filtered and dried *in vacuo* to afford the title compound (113 g, 71 %) as an off-white solid. ¹H NMR (DMSO-*d₆*, 400 MHz): δ: 9.85 (s, 1H), 7.65-7.45 (m, 1H), 7.40 (t, *J =* 7.2 Hz, 1H), 7.38-7.34 (m, 2H), 7.16 (t, *J =* 8.8 Hz, 2H), 1.85 (s, 3H).

### c) (2-amino-6-chloro-phenyl)-(2,6-difluorophenyl)methanone

To a solution of *N*-[3-chloro-2-(2,6-difluorobenzoyl)phenyl]acetamide (113 g, 364.9 mmol) in ethanol (250 mL) was added aqueous hydrochloric acid (12 m, 200 mL). The reaction mixture was stirred at 100 °C for 1 h, then diluted with ethyl acetate (1100 mL). The organic phase was washed with water (1100 mL), saturated aqueous sodium bicarbonate (1100 mL) and brine (1100 mL), dried over sodium sulfate and concentrated *in vacuo.* Petroleum ether (120 mL) was added to the crude and the suspension was stirred at room temperature for 20 min. The solid was filtered and dried to afford the title compound (88 g, 90 %) as a yellow solid. ¹H NMR (DMSO-*d₆*, 400 MHz): δ: 7.62-7.56 (m, 1H), 7.21-7.15 (m, 3H), 6.83 (d, *J =* 7.6 Hz, 1H), 6.74 (s, 2H), 6.58 (d, *J =* 7.6 Hz, 1H).

### d) (6-amino-3-bromo-2-chloro-phenyl)-(2,6-difluorophenyl)methanone

To a solution of (2-amino-6-chloro-phenyl)-(2,6-difluorophenyl)methanone (88.0 g, 328.8 mmol) in dichloromethane (225 mL) and N,N-dimethylformamide (225 mL) was added 1-bromopyrrolidine-2,5-dione (64.4 g, 362 mmol) at 0 °C. The reaction mixture was stirred at 30 °C for 1 h. The mixture was diluted with dichloromethane (600 mL) and washed with water (500 mL) and brine (4 × 500 mL), dried over sodium sulfate and concentrated *in vacuo.* The residue was purified by chromatography (silica, petroleum ether / ethyl acetate, 1:0 to 2:1). The solid was suspended in petroleum ether (200 mL) and stirred at room temperature for 20 min. The suspension was filtered and the solid was dried *in vacuo* to afford the title compound (96.0 g, 84 %) as a yellow solid. MS: 345.9 ([{⁷⁹Br, ³⁵Cl}M+H]⁺), 347.8 ([{⁸¹Br, ³⁵Cl or ⁷⁹Br, ³⁷Cl }M+H]⁺), ESI pos.

### e) 7-bromo-6-chloro-5-(2,6-difluorophenyl)-1,3-dihydro-1,4-benzodiazepin-2-one

To a solution of (6-amino-3-bromo-2-chloro-phenyl)-(2,6-difluorophenyl)methanone (25.0 g, 72.1 mmol) in pyridine (625 mL) was added ethyl 2-aminoacetate hydrochloride (70.5 g, 505 mmol). The reaction mixture was stirred at 135 °C for 36 h. The reaction mixture was concentrated *in vacuo* to remove pyridine. The residue was diluted with ethyl acetate (2000 mL) and washed with HCl (1 m, 3 × 1500 mL), water (2000 mL) and brine (2 × 1000 mL), dried over sodium sulfate, filtered and concentrated *in vacuo.* The crude product was purified by flash column chromatography (silica, petroleum ether / ethyl acetate 10: 1 to 2:1) to afford the title compound (10.1 g, 12 %) as an off-white solid. MS: 385.0 ([{⁷⁹Br, ³⁵Cl}M+H]⁺), ESI pos.

### Building block C

### 6,7-dichloro-5-(2-fluorophenyl)-1,3-dihydro-1,4-benzodiazepin-2-one

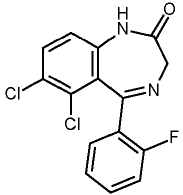

### a) N-[3-chloro-2-(2-fluorobenzoyl)phenyl]acetamide

To a solution of 5-chloro-2-methyl-3,1-benzoxazin-4-one (20.0 g, 102.3 mmol) and 1-bromo-2-fluorobenzene (17.89 g, 102.3 mmol) in tetrahydrofuran (600 mL) at -70 °C was added dropwise n-BuLi in tetrahydrofuran (2.5 m, 49.1 mL, 123 mmol). The reaction mixture was stirred at -60 °C for 1 h, then quenched with aqueous ammonium chloride (200 mL). The aqueous layer was extracted with tetrahydrofuran (2 × 250 mL) and ethyl acetate (2 × 250 mL). The combined organic phase was washed with brine (200 mL), dried over sodium sulfate and concentrated *in vacuo.* Purification by flash column chromatography (silica, petroleum ether / ethyl acetate 20:1 to 3:1) afforded the title compound (21 g, 70 %) as a white solid. MS: 292.3 ([M+H]⁺), ESI pos.

### b) (2-amino-6-chloro-phenyl)-(2-fluorophenyl)methanone

In analogy to experiment of building block A c, N-[3-chloro-2-(2-fluorobenzoyl)phenyl]acetamide was converted into the title compound (10 g, 58 %) which was obtained as a yellow solid. MS: 250.1 ([M+H]⁺), ESI pos.

### c) (6-amino-2,3-dichloro-phenyl)-(2-fluorophenyl)methanone

In analogy to experiment of building block A d, (2-amino-6-chloro-phenyl)-(2-fluorophenyl)methanone using 1-chloropyrrolidine-2,5-dione instead of 1-bromopyrrolidine-2,5-dione was converted into the title compound (234 mg, 53 %) which was obtained as an orange yellow solid. MS: 284.0 ([{³⁵Cl, ³⁵Cl}M+H]⁺), ESI pos.

### d) 6,7-dichloro-5-(2-fluorophenyl)-1,3-dihydro-1,4-benzodiazepin-2-one

In analogy to experiment of building block A e, (6-amino-2,3-dichloro-phenyl)-(2-fluorophenyl)methanone was converted into the title compound (193 mg, 74 %) which was obtained as a grey solid. MS: 323.2 ([{³⁵Cl, ³⁵Cl}M+H]⁺), ESI pos.

### Building block D

### 6,7-dichloro-5-(2,6-difluorophenyl)-1,3-dihydro-1,4-benzodiazepin-2-one

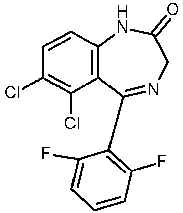

### a) (6-amino-2,3-dichloro-phenyl)-(2,6-difluorophenyl)methanone

In analogy to experiment of building block A d, (2-amino-6-chloro-phenyl)-(2,6-difluorophenyl)methanone using 1-chloropyrrolidine-2,5-dione instead of 1-bromopyrrolidine-2,5-dione was converted into the title compound (3.44 g, 47 %) which was obtained as a yellow solid. MS: 302.0 ([{³⁵Cl, ³⁵Cl}M+H]⁺), ESI pos.

### b) 6,7-dichloro-5-(2,6-difluorophenyl)-1,3-dihydro-1,4-benzodiazepin-2-one

In analogy to experiment of building block A e, (6-amino-2,3-dichloro-phenyl)-(2,6-difluorophenyl)methanone was converted into the title compound (2.6 g, 53 %) which was obtained as a yellow solid. MS: 341.0 ([{³⁵Cl, ³⁵Cl}M+H]⁺), ESI pos.

### Building block H 6-chloro-5-(2,6-difluorophenyl)-7-iodo-1,3-dihydro-1,4-benzodiazepin-2-one

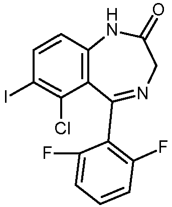

### a) (6-amino-2-chloro-3-iodo-phenyl)-(2,6-difluorophenyl)methanone

In analogy to experiment of building block A d, (2-amino-6-chloro-phenyl)-(2,6-difluorophenyl)methanone using 1-iodopyrrolidine-2,5-dione instead of 1-bromopyrrolidine-2,5-dione was converted into the title compound (7.1 g, 85 %) which was obtained as a yellow solid. MS: 393.8 ([M+H]⁺), ESI pos.

### b) 6-chloro-5-(2,6-difluorophenyl)-7-iodo-1,3-dihydro-1,4-benzodiazepin-2-one

In analogy to experiment of building block A e, (6-amino-2-chloro-3-iodo-phenyl)-(2,6-difluorophenyl)methanone was converted into the title compound (2.7 g, 68 %) which was obtained as a yellow solid. MS: 432.8 ([M+H]⁺), ESI pos.

### Building block L

### (3S)-7-bromo-6-chloro-5-(2,6-difluorophenyl)-3-methyl-1,3-dihydro-1,4-benzodiazepin-2-one

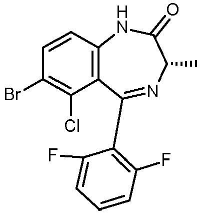

### a) tert-butyl N-[(15)-2-[4-bromo-3-chloro-2-(2.6-difluorobenzoyl)anilino]-1-methyl-2-oxo-ethyllcarbamate

To a solution of (6-amino-3-bromo-2-chloro-phenyl)-(2,6-difluorophenyl)methanone (1.0 g, 2.45 mmol) and (2*S*)-2-(*tert*-butoxycarbonylamino)propanoic acid (0.696 g, 3.68 mmol) in pyridine (12.0 mL) was added phosphoryl chloride (0.489 g, 3.19 mmol) slowly at -5 °C. The reaction mixture was stirred at -5 °C for 1 h, before being slowly poured into water (75 mL) and extracted with ethyl acetate (3 × 75 mL). The combined organic layers were washed with brine (40 mL), dried (Na₂SO₄) and concentrated *in vacuo.* The residue was purified by flash column chromatography (silica, heptane/ ethyl acetate, 10:0 to 4:1) to afford the title compound (1.41 g, 95 %) as a white foam. MS: 418.7 ([{⁸¹Br, ³⁵Cl or ⁷⁹Br, ³⁷Cl }M-C₄Hs-CO₂+H]⁺), 540.7 ([{⁸¹Br, ³⁵Cl or ⁷⁹Br, ¹⁷Cl }M+Na]⁺), ESI pos.

### b) (2S)-2-amino-N-[4-bromo-3-chloro-2-(2,6-difluorobenzoyl)phenyl]propanamide

To a solution of *tert*-butyl *N*-[(1*S*)-2-[4-bromo-3-chloro-2-(2,6-difluorobenzoyl)anilino]-1-methyl-2-oxo-ethyl]carbamate (0.534 g, 1.03 mmol) in dichloromethane (4.0 mL) was slowly added hydrochloric acid (4.0 m in 1,4-dioxane, 2.58 mL, 10.3 mmol). The reaction mixture was stirred at 25 °C for 3 h. Saturated aqueous sodium bicarbonate was added slowly until pH>8, then the mixture was extracted with dichloromethane (3 × 50 mL). The combined organic layers were washed with brine (20 mL), dried (Na₂SO₄) and concentrated *in vacuo* to afford the title compound (0.424 g, 98 %) as a light yellow oil, which was used in the following step without further purification.

### c) (3S)-7-bromo-6-chloro-5-(2,6-difluorophenyl)-3-methyl-1,3-dihydro-1,4-benzodiazepin-2-one

To a solution of (2*S*)-2-amino-N-[4-bromo-3-chloro-2-(2,6-difluorobenzoyl)phenyl]propanamide (960 mg, 2.30 mmol) in toluene (9.19 mL) was added silica (138 mg, 2.30 mmol). The reaction mixture was stirred at 90 °C for 15 h, then concentrated *in vacuo.* The residue was purified by flash column chromatography (petroleum ether / ethyl acetate 3:1) to afford the title compound (920 mg, 95 %) as a yellow solid. MS: 399.1 ([{⁷⁹Br, ³⁵Cl}M+H]⁺), 401.1 ([{⁸¹Br, ³⁵Cl or ⁷⁹Br, ³⁷Cl }M+H]⁺), ESI pos.

### Building block M

### (3S)-6,7-dichloro-5-(2,6-difluorophenyl)-3-methyl-1,3-dihydro-1,4-benzodiazepin-2-one

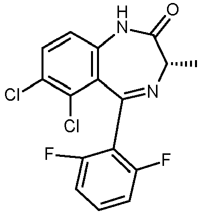

### a) tert-butyl N-[(1S)-2-[3,4-dichloro-2-(2,6-difluorobenzoyl)anilino]-1-methyl-2-oxo-ethyllcarbamate

In analogy to experiment of building block L a, (6-amino-2,3-dichloro-phenyl)-(2,6-difluorophenyl)methanone was converted into the title compound (5.0 g, 64 %) which was obtained as a yellow foam. The crude was used as such in the following step without further characterization.

### b) (2S)-2-amino-N-[3,4-dichloro-2-(2,6-difluorobenzoyl)phenyl]propanamide

In analogy to experiment of building block L b, tert-butyl *N*-[(1*S*)-2-[3,4-dichloro-2-(2,6-difluorobenzoyl)anilino]-1-methyl-2-oxo-ethyl]carbamate was converted into the title compound (3.6 g, 91 %) which was obtained as a yellow oil. MS: 373.0 ([{³⁵Cl, ³⁵Cl}M+H]⁺), ESI pos.

### c) (3S)-6,7-dichloro-5-(2,6-difluorophenyl)-3-methyl-1,3-dihydro-1,4-benzodiazepin-2-one

In analogy to experiment of building block L c, (2S)-2-amino-N-[3,4-dichloro-2-(2,6-difluorobenzoyl)phenyl]propanamide was converted into the title compound (3.20 g, 93 %) which was obtained as a yellow foam. MS: 355.0 ([{³⁵Cl, ³⁵Cl}M+H]⁺), ESI pos.

### Building block N

### 6,7-dichloro-5-(3-fluoro-2-pyridyl)-1,3-dihydro-1,4-benzodiazepin-2-one

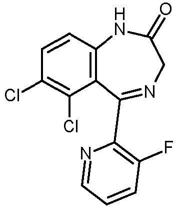

### a) N-[3-chloro-2-(3-fluoropyridine-2-carbonyl)phenyl]acetamide

To a solution of 2-bromo-3-fluoropyridine (18.0 g, 102 mmol) in methyl tert-butyl ether (720 mL) was added *n*-BuLi (2.5 m in tetrahydrofuran, 36.8 mL, 92.02 mmol) slowly at - 60 °C. The mixture was stirred for 1 h, then a solution of 5-chloro-2-methyl-3,1-benzoxazin-4-one (10.0 g, 51.1 mmol) in tetrahydrofuran (600 mL) was added dropwise. The mixture was stirred at -60 °C for 1 h, then quenched by addition of saturated aqueous ammonium chloride (50 mL). The mixture was diluted with water (100 mL) and extracted with ethyl acetate (3 × 100 mL). The organic layer was washed with brine (100 mL), dried over sodium sulfate and concentrated *in vacuo.* Purification by flash column chromatography (dichloromethane / methanol 50:1 to 20:1) afforded the title compound (5.20 g, 23.2%) as a light yellow solid. MS: 293.1 ([M+H]⁺), ESI pos.

### b) N-[3,4-dichloro-2-(3-fluoropyridine-2-carbonyl)phenyl]acetamide

In analogy to experiment of building block A d, *N*-[3-chloro-2-(3-fluoropyridine-2-carbonyl)phenyl]acetamide using 1-chloropyrrolidine-2,5-dione instead of 1-bromopyrrolidine-2,5-dione was converted into the title compound (36 g, 84 %) which was obtained as a white solid. MS: 327.0 ([{³⁵Cl, ³⁵Cl}M+H]⁺), ESI pos.

### c) (6-amino-2,3-dichloro-phenyl)-(3-fluoro-2-pyridyl)methanone

A suspension of *N*-[3,4-dichloro-2-(3-fluoropyridine-2-carbonyl)phenyl]acetamide (4.00 g, 12.2 mmol) in hydrochloric acid (4.0 m in methanol, 61 mL, 244 mmol) was stirred at 40 °C for 24 h. After cooling to room temperature a saturated aqueous solution of sodium bicarbonate was added dropwise to pH 8-9. The mixture was extracted with ethyl acetate (3 × 150 mL). The combined organic layer was washed with brine (100 mL), dried over sodium sulfate and concentrated *in vacuo.* Purification by flash column chromatography (silica, petroleum ether / ethyl acetate, 20:1 to 3:1) afforded the title compound (2.95 g, 85%) as an orange solid. MS: 285.0 ([{³⁵Cl, ³⁵Cl}M+H]⁺), ESI pos.

### d) 6,7-dichloro-5-(3-fluoro-2-pyridyl)-1,3-dihydro-1,4-benzodiazepin-2-one

In analogy to experiment of building block A e, (6-amino-2,3-dichloro-phenyl)-(3-fluoro-2-pyridyl)methanone was converted into the title compound (3.76 g, 28 %) which was obtained as a white solid. MS: 323.7 ([{³⁵Cl, ³⁵Cl}M+H]⁺), ESI pos.

### Building block Q

### (3S)-6-bromo-7-chloro-5-(2,6-difluorophenyl)-3-methyl-1,3-dihydro-1,4-benzodiazepin-2-one

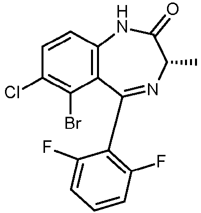

### a) 5-bromo-2-methyl-3,1-benzoxazin-4-one

A solution of 2-amino-6-bromo-benzoic acid (9.00 g, 41.7 mmol) in acetic anhydride (118 mL) was stirred at 120 °C for 3.5 h. The reaction mixture was cooled to -78 °C and stirred for 2 h. The resulting solid was filtered, washed with TBME and dried *in vacuo* to afford the title compound (8.55 g, 85 %) as light brown solid. ¹H NMR (CDCl₃, 300 MHz): δ: 7.74 (dd, *J=* 7.8, 1.3 Hz, 1H), 7.56 (dd, *J* = 8.0, 7.8 Hz, 1H), 7.48 (dd, *J =* 8.0, 1.3 Hz, 1H), 2.45 (s, 3H). This material was used as such in the following step without further characterization.

### b) N-[3-bromo-2-(2,6-difluorobenzoyl)phenyl]acetamide

To a solution of 2-bromo-1,3-difluorobenzene (7.56 g, 39.2 mmol) in anhydrous tetrahydrofuran (64 mL) at -70 °C was added dropwise n-BuLi in hexane (1.6 m, 24.5 mL, 39.2 mmol). The reaction mixture was stirred at -78 °C for 1 h, then added dropwise to an ice-cold solution of 5-bromo-2-methyl-3,1-benzoxazin-4-one (8.55 g, 35.6 mmol) in tetrahydrofuran (128 mL). The reaction mixture was stirred at 0 °C for 1.5 h. The reaction mixture was poured onto an ice-cold aqueous saturated ammonium chloride solution (100 mL). The aqueous layer was extracted with TBME. The combined organic phase was washed with brine, dried (MgSO₄) and concentrated *in vacuo.* The residue was purified by flash column chromatography (silica, 0-100% ethyl acetate in petroleum ether) to afford the title compound (7.72 g, 61 %) as light yellow solid. MS: 354.1 ([{⁷⁹Br, ³⁵Cl}M+H]⁺), 356.1 ([{⁸¹Br, ³⁵Cl or ⁷⁹Br, ³⁷Cl }M+H]⁺), ESI pos.

### c) N-[3-bromo-4-chloro-2-(2,6-difluorobenzoyl)phenyl]acetamide

In analogy to experiment of building block A d, N-(3-bromo-2-(2,6-difluorobenzoyl)phenyl)acetamide using 1-chloropyrrolidine-2,5-dione instead of 1-bromopyrrolidine-2,5-dione was converted into the title compound (10.1 g, 70 %) which was obtained as a light yellow solid. MS: 388.0 ([{⁷⁹Br, ³⁵Cl}M+H]⁺), 390.1 ([{⁸¹Br, ³⁵Cl or ⁷⁹Br, ³⁷Cl }M+H]⁺), ESI pos.

### d) (6-amino-2-bromo-3-chloro-phenyl)-(2,6-difluorophenyl)methanone

In analogy to experiment of building block A c, N-[3-bromo-4-chloro-2-(2,6-difluorobenzoyl)phenyl]acetamide was converted into the title compound (8.2 g, 92 %) which was obtained as a yellow solid. MS: 346.0 ([{⁷⁹Br, ³⁵Cl}M+H]⁺), 348.0 ([{⁸¹Br, ³⁵Cl or ⁷⁹Br, ³⁷Cl }M+H]⁺), ESI pos.

### e) tert-butyl N-[(1S)-2-[3-bromo-4-chloro-2-(2,6-difluorobenzoyl)anilino]-1-methyl-2-oxo-ethyllcarbamate

In analogy to experiment of building block L a, (6-amino-2-bromo-3-chloro-phenyl)-(2,6-difluorophenyl)methanone using (2*S*)-2-(*tert*-butoxycarbonylamino)propanoic acid was converted into the title compound (8.64 g, 69 %) which was obtained as a yellow solid. MS: 515.2 ([{⁷⁹Br, ³⁵Cl}M-H]⁻), 517.1 ([{⁸¹Br, ³⁵Cl or ⁷⁹Br, ³⁷Cl }M-H]⁻), ESI neg.

### f) (2S)-2-amino-N-[3-bromo-4-chloro-2-(2,6-difluorobenzoyl)phenyl]propanamide

In analogy to experiment of building block L b, tert-butyl *N*-[(1*S*)-2-[3-bromo-4-chloro-2-(2,6-difluorobenzoyl)anilino]-1-methyl-2-oxo-ethyl]carbamate was converted into the title compound (6.27 g, 90 %) which was obtained as a light brown oil. MS: 417.1 ([{⁷⁹Br, ³⁵Cl}M+H]⁺), 419.0 ([{⁸¹Br, ³⁵Cl or ⁷⁹Br, ³⁷Cl }M+H]⁺), ESI pos.

### g) (3S)-6-bromo-7-chloro-5-(2,6-difluorophenyl)-3-methyl-1,3-dihydro-1,4-benzodiazepin-2-one

In analogy to experiment of building block L c, (2S)-2-amino-N-[3-bromo-4-chloro-2-(2,6-difluorobenzoyl)phenyl]propanamide was converted into the title compound (3.98 g, 68 %) which was obtained as a yellow solid. MS: 399.1 ([{⁷⁹Br, ³⁵Cl}M+H]⁺), 401.0 ([{⁸¹Br, ³⁵Cl or ⁷⁹Br, ³⁷Cl }M+H]⁺), ESI pos.

### Building block V

### (3S)-6-chloro-5-(2,6-difluorophenyl)-7-trifluoromethyl-3-methyl-1,3-dihydro-1,4-benzodiazepin-2-one

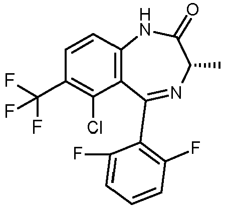

### a) tert-butyl N-[(1S)-2-[3-chloro-2-(2,6-difluorobenzoyl)-4-iodo-anilino]-1-methyl-2-oxo-ethyllcarbamate

In analogy to experiment of building block L a, (6-amino-2-chloro-3-iodo-phenyl)-(2,6-difluorophenyl)methanone using (2*S*)-2-(*tert*-butoxycarbonylamino)propanoic acid was converted into the title compound (5.8 g, 81 %) which was obtained as a yellow solid. MS: 465.0 ([M-C₄H₈-CO₂+H]⁺), 509.0 ([M-C₄H₈+H]⁺), ESI pos.

### b) (2S)-2-amino-N-[3-chloro-2-(2,6-difluorobenzoyl)-4-iodo-phenyl]propanamide

In analogy to experiment of building block L b, tert-butyl *N*-[(1*S*)-2-[3-chloro-2-(2,6-difluorobenzoyl)-4-iodo-anilino]-1-methyl-2-oxo-ethyl]carbamate was converted into the title compound (4.7 g, 99 %) which was obtained as a yellow solid. The crude was used as such in the following step without further characterization.

### c) (3S)-6-chloro-5-(2,6-difluorophenyl)-7-iodo-3-methyl-1,3-dihydro-1,4-benzodiazepin-2-one

In analogy to experiment of building block L c, (2S)-2-amino-N-[3-chloro-2-(2,6-difluorobenzoyl)-4-iodo-phenyl]propanamide was converted into the title compound (3.8 g, 94 %) which was obtained as a yellow solid. MS: 446.8 ([M+H]⁺), ESI pos.

### d) (3S)-6-chloro-5-(2,6-difluorophenyl)-3-methyl-7-(trifluoromethyl)-1,3-dihydro-1,4-benzodiazepin-2-one

A mixture of (3S)-6-chloro-5-(2,6-difluorophenyl)-7-iodo-3-methyl-1,3-dihydro-1,4-benzodiazepin-2-one (14.0 g, 31.35 mmol), methyl 2,2-difluoro-2-(fluorosulfonyl)acetate (18.07 g, 94.04 mmol) and copper (I) iodide (11.94 g, 62.69 mmol) in *N,N-*dimethylformamide (140 mL) and hexamethylphosphoric triamide (70 mL) was stirred under nitrogen at 70 °C for 16 h. The reaction mixture was poured into ice-water (150 mL) and the aqueous layer was extracted with ethyl acetate (4 x 150 mL). The combined organic phases were dried over sodium sulfate and concentrated in vacuo. The residue was purified by preparative HPLC (Phenomenex luna C18, water containing 0.1% trifluoroacetic acid / acetonitrile) and by chiral SFC (Daicel Chiralcel OJ, ethanol containing 0.1 % aqueous ammonia) to afford the title compound (4.2 g, 33 %) as a yellow solid. MS: 389.1 ([{³⁵Cl}M+H]+), ESI pos. ¹H NMR (CDCl₃, 400 MHz) δ: 8.71 (s, 1H), 7.81 (d, *J =* 8.6 Hz, 1H), 7.34 (tt, *J =* 6.3, 8.4 Hz, 1H), 7.18 (d, *J =* 8.5 Hz, 1H), 7.08 - 6.58 (m, 2H), 3.97 (q, *J =* 6.4 Hz, 1H), 1.79 (d, *J =* 6.4 Hz, 3H).

### Building block Z 6-bromo-7-chloro-5-(2,6-difluorophenyl)-1,3-dihydro-1,4-benzodiazepin-2-one

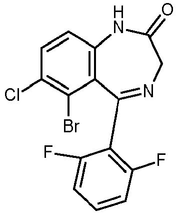

### 6-bromo-7-chloro-5-(2,6-difluorophenyl)-1,3-dihydro-1,4-benzodiazepin-2-one

To a solution of (6-amino-2-bromo-3-chloro-phenyl)-(2,6-difluorophenyl)methanone (100 mg, 0.289 mmol) in pyridine (1.8 mL) was added magnesium sulfate hydrate (399 mg, 2.89 mmol). The mixture was warmed to 90 °C and ethyl 2-aminoacetate hydrochloride (282 mg, 2.02 mmol) was added. The reaction mixture was stirred at 110 °C for 3 h. Further ethyl 2-aminoacetate hydrochloride (282 mg, 2.02 mmol) was added at 90 °C and stirring was continued at 110 °C. This was repeated until sufficient conversion.

The reaction mixture was cooled down to room temperature and most of pyridine was removed *in vacuo.* The residue was diluted with water and extracted with ethyl acetate. The organic phase was washed with aqueous HCl (1.0 m) and brine, dried (MgSO₄), filtered and concentrated *in vacuo.* The crude was purified by flash column chromatography (silica, petroleum ether / ethyl acetate 1:0 to 1:1) to afford the title compound (12 mg, 11 %) which was obtained as yellow solid. MS: 385.1 ([{⁷⁹Br, ³⁵Cl}M+H]⁺), 387.1 ([{⁸¹Br, ³⁵Cl or ⁷⁹Br, ³⁷Cl }M+H]⁺), ESI pos.

### Example 1

### 8,9-dichloro-7-(2-fluorophenyl)-5H-pyrimido[1,2-a][1,4]benzodiazepin-3-one

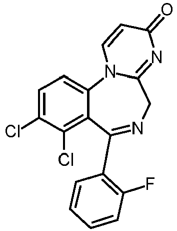

### a) 6,7-dichloro-5-(2-fluorophenyl)-1,3-dihydro-1,4-benzodiazepine-2-thione

To a solution of 6,7-dichloro-5-(2-fluorophenyl)-1,3-dihydro-1,4-benzodiazepin-2-one (building block C, 3.22 g, 9.96 mmol) in diglyme (40 mL) was added phosphorus pentasulfide (3.32 g, 14.9 mmol) and sodium bicarbonate (2.51 g, 29.9 mmol). The mixture was stirred at 80 °C until complete conversion as judged by LC-MS. The mixture was poured onto ice followed by stirring for 0.5 h and then extracted with ethyl acetate. The organic layer was washed with 1.0 m aqueous NaHCO₃ and brine, dried (Na₂SO₄) and concentrated *in vacuo.* The residue was purified by flash column chromatography (silica, 0-25 % ethyl acetate in heptane) to give the title compound (2.62 g, 78 %) as an orange solid. MS: 339.0 ([{³⁵Cl, ³⁵Cl}M+H]⁺), 341.0 ([{³⁵Cl, ³⁷Cl}M+H]⁺), ESI pos.

### b) 6,7-dichloro-5-(2-fluorophenyl)-3H-1,4-benzodiazepin-2-amine

To a solution of aqueous ammonia (25 wt.%, 5.7 ml, 75 mmol) in methanol (1.7 mL) was added a solution of 6,7-dichloro-5-(2-fluorophenyl)-1,3-dihydro-1,4-benzodiazepine-2-thione (339 mg, 1.0 mmol) in tetrahydrofuran (4.5 mL). The mixture was stirred at 22 °C for 3 days, before all volatiles were removed under vacuum. The resulting precipitate was re-dissolved in hot 1,4-dioxane, then allowed to cool to room temperature. The resulting solid was filtered off and dried under high vacuum to provide the title compound (105 mg, 33 %) as a white solid. MS: 322.1 ([{³⁵Cl, ³⁵Cl}M+H]⁺), 324.1 ([{³⁵Cl, ³⁷Cl}M+H]⁺), ESI pos.

### c) 8,9-dichloro-7-(2-fluorophenyl)-5H-pyrimido[1,2-a][1,4]benzodiazepin-3-one

To a suspension of 6,7-dichloro-5-(2-fluorophenyl)-3H-1,4-benzodiazepin-2-amine (50 mg, 0.16 mmol) in ethanol (2 mL) was added ethyl propiolate (33 mg, 0.34 mmol). The reaction mixture was heated to 60 °C for 18 h, before being cooled to room temperature. The mixture was concentrated *in vacuo* then purified by flash column chromatography (silica, 2-10% methanol in dichloromethane) followed by preparative HPLC (YMC-Triart C18, 5 µm, 100x30mm, 0.1% formic acid in water / acetonitrile) to provide the title compound (17 mg, 29 %) as a white solid. MS: 374.0 ([{³⁵Cl, ³⁵Cl}M+H]⁺), 376.0 ([{³⁵Cl, ³⁷Cl}M+H]⁺), ESI pos.

### Example 2

### 8,9-dichloro-7-(2,6-difluorophenyl)-5H-pyrimido[1,2-a][1,4]benzodiazepin-3-one

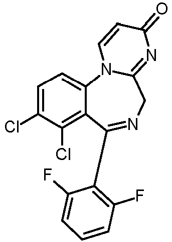

### a) 6,7-dichloro-5-(2,6-difluorophenyl)-1,3-dihydro-1,4-benzodiazepine-2-thione

In analogy to experiment of example 1 a, 6,7-dichloro-5-(2,6-difluorophenyl)-1,3-dihydro-1,4-benzodiazepin-2-one (building block D, 1.43 g, 4.19 mmol) was converted into the title compound (1.43 g, 96 %) which was obtained after precipitation from water as a light brown solid. MS: 357.1 ([{³⁵Cl, ³⁵Cl}M+H]⁺), 359.1 ([{³⁵Cl, ³⁷Cl}M+H]⁺), ESI pos. The crude product was used in the next step without further purification.

### b) 6,7-dichloro-5-(2,6-difluorophenyl)-3H-1,4-benzodiazepin-2-amine

In analogy to experiment of example 1 b, 6,7-dichloro-5-(2,6-difluorophenyl)-1,3-dihydro-1,4-benzodiazepine-2-thione (1.40 g, 3.92 mmol) was converted into the title compound (1.25 g, 94 %) which was obtained as an orange solid. MS: 340.2 ([{³⁵Cl, ³⁵Cl}M+H]⁺), 342.2 ([{³⁵Cl, ³⁷Cl}M+H]⁺), ESI pos. This crude material was used in the next step without further purification.

### c) 8,9-Dichloro-7-(2,6-difluorophenyl)-5H-pyrimido[1,2-a][1,4]benzodiazepin-3-one

In analogy to experiment of example 1 c, 6,7-dichloro-5-(2,6-difluorophenyl)-3H-1,4-benzodiazepin-2-amine (50 mg, 0.15 mmol) was converted into the title compound (23 mg, 40 %) which was obtained as an off-white solid. MS: 392.1 ([{³⁵Cl, ³⁵Cl}M+H]⁺), 394.1 ([{³⁵Cl, ³⁷Cl}M+H]⁺), ESI pos.

### Example 3

### 8,9-dichloro-7-(3-fluoro-2-pyridyl)-5H-pyrimido[1,2-a][1,4]benzodiazepin-3-one

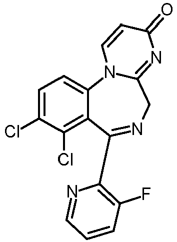

### a) 6,7-dichloro-5-(3-fluoro-2-pyridyl)-1,3-dihydro-1,4-benzodiazepine-2-thione

To a solution of 6,7-dichloro-5-(3-fluoro-2-pyridyl)-1,3-dihydro-1,4-benzodiazepin-2-one (building block N, 3.55 g, 11.0 mmol) in a 1:1 mixture of toluene and tetrahydrofuran (80 mL) was added Lawesson's reagent (4.65 g, 11.5 mmol). The mixture was stirred at 90 °C for 1 h until complete conversion as judged by TLC (petroleum ether / ethyl acetate 1:1). The reaction mixture was cooled to room temperature then concentrated *in vacuo.* The residue was purified by flash column chromatography (silica, dichloromethane/ethyl acetate 5:1) followed by trituration in ethyl acetate. The solid was filtered off and dried under high vacuum to provide the title compound (2.56 g, 69 %) as a light yellow solid. MS: 340.0 ([{³⁵Cl, ³⁵Cl}M+H]⁺), 341.9 ([{³⁵Cl, ³⁷Cl}M+H]⁺), ESI pos.

### b) 6,7-dichloro-5-(3-fluoro-2-pyridyl)-3H-1,4-benzodiazepin-2-amine

In analogy to experiment of example 1 b, 6,7-dichloro-5-(3-fluoro-2-pyridyl)-1,3-dihydro-1,4-benzodiazepine-2-thione (0.30 g, 0.88 mmol) was converted into the title compound (0.38 g, quant.) which was obtained as a yellow solid. MS: 322.9 ([{³⁵Cl, ³⁵Cl}M+H]⁺), 325.0 ([{³⁵Cl, ³⁷Cl}M+H]⁺), ESI pos. This crude material was used in the next step without further purification.

### c) 8,9-dichloro-7-(3-fluoro-2-pyridyl)-5H-pyrimido[1.2-a][1.4]benzodiazepin-3-one

In analogy to experiment of example 1 c, 6,7-dichloro-5-(3-fluoro-2-pyridyl)-3H-1,4-benzodiazepin-2-amine (0.38 g, 0.88 mmol) was converted into the title compound (148 mg, 45 %) which was obtained as a white solid after purification by preparative HPLC (Waters XBridge 5 µm, 150x25mm, NH₄OH in water / acetonitrile). MS: 375.0 ([{³⁵Cl, ³⁵Cl}M+H]⁺), 377.0 ([{³⁵Cl, ³⁷Cl}M+H]⁺), ESI pos.

### Example 4

### (5S)-8,9-dichloro-7-(2,6-difluorophenyl)-5-methyl-5H-pyrimido[1,2-a][1,4]benzodiazepin-3-one

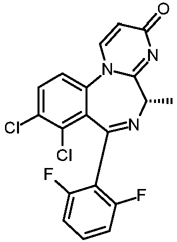

### a) (3S)-6,7-dichloro-5-(2,6-difluorophenyl)-3-methyl-1,3-dihydro-1,4-benzodiazepine-2-thione

A solution of (3*S*)-6,7-dichloro-5-(2,6-difluorophenyl)-3-methyl-1,3-dihydro-1,4-benzodiazepin-2-one (building block M, 5.0 g, 14.1 mmol) and Lawesson's reagent (5.69 g, 14.1 mmol) in toluene (100 mL) was heated to 110 °C until complete conversion as judged by TLC (petroleum ether / ethyl acetate 2:1). The reaction mixture was cooled to room temperature then concentrated *in vacuo.* The residue was purified by flash column chromatography (silica, petroleum ether / ethyl acetate 20:1 to 5:1) to provide the title compound (6.2 g, quant.) as a yellow solid. This material was used in the following step without further purification.

### b) (3S)-6,7-dichloro-5-(2,6-difluorophenyl)-3-methyl-3H-1,4-benzodiazepin-2-amine

To a solution of aqueous ammonia (33 wt.%, 60 mL, 16.7 mmol) in methanol (12 mL) was added a solution of (3*S*)-6,7-dichloro-5-(2,6-difluorophenyl)-3-methyl-1,3-dihydro-1,4-benzodiazepine-2-thione (6.2 g, 14 mmol) in tetrahydrofuran (60 mL). The mixture was stirred at room temperature for 12 h, before all volatiles were removed under vacuum. The residue was purified by flash column chromatography (silica, dichloromethane / methanol 120:1 to 30: 1) to provide the title compound (4.5 g, 90 %) as a yellow solid. This material was used in the following step without further purification.

### c) (5S)-8,9-dichloro-7-(2,6-difluorophenyl)-5-methyl-5H-pyrimido[1,2-a][1,4]benzodiazepin-3-one

To a suspension of (3*S*)-6,7-dichloro-5-(2,6-difluorophenyl)-3-methyl-3H-1,4-benzodiazepin-2-amine (4.00 g, 11.3 mmol) in ethanol (57 mL) was added ethyl propiolate (5.72 mL, 56.5 mmol). The mixture was heated to 60 °C for 12 h, before being cooled to room temperature. The mixture was concentrated *in vacuo* then purified by flash column chromatography (Welch Ultimate XB-C18, 20-40 µm, 120 Å, 0.25% formic acid in water / acetonitrile) to provide the racemic title compound (2.95 g, 64 %) as yellow solid. Chiral separation by SFC (Chiralcel OD, 10 µm, 250x30mm, 0.1% NH₄OH in methanol, elution: 5-40% in CO₂ at 70 g/mL) afforded the enantiopure (-)-title compound (1.44 g, 31 %) as a white solid. MS: 406.0 ([{³⁵Cl, ³⁵Cl}M+H]⁺), 408.0 ([{³⁵Cl, ³⁷Cl}M+H]⁺), ESI pos.

### Example 5

### 2-bromo-8,9-dichloro-7-(2,6-difluorophenyl)-5H-pyrimido[1,2-a][1,4]benzodiazepin-3-one

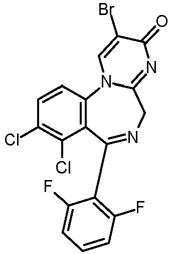

### 2-bromo-8,9-dichloro-7-(2,6-difluorophenyl)-5H-pyrimido[1,2-a][1,4]benzodiazepin-3-one

To a solution of 8,9-dichloro-7-(2,6-difluorophenyl)-5H-pyrimido[1,2-a][1,4]benzodiazepin-3-one (example 2, 30 mg, 0.077 mmol) in dichloromethane (0.6 mL) was added *N*-bromosuccinimide (15 mg, 0.084 mmol). The mixture was stirred at 22 °C for 16 h, before being concentrated *in vacuo.* The residue was purified by flash column chromatography (silica, 30-100% ethyl acetate in heptane) to give the title compound (8.7 mg, 24 %) as an off-white solid. MS: 470.0 ([{⁷⁹Br, ³⁵Cl, ³⁵Cl}M+H]⁺), 472.0 ([{⁷⁹Br, ³⁵Cl, ³⁷Cl or ⁸¹Br, ³⁵Cl, ³⁵Cl}M+H]⁺), 474.0 ([{⁸¹Br, ³⁵Cl, ³⁷Cl or ⁷⁹Br, ³⁷Cl, ³⁷Cl}M+H]⁺), ESI pos.

### Example 6

### (5S)-8-bromo-9-chloro-7-(2,6-difluorophenyl)-5-methyl-5H-pyrimido[1,2-a][1,4]benzodiazepin-3-one

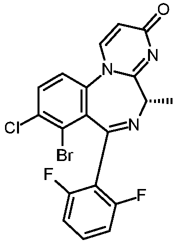

### a) (3S)-6-bromo-7-chloro-5-(2,6-difluorophenyl)-3-methyl-1,3-dihydro-1,4-benzodiazepine-2-thione

In analogy to experiment of example 3 a, (3S)-6-bromo-7-chloro-5-(2,6-difluorophenyl)-3-methyl-1,3-dihydro-1,4-benzodiazepin-2-one (building block Q, 3.30 g, 8.26 mmol) was converted into the title compound (2.72 g, 79 %) which was obtained as a yellow solid. MS: 415.0 ([{⁷⁹Br, ³⁵Cl}M+H]⁺), 417.0 ([{⁸¹Br, ³⁵Cl or ⁷⁹Br, ³⁷Cl}M+H]⁺), ESI pos.

### b) (3S)-6-bromo-7-chloro-5-(2,6-difluorophenyl)-3-methyl-3H-1,4-benzodiazepin-2-amine

In analogy to experiment of example 1 b, (3*S*)-6-bromo-7-chloro-5-(2,6-difluorophenyl)-3-methyl-1,3-dihydro-1,4-benzodiazepine-2-thione (400 mg, 0.962 mmol) was converted into the title compound (311 mg, 81 %) which was obtained as an orange solid. MS: 398.0 ([{⁷⁹Br, ³⁵Cl}M+H]⁺), 400.0 ([{⁸¹Br, ³⁵Cl or ⁷⁹Br, ³⁷Cl}M+H]⁺), ESI pos. The crude material was used in the following step without further purification.

### c) (5S)-8-bromo-9-chloro-7-(2,6-difluorophenyl)-5-methyl-5H-pyrimido[1,2-a][1,4]benzodiazepin-3-one

In analogy to experiment of example 1 c, (3*S*)-6-bromo-7-chloro-5-(2,6-difluorophenyl)-3-methyl-3H-1,4-benzodiazepin-2-amine (311 mg, 0.780 mmol) was converted into the (-)-title compound (73 mg, 21 %) which was obtained in enantiopure form after preparative chiral HPLC (Chiralpak AD, 20 µm, 500x50mm, 0.01 m NH₄OAc in ethanol, elution: 40% in heptane) as a white solid. MS: 450.0 ([{⁷⁹Br, ³⁵Cl}M+H]⁺), 452.0 ([{⁸¹Br, ³⁵Cl or ⁷⁹Br, ³⁷Cl}M+H]⁺), ESI pos.

### Example 7

### (5S)-8,9-dichloro-7-(2,6-difluorophenyl)-2,5-dimethyl-5H-pyrimido[1,2-a][1,4]benzodiazepin-3-one

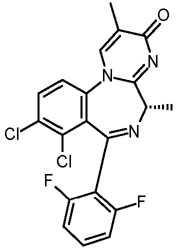

### a) (5S)-2-bromo-8,9-dichloro-7-(2,6-difluorophenyl)-5-methyl-5H-pyrimido[1,2-a][1,4]benzodiazepin-3-one

In analogy to experiment of example 5, (5S)-8,9-dichloro-7-(2,6-difluorophenyl)-5-methyl-5H-pyrimido[1,2-a][1,4]benzodiazepin-3-one (example 4, 580 mg, 1.43 mmol) was converted into the title compound (300 mg, 43 %) which was obtained as a yellow solid. This material was used in the following step without further purification.

### b) (5S)-8,9-dichloro-7-(2,6-difluorophenyl)-2,5-dimethyl-5H-pyrimido[1,2-a][1,4]benzodiazepin-3-one

A mixture of (5S)-2-bromo-8,9-dichloro-7-(2,6-difluorophenyl)-5-methyl-5H-pyrimido[1,2-a][1,4]benzodiazepin-3-one (260 mg, 0.536 mmol), potassium carbonate (222 mg, 1.61 mmol), 1,1 '-bis( diphenylphosphino )ferrocene-palladium(II)dichloride·CH₂Cl₂ (44 mg, 0.050 mmol) and methylboronic acid (160 mg, 2.68 mmol) in a 10:1 mixture of 1,4-dioxane/water (11 mL) was degassed by alternative evacuation and back filling with nitrogen. The reaction mixture was stirred under nitrogen at 60 °C for 16 h, before being concentrated under vacuum to roughly 1/10 of the initial volume. The residue was diluted with dichloromethane and washed with water and brine. The organic layer was separated, dried (Na₂SO₄), filtered and concentrated *in vacuo.* The residue was purified by flash column chromatography (silica, petroleum ether / ethyl acetate 3:1 to dichloromethane / methanol 20:1), followed by preparative HPLC (Phenomenex Synergi C18, 10 µm, 150x25 mm, 0.1% trifluoroacetic acid in water / acetonitrile) to provide the racemic title compound (40 mg, 18 %) as a white solid. Chiral separation by SFC (Cellucoat, 3 µm, 50×4.6mm, 0.05% DEA in methanol; gradient elution: 5% to 40% in CO₂, 3mL/min at 35 °C) afforded the enantiopure (-)-title compound (11.8 mg, 5 %) as a white solid. MS: 420.1 ([{³⁵Cl, ³⁵Cl}M+H]⁺), 422.1 ([{³⁵Cl, ³⁷Cl}M+H]⁺), ESI pos.

### Example 8

### (5S)-8-chloro-7-(2,6-difluorophenyl)-2,5,9-trimethyl-5H-pyrimido[1,2-a][1,4]benzodiazepin-3-one

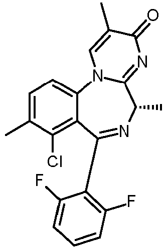

### (5S)-8-chloro-7-(2,6-difluorophenyl)-2,5,9-trimethyl-5H-pyrimido[1,2-a][1,4]benzodiazepin-3-one

A mixture of (5S)-2-bromo-8,9-dichloro-7-(2,6-difluorophenyl)-5-methyl-5H-pyrimido[1,2-a][1,4]benzodiazepin-3-one (example 7 a, 2.50 g, 5.15 mmol), cesium carbonate (5.04 g, 15.5 mmol), methylboronic acid (925 mg, 15.5 mmol) and (2-dicyclohexylphosphino-2',4',6'-triisopropyl-1,1'-biphenyl)[2-(2'-amino-1,1'-biphenyl)]palladium(II) methanesulfonate (436 mg, 0.520 mmol) in a 5:1 mixture of toluene and water (30 mL) was stirred under nitrogen at 80 °C for 16 h. The reaction mixture was allowed to cool to room temperature then filtered directly through a plug of celite. The filter cake was rinsed with ethyl acetate (50 mL) and the filtrate was concentrated *in vacuo.* The residue was purified by flash column chromatography (silica, petroleum ether / ethyl acetate 1:1 to 1: 2) followed by preparative HPLC (Phenomenex Luna C18, 15 µm, 150x40 mm, 0.1% trifluoroacetic acid in water / acetonitrile). The fractions containing the title compound were combined and washed with saturated aqueous NaHCO₃, then extracted with ethyl acetate. The organic layer was separated and washed with brine, dried (Na₂SO₄) and concentrated *in vacuo* to provide the racemic title compound (350 mg, 17 %) as a white solid. Chiral separation by SFC (Daicel Chiralcel OD, 10 µm, 250x30mm, 0.1% NH₄OH in ethanol, 50% in CO₂) afforded the enantiopure (-)-title compound (130 mg, 5 %) as a white solid. MS: 400.1 ([{³⁵Cl}M+H]+), 402.0 ([{³⁷Cl }M+H]⁺), ESI pos.

### Example 9

### (5S)-8-chloro-7-(2,6-difluorophenyl)-2,5-dimethyl-9-(trifluoromethyl)-5H-pyrimido [1,2-a] [1,4] benzodiazepin-3-one

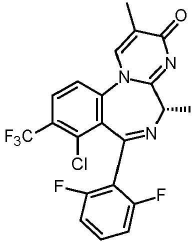

### a) (5S)-2-bromo-8-chloro-7-(2,6-difluorophenyl)-5-methyl-9-(trifluoromethyl)-5H-pyrimido[1,2-a][1-4]benzodiazepin-3-one

In analogy to experiment of example 5, (5S)-8-chloro-7-(2,6-difluorophenyl)-5-methyl-9-(trifluoromethyl)-5H-pyrimido[1,2-a][1,4]benzodiazepin-3-one (example 10, 4.80 g, 10.9 mmol) was converted into the title compound (5.6 g, 99 %) which was obtained as a yellow solid. This material was used in the following step without further purification.

### b) (5S)-8-chloro-7-(2,6-difluorophenyl)-2,5-dimethyl-9-(trifluoromethyl)-5H-pyrimido[1,2-a][1-4]benzodiazepin-3-one

A mixture of (5S)-2-bromo-8-chloro-7-(2,6-difluorophenyl)-5-methyl-9-(trifluoromethyl)-5H-pyrimido[1,2-a][1,4]benzodiazepin-3-one (2.50 g, 4.82 mmol), cesium carbonate (4.71 g, 14.5 mmol), methylboronic acid (1.44 g, 24.1 mmol) and methylboronic acid (925 mg, 15.5 mmol) and (2-dicyclohexylphosphino-2',4',6'-triisopropyl-1,1'-biphenyl)[2-(2'-amino-1,1'-biphenyl)]palladium(II) methanesulfonate (408 mg, 0.480 mmol) in a 5:1 mixture of toluene and water 5:1 (30 mL) was stirred under nitrogen at 40 °C for 24 h. The reaction mixture was allowed to cool to room temperature then filtered directly through a plug of celite. The filter cake was rinsed with ethyl acetate (50 mL) and the filtrate was concentrated *in vacuo.* The residue was purified by flash column chromatography (silica, petroleum ether / ethyl acetate 1:1 to 1: 2) followed by preparative HPLC (Waters Xbridge C18, 10 µm, 150x50 mm, 0.01 m NH₄HCO₃ in water / acetonitrile) to provide the racemic title compound (900 mg, 41 %) as a white solid. Chiral separation (using 600 mg of racemate) by SFC (Daicel Chiralpak AD, 10 µm, 250x30mm, 0.1% NH₄OH in isopropanol, 30% in CO₂) afforded the enantiopure (-)-title compound (312 mg, 21 %) as a white solid. MS: 454.3 ([{³⁵Cl}M+H]+), 456.3 ([{³⁷Cl}M+H]⁺), ESI pos.

### Example 10

### (5S)-8-chloro-7-(2,6-difluorophenyl)-5-methyl-9-(trifluoromethyl)-5H-pyrimido[1,2-a][1,4]benzodiazepin-3-one

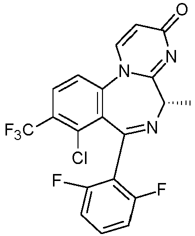

### a) (3S)-6-chloro-5-(2,6-difluorophenyl)-3-methyl-7-(trifluoromethyl)-1,3-dihydro-1,4-benzodiazepine-2-thione

In analogy to experiment of example 4 a, (3*S*)-6-chloro-5-(2,6-difluorophenyl)-3-methyl-7-(trifluoromethyl)-1,3-dihydro-1,4-benzodiazepin-2-one (building block V, 500 mg, 1.29 mmol) was converted into the title compound (490 mg, 93 %) which was obtained as a yellow solid. This material was used in the following step without further purification.

### b) (3S)-6-chloro-5-(2,6-difluorophenyl)-3-methyl-7-(trifluoromethyl)-3H-1,4-benzodiazepin-2-amine

A mixture of (3S)-6-chloro-5-(2,6-difluorophenyl)-3-methyl-7-(trifluoromethyl)-1,3-dihydro-1,4-benzodiazepine-2-thione (400 mg, 0.988 mmol) and aqueous ammonia (33 wt.%, 1 mL) in a 5:1 mixture of tetrahydrofuran and methanol (4.8 mL) was stirred at 35 °C for 16 h. All volatiles were removed under vacuum and the residue was triturated by addition of water. The resulting solid was collected by filtration and dried under high vacuum to provide the title compound (400 mg, quantitative) as a light yellow solid. This material was used in the following step without further purification.

### c) (5S)-8-chloro-7-(2,6-difluorophenyl)-5-methyl-9-(trifluoromethyl)-5H-pyrimido[1,2-a][1,4]benzodiazepin-3-one

To a solution of (3*S*)-6-chloro-5-(2,6-difluorophenyl)-3-methyl-7-(trifluoromethyl)-3H-1,4-benzodiazepin-2-amine (300 mg, 0.741 mmol) in ethanol (3.2 mL) was added ethyl propiolate (0.24 mL, 2.3 mmol). The reaction mixture was heated to 60 °C for 16 h, before being cooled to room temperature. The mixture was concentrated *in vacuo* then purified by flash column chromatography (silica, dichloromethane / methanol 50:1) to provide the racemic title compound (300 mg, 92 %) as an off-white solid. Chiral separation (using 70 mg of racemate) by SFC (Daicel Chiralpak AD, 10 µm, 250x30mm, ethanol, 50% in CO₂) afforded the enantiopure (-)-title compound (32.3 mg, 42 %) as a white solid. MS: 440.0 ([{³⁵Cl}M+H]+), 442.0 ([{³⁷Cl}M+H]⁺), ESI pos.

### Example 11

### (5S)-8-chloro-7-(2,6-difluorophenyl)-2-ethyl-5-methyl-9-(trifluoromethyl)-5H-pyrimido[1,2-a][1,4]benzodiazepin-3-one

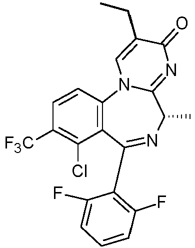

### a) (5S)-8-chloro-7-(2,6-difluorophenyl)-5-methyl-9-(trifluoromethyl)-2-vinyl-5H-pyrimido[1,2-a][1-4]benzodiazepin-3-one

A mixture of cesium carbonate (190 mg, 0.580 mmol), [1,1'- bis(diphenylphosphino)ferrocene]dichloropalladium(II) (20 mg, 0.03 mmol), (5*S*)-2-bromo-8-chloro-7-(2,6-difluorophenyl)-5-methyl-9-(trifluoromethyl)-5H-pyrimido[1,2-a][1,4]benzodiazepin-3-one (example 9 a, 100 mg, 0.190 mmol) and potassium vinyltrifluoroborate (130 mg, 0.970 mmol) in a 5:1 mixture of toluene and water (6 mL) was stirred under nitrogen at 80 °C for 16 h. The reaction mixture was allowed to cool to room temperature then concentrated *in vacuo.* The residue was purified by flash column chromatography (silica, petroleum ether / ethyl acetate 10:1 to 3:1) followed by preparative HPLC (Phenomenex Luna C18, 10 µm, 150x25mm, 0.1% trifluoroacetic acid in water / acetonitrile) to provide the title compound (68 mg, 75 %) as a white solid. MS: 466.2 ([{³⁵Cl}M+H]⁺), 468.1 ([{³⁷Cl }M+H]⁺), ESI pos.

### b) (5S)-8-chloro-7-(2,6-difluorophenyl)-2-ethyl-5-methyl-9-(trifluoromethyl)-5H-pyrimido[1,2-a][1-4]benzodiazepin-3-one

A mixture of (5S)-8-chloro-7-(2,6-difluorophenyl)-5-methyl-9-(trifluoromethyl)-2-vinyl-SH-pyrimido[1,2-a][1,4]benzodiazepin-3-one (78 mg, 0.17 mmol), 10 wt.% Pd/C (80 mg) and zinc bromide (110 mg, 0.490 mmol) in ethyl acetate (3 mL) and methanol (1 mL) was stirred under hydrogen at 25 °C for 2 h. The reaction mixture was filtered directly through a plug of celite. The filter cake was rinsed with ethyl acetate (10 mL) and the filtrate was concentrated *in vacuo.* The residue was purified by preparative HPLC (Phenomenex Gemini-NX C18, 3 µm, 75x30mm, 0.1% trifluoroacetic acid in water / acetonitrile) to provide the racemic title compound (30 mg, 38 %). Chiral separation by SFC (Daicel Chiralcel OJ, 10 µm, 250x30mm, 0.1% NH₄OH in *iso*-propanol, 30% in CO₂) afforded the enantiopure (-)-title compound (11 mg, 37 %) as a white solid. MS: 468.0 ([{³⁵Cl}M+H]+), 469.9 ([{³⁷Cl }M+H]⁺), ESI pos.

### Example 12

### 9-bromo-8-chloro-7-(2,6-difluorophenyl)-5H-pyrimido[1,2-a] [1,4]benzodiazepin-3-one

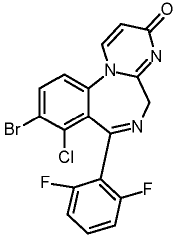

### a) 7-bromo-6-chloro-5-(2,6-difluorophenyl)-1,3-dihydro-1,4-benzodiazepine-2-thione

In analogy to experiment of example 4 a, 7-bromo-6-chloro-5-(2,6-difluorophenyl)-1,3-dihydro-1,4-benzodiazepin-2-one (building block A, 180 mg, 0.364 mmol, 78% purity) was converted into the title compound (140 mg, quant.) which was obtained as a yellow solid. MS: 401.0 ([{⁷⁹Br, ³⁵Cl}M+H]⁺), 403.0 ([{⁸¹Br, ³⁵Cl or ⁷⁹Br, ³⁷Cl}M+H]⁺), ESI pos.

### b) 7-bromo-6-chloro-5-(2,6-difluorophenyl)-3H-1,4-benzodiazepin-2-amine

In analogy to experiment of example 10 b, 7-bromo-6-chloro-5-(2,6-difluorophenyl)-1,3-dihydro-1,4-benzodiazepine-2-thione (140 mg, 0.310 mmol, 88% purity) was converted into the title compound (155 mg, quant.) which was obtained as an orange solid. MS: 384.0 ([{⁷⁹Br, ³⁵Cl}M+H]⁺), 386.0 ([{⁸¹Br, ³⁵Cl or ⁷⁹Br, ³⁷Cl}M+H]⁺), ESI pos. This crude material was used in the following step without further purification.

### c) 9-bromo-8-chloro-7-(2,6-difluorophenyl)-5H-pyrimido[1,2-a][1-4]benzodiazepin-3-one

In analogy to experiment of example 4 c, 7-bromo-6-chloro-5-(2,6-difluorophenyl)-3H-1,4-benzodiazepin-2-amine (155 mg, 0.360 mmol, 83% purity) was converted into the title compound (12 mg, 8 %) which was obtained as a white solid after purification by preparative HPLC (Phenomenex Synergi C18, 10 µm, 150x20 mm, 0.1% trifluoroacetic acid in water / acetonitrile). MS: 436.0 ([{⁷⁹Br, ³⁵Cl}M+H]⁺), 438.0 ([{⁸¹Br, ³⁵Cl or ⁷⁹Br, ³⁷Cl}M+H]⁺), ESI pos.

### Example 13

### (5S)-9-bromo-8-chloro-7-(2,6-difluorophenyl)-5-methyl-5H-pyrimido[1,2-a][1,4]benzodiazepin-3-one

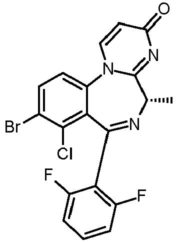

### a) (3S)-7-bromo-6-chloro-5-(2,6-difluorophenyl)-3-methyl-1,3-dihydro-1,4-benzodiazepine-2-thione

In analogy to experiment of example 4 a, (3*S*)-7-bromo-6-chloro-5-(2,6-difluorophenyl)-3-methyl-1,3-dihydro-1,4-benzodiazepin-2-one (building block L, 385 mg, 0.960 mmol) was converted into the title compound (342 mg, 85 %) which was obtained as a yellow solid. MS: 414.8 ([{⁷⁹Br, ³⁵Cl}M+H]⁺), 416.8 ([{⁸¹Br, ³⁵Cl or ⁷⁹Br, ³⁷Cl}M+H]⁺), ESI pos.

### b) (3S)-7-bromo-6-chloro-5-(2,6-difluorophenyl)-3-methyl-3H-1,4-benzodiazepin-2-amine

In analogy to experiment of example 10 b, (3*S*)-7-bromo-6-chloro-5-(2,6-difluorophenyl)-3-methyl-1,3-dihydro-1,4-benzodiazepine-2-thione (342 mg, 0.820 mmol) was converted into the title compound (380 mg, quant.) which was obtained as yellow solid. MS: 398.0 ([{⁷⁹Br, ³⁵Cl}M+H]⁺), 400.0 ([{⁸¹Br, ³⁵Cl or ⁷⁹Br, ³⁷Cl}M+H]⁺), ESI pos. This material was used in the following step without further purification.

### c) (5S)-9-bromo-8-chloro-7-(2,6-difluorophenyl)-5-methyl-5H-pyrimido[1,2-a][1,4]benzodiazepin-3-one

In analogy to experiment of example 4 c, (3*S*)-7-bromo-6-chloro-5-(2,6-difluorophenyl)-3-methyl-3H-1,4-benzodiazepin-2-amine (380 mg, 0.950 mmol) was converted into the racemic title compound which was obtained as a light yellow solid (260 mg, 61 %) after purification by preparative HPLC (Phenomenex Synergi C18, 10 µm, 150x25 mm, 0.1% trifluoroacetic acid in water / acetonitrile). Chiral separation (using 60 mg of racemate) by SFC (Daicel Chiralcel OJ, 10 µm, 250x30mm, 0.1% NH₄OH in methanol, 30% in CO₂) afforded the enantiopure (-)-title compound (17 mg, 28 %) as a white solid. MS: 449.9 ([{⁷⁹Br, ³⁵Cl}M+H]⁺), 451.9 ([{⁸¹Br, ³⁵Cl or ⁷⁹Br, ³⁷Cl}M+H]⁺), ESI pos.

### Example 14

### (5S)-8,9-dichloro-7-(2,6-difluorophenyl)-2-ethyl-5-methyl-5H-pyrimido[1,2-a][1,4]benzodiazepin-3-one

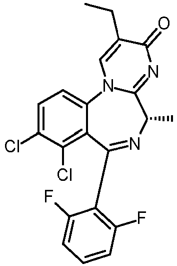

### a) (5S)-8,9-dichloro-7-(2,6-difluorophenyl)-5-methyl-2-vinyl-5H-pyrimido[1,2-a][1,4]benzodiazepin-3-one

A mixture of potassium acetate (243.0 mg, 2.48 mmol), [1,1'-bis(diphenylphosphino)ferrocene]dichloropalladium(II) (90 mg, 0.12 mmol), (5*S*)-2-bromo-8,9-dichloro-7-(2,6-difluorophenyl)-5-methyl-5H-pyrimido[1,2-a][1,4]benzodiazepin-3-one (example 7 a, 600 mg, 1.24 mmol) and potassium vinyltrifluoroborate (180 mg, 1.34 mmol) in a 5:1 mixture of toluene and water (12 mL) was stirred under nitrogen at 80°C for 16 h. The reaction mixture was allowed to cool to room temperature then diluted with water (20 mL). The resulting suspension was extracted with ethyl acetate (2 × 40 mL). The combined organic layers were dried (Na₂SO₄) and concentrated *in vacuo.* The residue was purified by flash column chromatography (silica, petroleum ether / ethyl acetate 10:1 to 0:1) to give the title compound (387 mg, 72 %) as a light yellow solid. MS: 432.1 ([{³⁵Cl, ³⁵Cl}M+H]⁺), 434.1 ([{³⁵Cl, ³⁷Cl}M+H]⁺), ESI pos.

### b) (5S)-8,9-dichloro-7-(2,6-difluorophenyl)-2-ethyl-5-methyl-5H-pyrimido[1,2-a][1,4]benzodiazepin-3-one

In analogy to experiment of example 11 b, (5S)-8,9-dichloro-7-(2,6-difluorophenyl)-5-methyl-2-vinyl-5H-pyrimido[1,2-a][1,4]benzodiazepin-3-one (105 mg, 0.243 mmol) was converted into the racemic title compound (65 mg, 65 %) which was obtained as a white solid after purification by preparative HPLC (Phenomenex Synergi C18, 10 µm, 150x25mm, 0.1% trifluoroacetic acid in water / acetonitrile). Chiral separation by SFC (Daicel Chiralpak AD, 10 µm, 250x30mm, 0.1% NH₄OH in iso-propanol, 45% in CO2) afforded the enantiopure (-)-title compound (13 mg, 12 %) as a white solid. MS: 434.1 ([{³⁵Cl, ³⁵Cl}M+H]⁺), 436.1 ([{³⁵Cl, ³⁷Cl}M+H]⁺), ESI pos.

### Example 15

### 2,9-dibromo-8-chloro-7-(2,6-difluorophenyl)-5H-pyrimido[1,2-a][1,4] benzodiazepin-3-one

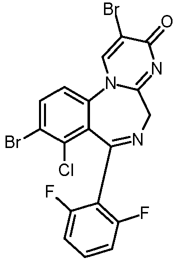

### 2,9-dibromo-8-chloro-7-(2,6-difluorophenyl)-5H-pyrimido[1,2-a][1,4]benzodiazepin-3-one

To a solution of 9-bromo-8-chloro-7-(2,6-difluorophenyl)-5H-pyrimido[1,2-a][1,4]benzodiazepin-3-one (example 12, 348 mg, 0.800 mmol) in *N,N-*dimethylformamide (5 mL) was added a solution of *N*-bromosuccinimide (165 mg, 0.930 mmol) in *N,N*-dimethylformamide (2 mL). The mixture was stirred at 50 °C for 2 h before being cooled to room temperature. The mixture was diluted with water (20 mL) and extracted with ethyl acetate (2 × 30 mL). The combined organic layers were dried (Na₂SO₄), filtered and concentrated *in vacuo.* The residue was purified by preparative HPLC (Phenomenex Luna C18, 15 µm, 150x40mm, 0.1% trifluoroacetic acid in water / acetonitrile) to provide the title compound (50 mg, 11 %) as a yellow solid. MS: 513.9 ([{⁷⁹Br, ⁷⁹Br, ³⁵Cl}M+H]⁺), 515.9 ([{⁷⁹Br, ⁷⁹Br, ³⁷Cl or ⁷⁹Br, ⁸¹Br, ³⁵Cl}M+H]⁺), 517.9 ([{⁷⁹Br, ⁸¹Br, ³⁷Cl or ⁸¹Br, ⁸¹Br, ³⁵Cl}M+H]⁺), ESI pos.

### Example 16

### (5S)-2,9-dibromo-8-chloro-7-(2,6-difluorophenyl)-5-methyl-5H-pyrimido[1,2-a][1,4]benzodiazepin-3-one

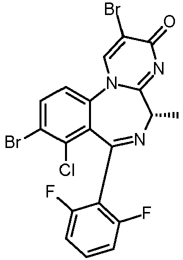

### (5S)-2,9-dibromo-8-chloro-7-(2,6-difluorophenyl)-5-methyl-5H-pyrimido[1,2-a][1,4]benzodiazepin-3-one

In analogy to experiment of example 15, (5S)-9-bromo-8-chloro-7-(2,6-difluorophenyl)-5-methyl-5H-pyrimido[1,2-a][1,4]benzodiazepin-3-one (Example 13, 195 mg, 0.433 mmol) was converted into the racemic title compound (83 mg, 36 %) which was obtained as a light yellow solid after flash column chromatography (silica, petroleum ether / ethyl acetate 5:1 to 1:3). Chiral separation (using 100 mg of racemate) by SFC (Daicel Chiralcel OD, 10 µm, 250x30mm, 0.1% NH₄OH in methanol, 50% in CO₂) afforded the enantiopure (-)-title compound (40 mg, 10 %) as an off-white solid. MS: 528.1 ([{⁷⁹Br, ⁷⁹Br, ³⁵Cl}M+H]⁺), 530.1 ([{⁷⁹Br, ⁷⁹Br, ³⁷Cl or ⁷⁹Br, ⁸¹Br, ³⁵Cl}M+H]⁺), 532.1 ([{⁷⁹Br, ⁸¹Br, ³⁷Cl or ⁸¹Br, ⁸¹Br, ³⁵Cl}M+H]⁺), ESI pos.

### Example 17

### 8-bromo-9-chloro-7-(2,6-difluorophenyl)-5H-pyrimido[1,2-a] [1,4]benzodiazepin-3-one

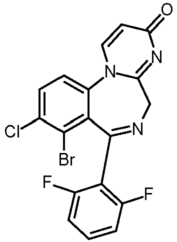

### a) 6-bromo-7-chloro-5-(2,6-difluorophenyl)-1,3-dihydro-1,4-benzodiazepine-2-thione

To a solution of 6-bromo-7-chloro-5-(2,6-difluorophenyl)-1,3-dihydro-1,4-benzodiazepin-2-one (building block Z, 240 mg, 0.400 mmol, 64% purity) in toluene (5 mL) was added Lawesson's reagent (212 mg, 0.525 mmol). The mixture was heated to 100 °C for 5 h until complete conversion as judged by TLC (petroleum ether / ethyl acetate 2:1). The reaction mixture was cooled to room temperature then concentrated *in vacuo.* The residue was purified by flash column chromatography (silica, petroleum ether / ethyl acetate 1:0 to 3:1) to provide the title compound (231 mg, quant.) as a yellow solid. MS: 400.9 ([{⁷⁹Br, ³⁵Cl}M+H]⁺), 402.9 ([{⁷⁹Br, ³⁷Cl or ⁸¹Br, ³⁵Cl}M+H]⁺), ESI pos.

### b) 6-bromo-7-chloro-5-(2,6-difluorophenyl)-3H-1,4-benzodiazepin-2-amine

To a solution of aqueous ammonia (33 wt.%, 0.5 mL, 8.5 mmol) in methanol (1 mL) was added a solution of 6-bromo-7-chloro-5-(2,6-difluorophenyl)-1,3-dihydro-1,4-benzodiazepine-2-thione (231 mg, 0.400 mmol) in tetrahydrofuran (5 mL). The mixture was stirred at 60 °C for 16 h, before all volatiles were removed under vacuum. The resulting crude title compound (248 mg, quant.) obtained as a yellow solid was used in the following step without further purification. MS: 383.9 ([{⁷⁹Br, ³⁵Cl}M+H]⁺), 385.9 ([{⁷⁹Br, ³⁵Cl or ⁸¹Br, ³⁵Cl}M+H]⁺), ESI pos.

### c) 8-bromo-9-chloro-7-(2,6-difluorophenyl)-5H-pyrimido[1,2-a][1,4]benzodiazepin-3-one

In analogy to experiment of example 4 c, 6-bromo-7-chloro-5-(2,6-difluorophenyl)-3H-1,4-benzodiazepin-2-amine (248 mg, 0.400 mmol) was converted into the title compound (48 mg, 27 %) which was obtained as a white solid after purification by preparative HPLC (Phenomenex Luna C18, 10 µm, 150x25mm, 0.225% formic acid in water / acetonitrile). MS: 436.1 ([{⁷⁹Br, ³⁵Cl }M+H]⁺), 438.1 ([{⁸¹Br, ³⁵Cl or ⁷⁹Br, ³⁷Cl}M+H]⁺), ESI pos.

### Example 18

### (5S)-8-chloro-7-(2,6-difluorophenyl)-2-[(E)-2-ethoxyvinyl]-5-methyl-9-(trifluoromethyl)-5H-pyrimido[1,2-a][1,4]benzodiazepin-3-one

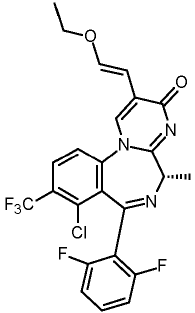

### (5S)-8-chloro-7-(2,6-difluorophenyl)-2-[(E)-2-ethoxyvinyl]-5-methyl-9-(trifluoromethyl)-5H-pyrimido[1,2-a][1,4]benzodiazepin-3-one

Racemic mixture 8-chloro-7-(2,6-difluorophenyl)-5-methyl-2-[(*E*)-2-ethoxyvinyl]-9-(trifluoromethyl)-5H-pyrimido[1,2-a][1,4]benzodiazepin-3-one (example 20 a, 70 mg, 0.14 mmol) was separated by chiral SFC (Daicel Chiralcel IC, 10 µm, 250x30 mm, 0.1% NH₄OH in *iso*-propanol, 50% in CO₂) to provide the enantiopure (-)-title compound (19 mg, 27 %) as an off-white solid. MS: 510.3 ([{³⁵Cl}M+H]⁺), 512.3 ([³⁷Cl}M+H]⁺), ESI pos.

### Example 19

### (5S)-8,9-dichloro-7-(2,6-difluorophenyl)-2-[(E)-2-ethoxyvinyl]-5-methyl-5H-pyrimido[1,2-a][1,4]benzodiazepin-3-one

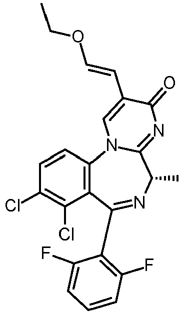

### (5S)-8,9-dichloro-7-(2,6-difluorophenyl)-2-[(E)-2-ethoxyvinyl]-5-methyl-5H-pyrimido[1,2-a][1-4]benzodiazepin-3-one

Racemic mixture 8,9-dichloro-7-(2,6-difluorophenyl)-2-[(*E*)-2-ethoxyvinyl]-5-methyl-5H-pyrimido[1,2-a][1,4]benzodiazepin-3-one (example 21 a, 70 mg, 0.15 mmol) was separated by chiral SFC (Daicel Chiralcel AD, 10 µm, 250x30 mm, 0.1% NH₄OH in Ethanol, 40% in CO₂) to provide the enantiopure (-)-title compound (18 mg, 26 %) as a light yellow solid. MS: 476.0 ([{³⁵Cl}M+H]⁺), 478.0 ([{³⁷Cl}M+H]⁺), ESI pos.

### Example 20

### (5S)-8-chloro-7-(2,6-difluorophenyl)-2-(2-ethoxyethyl)-5-methyl-9-(trifluoromethyl)-5H-pyrimido[1,2-a][1,4]benzodiazepin-3-one

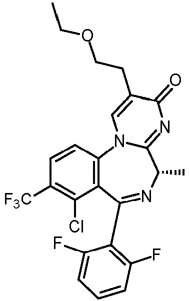

### a) 8-chloro-7-(2,6-difluorophenyl)-2-[(E)-2-ethoxyvinyl]-5-methyl-9-(trifluoromethyl)-5H-pyrimido[1,2-a][1,4]benzodiazepin-3-one

A mixture of cesium carbonate (950 mg, 2.92 mmol), [1,1'-bis(diphenylphosphino)ferrocene]dichloropalladium(II) (70 mg, 0.10 mmol), (5*S*)-2-bromo-8-chloro-7-(2,6-difluorophenyl)-5-methyl-9-(trifluoromethyl)-5H-pyrimido[1,2-a][1,4]benzodiazepin-3-one (example 9 a, 500 mg, 965 mmol) and 2-[(*E*)-2-ethoxyvinyl]-4,4,5,5-tetramethyl-1,3,2-dioxaborolane (961 mg, 4.85 mmol) in a 5:1 mixture of 1,4-dioxane and water (30 mL) was stirred under nitrogen at 80 °C for 16 h. The mixture was allowed to cool to room temperature then quenched by addition of water (20 mL). The product was extracted with ethyl acetate (3 × 30 mL). The combined organic layers were dried (Na₂SO₄), filtered and concentrated *in vacuo.* The residue was purified by flash column chromatography (silica, petroleum ether / ethyl acetate 1:0 to 1:2) followed by preparative HPLC (Phenomenex Luna C18, 15 µm, 150x40mm, 0.1% trifluoroacetic acid in water / acetonitrile) to provide the title compound (236 mg, 48 %) as an off-white solid. MS: 510.3 ([{³⁵Cl}M+H]⁺), 512.3 ([{³⁷Cl }M+H]⁺), ESI pos.

### b) (5S)-8-chloro-7-(2,6-difluorophenyl)-2-(2-ethoxyethyl)-5-methyl-9-(trifluoromethyl)-5H-pyrimido[1,2-a][1,4]benzodiazepin-3-one

In analogy to experiment of example 11 b, 8-chloro-7-(2,6-difluorophenyl)-2-[(E)-2-ethoxyvinyl]-5-methyl-9-(trifluoromethyl)-5H-pyrimido[1,2-a][1,4]benzodiazepin-3-one (166 mg, 0.325 mmol) was converted into the racemic title compound (78 mg, 47 %) after purification by preparative HPLC (Phenomenex Luna C18, 3 µm, 75x30mm, 0.1% trifluoroacetic acid in water / acetonitrile). Chiral separation by SFC (Daicel Chiralcel OD, 10 µm, 250x30mm, 0.1% NH₄OH in Ethanol, 30% in CO₂ and Daicel Chiralcel OJ, 10 µm, 250x30mm, 0.1% NH₄OH in *iso*-propanol, 25% in CO₂) afforded the enantiopure (-)-title compound (10 mg, 13 %) as a light yellow solid. MS: 512.3 ([{³⁵Cl}M+H]⁺), 514.3 ([³⁷Cl}M+H]⁺), ESI pos.

### Example 21

### (5S)-8,9-dichloro-7-(2,6-difluorophenyl)-2-(2-ethoxyethyl)-5-methyl-5H-pyrimido[1,2-a][1,4]benzodiazepin-3-one

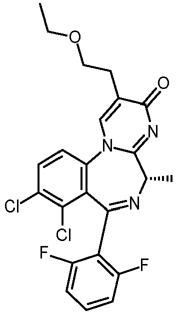

### a) 8,9-dichloro-7-(2,6-difluorophenyl)-2-[(E)-2-ethoxyvinyl]-5-methyl-5H-pyrimido[1,2-a][1,4]benzodiazepin-3-one

In analogy to experiment of example 20 a, 2-bromo-8,9-dichloro-7-(2,6-difluorophenyl)-5-methyl-5H-pyrimido[1,2-a][1,4]benzodiazepin-3-one (example 7 a, 500 mg, 1.03 mmol) using potassium acetate instead of cesium carbonate was converted into the title compound (203 mg, 40 %) which was obtained as a light yellow solid. MS: 476.3 ([{³⁵Cl, ³⁵Cl}M+H]⁺), 478.3 ([{³⁵Cl, ³⁷Cl}M+H]⁺), ESI pos.

### b) (5S)-8,9-dichloro-7-(2,6-difluorophenyl)-2-(2-ethoxyethyl)-5-methyl-5H-pyrimido[1,2-a][1,4]benzodiazepin-3-one

In analogy to experiment of example 20 b, 8,9-dichloro-7-(2,6-difluorophenyl)-2-[(*E*)-2-ethoxyvinyl]-5-methyl-5H-pyrimido[1,2-a][1,4]benzodiazepin-3-one (133 mg, 0.280 mmol) was converted into the racemic title compound (92 mg, 90 %) after purification by preparative HPLC (Phenomenex Synergi C18, 10 µm, 150x25mm, 0.1% trifluoroacetic acid in water / acetonitrile). Chiral separation by SFC (Daicel Chiralcel OJ, 10 µm, 250x30mm, 0.1% NH₄OH in Ethanol, 40% in CO₂ and Daicel Chiralcel OJ, 10 µm, 250x30mm, 0.1% NH₄OH in iso-propanol, 30% in CO₂) afforded the enantiopure (-)-title compound (17 mg, 18 %) as a white solid. MS: 478.2 ([{³⁵Cl, ³⁵Cl}M+H]⁺), 480.2 ([{³⁵Cl, ³⁷Cl}M+H]⁺), ESI pos.

### Example 22

### 8,9-dichloro-7-(2,6-difluorophenyl)-2-[(E)-2-ethoxyvinyl]-5H-pyrimido[1,2-a][1,4]benzodiazepin-3-one

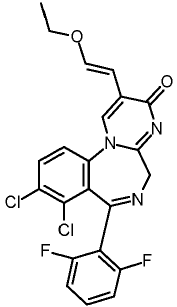

### 8,9-dichloro-7-(2,6-difluorophenyl)-2-[(E)-2-ethoxyvinyl]-5H-pyrimido[1,2-a][1,4]benzodiazepin-3-one

In analogy to experiment of example 21 a, 2-bromo-8,9-dichloro-7-(2,6-difluorophenyl)-5H-pyrimido[1,2-a][1,4]benzodiazepin-3-one (example 5, 200 mg, 0.425 mmol) was converted into the title compound (48 mg, 24 %) which was obtained as a light yellow solid. MS: 461.8 ([{³⁵Cl, ³⁵Cl}M+H]⁺), 463.8 ([{³⁵Cl, ³⁷Cl}M+H]⁺), ESI pos.

### Example 23

### (5S)-2,8-dibromo-9-chloro-7-(2,6-difluorophenyl)-5-methyl-5H-pyrimido[1,2-a][1,4]benzodiazepin-3-one

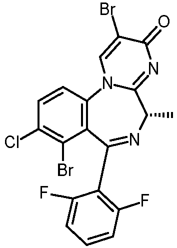

### (5S)-2,8-dibromo-9-chloro-7-(2,6-difluorophenyl)-5-methyl-5H-pyrimido[1,2-a][1,4]benzodiazepin-3-one

To a solution of (5S)-8-bromo-9-chloro-7-(2,6-difluorophenyl)-5-methyl-5H-pyrimido[1,2-a][1,4]benzodiazepin-3-one (example 6, 250 mg, 0.555 mmol) in *N,N-*dimethylformamide (4.0 mL) was added a solution of N-bromosuccinimide (116 mg, 0.650 mmol) in *N,N*-dimethylformamide (1.0 mL). The reaction mixture was stirred at 50 °C for 2 h. The mixture was cooled to room temperature then quenched by addition of water (10 mL). The product was extracted with ethyl acetate (3 × 10 mL). The combined organic layers were dried (Na₂SO₄), filtered and concentrated *in vacuo.* The residue was purified by flash column chromatography (silica, petroleum ether / ethyl acetate 1:0 to 1:2) to provide the racemic title compound (69 mg, 23 %) as a light yellow solid. Chiral separation by SFC (Daicel Chiralcel OJ, 10 µm, 250x30mm, 0.1% NH₄OH in methanol, 50% in CO₂) afforded the enantiopure (-)-title compound (32 mg, 11 %) as a light yellow solid. MS: 528.1 ([{⁷⁹Br, ⁷⁹Br, ³⁵Cl}M+H]⁺), 530.1 ([{⁷⁹Br, ⁷⁹Br, ³⁷Cl or ⁷⁹Br, ⁸¹Br, ³⁵Cl}M+H]⁺), 532.1 ([{⁸¹Br, ⁸¹Br, ³⁵Cl or ⁷⁹Br, ⁸¹Br, ³⁷Cl}M+H]⁺), ESI pos.

### Example 24

### 8,9-dichloro-7-(2,6-difluorophenyl)-2-(2-ethoxyethyl)-5H-pyrimido[1,2-a][1,4]benzodiazepin-3-one

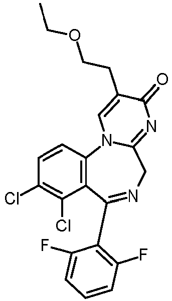

### a) 8,9-dichloro-7-(2,6-difluorophenyl)-2-(2-ethoxyethyl)-2,5-dihydro-1H-pyrimido[1,2-a][1,4]benzodiazepin-3-one

In analogy to experiment of example 21 b, 8,9-dichloro-7-(2,6-difluorophenyl)-2-[(E)-2-ethoxyvinyl]-5H-pyrimido[1,2-a][1,4]benzodiazepin-3-one (example 22, 28 mg, 0.061 mmol) was converted into the title compound (25 mg, 89 %) which was obtained as a yellow solid. MS: 466.2 ([{³⁵Cl, ³⁵Cl}M+H]⁺), 468.2 ([{³⁵Cl, ³⁷Cl}M+H]⁺), ESI pos.

### b) 8,9-dichloro-7-(2,6-difluorophenyl)-2-(2-ethoxyethyl)-5H-pyrimido[1,2-a][1,4]benzodiazepin-3-one

To a solution of 8,9-dichloro-7-(2,6-difluorophenyl)-2-(2-ethoxyethyl)-2,5-dihydro-1H-pyrimido[1,2-a][1,4]benzodiazepin-3-one (27 mg, 0.058 mmol) in dichloromethane (10 mL) was added manganese dioxide (36.0 mg, 0.41 mmol). The reaction mixture was stirred at 50 °C for 1 h, before being filtered directly through a plug of celite. The filter cake was rinsed with dichloromethane (20 mL) and the filtrate was concentrated *in vacuo.* The residue was purified by preparative HPLC (Phenomenex Synergi C18, 10 µm, 150x25mm, 0.1% trifluoroacetic acid in water / acetonitrile) to provide the title compound (7 mg, 26 %) as a light yellow solid. MS: 464.2 ([{³⁵Cl, ³⁵Cl}M+H]⁺), 466.2 ([{³⁵Cl, ³⁷Cl}M+H]⁺), ESI pos.

### Example 25

### 8-chloro-7-(2,6-difluorophenyl)-2-[(E)-2-ethoxyvinyl]-9-(trifluoromethyl)-5H-pyrimido[1,2-a][1,4]benzodiazepin-3-one

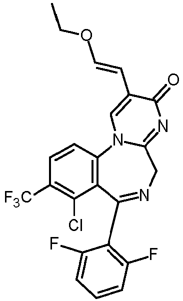

### a) 6-chloro-5-(2,6-difluorophenyl)-7-(trifluoromethyl)-1,3-dihydro-1,4-benzodiazepine-2-one

To a solution of 6-chloro-5-(2,6-difluorophenyl)-7-iodo-1,3-dihydro-1,4-benzodiazepin-2-one (building block H, 203 mg, 0.469 mmol) in *N,N*-dimethylformamide (5.0 mL) was added iodocopper (178 mg, 0.938 mmol), methyl 2,2-difluoro-2-fluorosulfonyl-acetate (225 mg, 1.17 mmol) and (1*R*,2*R*)-*N*,*N*-dimethylcyclohexane-1,2-diamine (133 mg, 0.938 mmol). The reaction mixture was heated to 70 °C for 16 h, before being diluted with ethyl acetate (30 mL). The resulting suspension was filtered directly through a plug of celite. The filter cake was rinsed with ethyl acetate (20 mL) and the filtrate was washed with water (20 mL) and brine (20 mL), dried (Na₂SO₄) and concentrated *in vacuo.* The crude was purified by preparative HPLC (Phenomenex Synergi C18, 0.1 % trifluoroacetic acid in water / acetonitrile) to provide the title compound (120 mg, 69 %) as a dark red oil. MS: 375.0 ([M+H]+), ESI pos.

### b) 6-chloro-5-(2,6-difluorophenyl)-7-(trifluoromethyl)-1,3-dihydro-1,4-benzodiazepine-2-thione

In analogy to experiment 4 a, 6-chloro-5-(2,6-difluorophenyl)-7-(trifluoromethyl)-1,3-dihydro-1,4-benzodiazepine-2-one (1.80 g, 4.80 mmol) was converted into the title compound (1.50 g, 80 %) as a light yellow solid. MS: 391.0 ([{³⁵Cl}M+H]⁺), 393.0 ([³⁷Cl}M+H]⁺), ESI pos.

### c) 6-chloro-5-(2,6-difluorophenyl)-7-(trifluoromethyl)-3H-1,4-benzodiazepin-2-amine

In analogy to experiment 4 b, 6-chloro-5-(2,6-difluorophenyl)-7-(trifluoromethyl)-1,3-dihydro-1,4-benzodiazepine-2-thione (600 mg, 1.54 mmol) was converted into the title compound (500 mg, 87 %) which was obtained as a white solid. MS: 374.0 ([{³⁵Cl}M+H]⁺), 376.1 ([³⁷Cl}M+H]⁺), ESI pos.

### d) 8-chloro-7-(2,6-difluorophenyl)-9-(trifluoromethyl)-5H-pyrimido[1,2-a][1,4]benzodiazepin-3-one

In analogy to experiment 1 c, 6-chloro-5-(2,6-difluorophenyl)-7-(trifluoromethyl)-3H-1,4-benzodiazepin-2-amine (600 mg, 1.61 mmol) was converted into the title compound (420 mg, 61 %) which was obtained as a light yellow solid. MS: 426.0 ([{³⁵Cl}M+H]⁺), 428.0 ([³⁷Cl}M+H]⁺), ESI pos.

### e) 2-bromo-8-chloro-7-(2,6-difluorophenyl)-9-(trifluoromethyl)-5H-pyrimido[1,2-a][1,4]benzodiazepin-3-one

In analogy to experiment 5, 8-chloro-7-(2,6-difluorophenyl)-9-(trifluoromethyl)-5H-pyrimido[1,2-a][1,4]benzodiazepin-3-one (520 mg, 1.22 mmol) was converted into the title compound (195 mg, 32 %) which was obtained after trituration and filtration from petroleum ether as a white solid. MS: 503.8 ([{⁷⁹Br, ³⁵Cl}M+H]⁺), 505.8 ([{⁷⁹Bᵣ, ¹⁷Cl or ⁸¹Br, ³⁵Cl} M+H]⁺), ESI pos.

### f) 8-chloro-7-(2,6-difluorophenyl)-2-[(E)-2-ethoxyvinyl]-9-(trifluoromethyl)-5H-pyrimido[1,2-a][1-4]benzodiazepin-3-one

In analogy to experiment of example 20 a, 2-bromo-8-chloro-7-(2,6-difluorophenyl)-9-(trifluoromethyl)-5H-pyrimido[1,2-a][1,4]benzodiazepin-3-one (195 mg, 386 mmol) was converted into the title compound (5.1 mg, 3%) which was obtained as a white solid after purification by preparative HPLC (Phenomenex Synergi C18, 10 µm, 150x25mm, 0.1% trifluoroacetic acid in water / acetonitrile). MS: 496.1 ([{³⁵Cl}M+H]⁺), 498.1 ([³⁷Cl}M+H]⁺), ESI pos.

### Example 26

### 8-chloro-7-(2,6-difluorophenyl)-2-(2-ethoxyethyl)-9-(trifluoromethyl)-5H-pyrimido[1,2-a][1,4]benzodiazepin-3-one

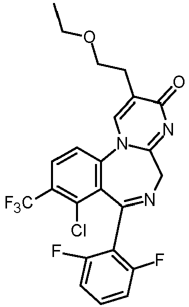

### 8-chloro-7-(2,6-difluorophenyl)-2-(2-ethoxyethyl)-9-(trifluoromethyl)-5H-pyrimido[1,2-a][1,4]benzodiazepin-3-one

In analogy to experiment of example 11 b, 8-chloro-7-(2,6-difluorophenyl)-2-[(*E*)-2-ethoxyvinyl]-9-(trifluoromethyl)-5H-pyrimido[1,2-a][1,4]benzodiazepin-3-one was converted into the title compound (4.5 mg, 6 %) which was obtained as white solid after purification by preparative HPLC (Phenomenex Gemini-NX C18, 3 µm, 75x30mm, 0.225% formic acid in water / acetonitrile) followed by preparative TLC (DCM / methanol / ethyl acetate 20:1:4). MS: 498.1 ([{³⁵Cl}M+H]⁺), 500.1 ([{³⁷Cl}M+H]⁺), ESI pos.

### Example 27

### (5S)-8-chloro-7-(2,6-difluorophenyl)-N,5-dimethyl-3-oxo-9-(trifluoromethyl)-5H-pyrimido [1,2-a] [1,4] benzodiazepine-2-carboxamide

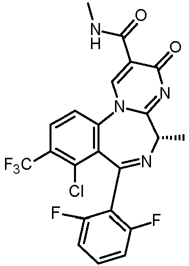

### a) methyl 8-chloro-7-(2,6-difluorophenyl)-5-methyl-3-oxo-9-(trifluoromethyl)-5H-pyrimido[1,2-a][1,4]benzodiazepine-2-carboxylate

To a solution of 2-bromo-8-chloro-7-(2,6-difluorophenyl)-5-methyl-9-(trifluoromethyl)-5H-pyrimido[1,2-a][1,4]benzodiazepin-3-one (example 9 a, 1.56 g, 3.01 mmol) in a 1:1 mixture of methanol and *N*,*N*-dimethylformamide (70 mL) was added palladium(II) acetate (405 mg, 1.80 mmol) and 1,1'-bis(diphenylphosphino)ferrocene (1.00 g, 1.80 mmol). The mixture was heated to 70 °C for 16 h under a CO atmosphere. The mixture was combined with another batch (1.64 g, 3.16 mmol), filtered through a sintered funnel then the filtrate was concentrated *in vacuo.* The residue was purified by flash column chromatography (silica, petroleum ether / ethyl acetate 1:1 and dichloromethane / methanol 50:1 to 20: 1) followed by preparative HPLC (Phenomenex Luna C18, 15 µm, 250x70mm, 0.225% formic acid in water / acetonitrile). The fractions containing the title compound were combined and extracted with ethyl acetate. The organic phase was washed with brine, dried (Na₂SO₄), filtered and concentrated *in vacuo* to provide the title compound (2.50 g, 81 %) as a red solid. MS: 498.0 ([{³⁵Cl}M+H]⁺), 500.0 ([³⁷Cl}M+H]⁺), ESI pos.

### b) 8-chloro-7-(2,6-difluorophenyl)-5-methyl-3-oxo-9-(trifluoromethyl)-5H-pyrimido[1,2-a][1,4]benzodiazepine-2-carboxylic acid

A solution of methyl 8-chloro-7-(2,6-difluorophenyl)-5-methyl-3-oxo-9-(trifluoromethyl)-SH-pyrimido[1,2-a][1,4]benzodiazepine-2-carboxylate (2.5 g, 5.02 mmol) and lithium iodide (1.45 g, 10.8 mmol) in ethyl acetate (20 mL) was stirred at 70 °C for 6 h. The reaction mixture was cooled to room temperature then concentrated *in vacuo.* The residue was diluted with saturated aqueous NaHCO₃ (30 mL) and extracted with ethyl acetate (2 × 30 mL). The aqueous layer was separated then carefully acidified with 1.0 m aqueous HCl to pH 4.0, before being extracted with dichloromethane (2 × 20 mL). The combined organic layers (CH₂Cl₂) were dried (MgSO₄), filtered and concentrated *in vacuo* to provide the title compound (1.10 g, 45 %) as a red solid. MS: 484.2 ([{³⁵Cl}M+H]⁺), 486.2 ([{³⁷Cl}M+H]⁺), ESI pos. This material was used in the following step without further purification

### c) (5S)-8-chloro-7-(2,6-difluorophenyl)-N,5-dimethyl-3-oxo-9-(trifluoromethyl)-5H-pyrimido[1,2-a][1,4]benzodiazepine-2-carboxamide

A mixture of methylamine hydrochloride (17.4 mg, 0.260 mmol) and 1-[bis(dimethylamino)methylene]-1H-1,2,3-triazolo[4,5-b]pyridinium 3-oxide hexafluorophosphate (147 mg, 0.390 mmol) in anhydrous *N*,*N*-dimethylformamide (2 mL) was stirred at 20 °C. After 30 minutes, *N*,*N*-diisopropylethylamine (0.13 mL, 0.77 mmol) and 8-chloro-7-(2,6-difluorophenyl)-5-methyl-3-oxo-9-(trifluoromethyl)-5H-pyrimido[1,2-a][1,4]benzodiazepine-2-carboxylic acid (150 mg, 0.310 mmol) were added. The reaction mixture was stirred at 20 °C for 1 h, then purified directly by preparative HPLC (Phenomenex Gemini-NX C18, 3 µm, 75x30mm, 0.225% formic acid in water / acetonitrile) to provide the racemic title compound (54 mg, 35 %). Chiral separation by SFC (Daicel Chiralcel OJ-H, 5 µm, 250x30mm, 0.1% NH₄OH in *iso*-propanol, 30% in CO₂) afforded the enantiopure (-)-title compound (11 mg, 7 %) as a white solid. MS: 497.2 ([{³⁵Cl}M+H]⁺), 499.2 ([³⁷Cl}M+H]⁺), ESI pos.

### Example 28

### (5S)-8-chloro-N-cyclopropyl-7-(2,6-difluorophenyl)-5-methyl-3-oxo-9-(trifluoromethyl)-5H-pyrimido[1,2-a][1,4]benzodiazepine-2-carboxamide

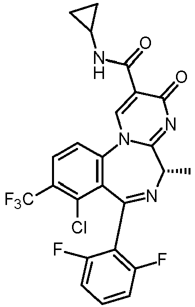

### (5S)-8-chloro-N-cyclopropyl-7-(2,6-difluorophenyl)-5-methyl-3-oxo-9-(trifluoromethyl)-5H-pyrimido[1,2-a][1,4]benzodiazepine-2-carboxamide

In analogy to experiment of example 27 c, 8-chloro-7-(2,6-difluorophenyl)-5-methyl-3-oxo-9-(trifluoromethyl)-5H-pyrimido[1,2-a][1,4]benzodiazepine-2-carboxylic acid, using cyclopropylamine instead of methylamine hydrochloride, was converted to the racemic title compound (40 mg, 25 %) after purification by preparative HPLC (Phenomenex Gemini-NX C18, 3 µm, 75x30mm, 0.225% formic acid in water / acetonitrile) followed by a second preparative HPLC (Phenomenex Synergi C18, 10 µm, 150x25mm, 0.1% trifluoroacetic acid in water / acetonitrile) and a final preparative TLC (ethyl acetate). Chiral separation by SFC (Daicel Chiralcel OJ, 10 µm, 250x30mm, 0.1% NH₄OH in methanol, 30% in CO₂) afforded the enantiopure (-)-title compound (6 mg, 4 %) as a white solid. MS: 523.2 ([{³⁵Cl}M+H]⁺), 525.2 ([{³⁷Cl}M+H]⁺), ESI pos.

### Example 29

### (5S)-8-chloro-7-(2,6-difluorophenyl)-2-(hydroxymethyl)-5-methyl-9-(trifluoromethyl)-5H-pyrimido[1,2-a][1,4]benzodiazepin-3-one

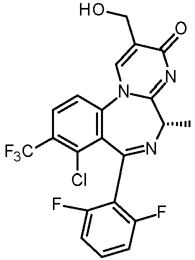

A total of 1.8 L Frankia asymbiotica CCOS 1990, was cultivated for 48 h in baffled glass Erlenmeyer flasks filled with 20% volume Medium 84 (per L deionized water; Bacto Malt Extract 10.0 g, Yeast Extract 4.0 g, D-(+)-glucose monohydrate 4.0 g, pH adjusted to 7.0) at 270 C° with agitation at 180 rpm (5 cm radius). 81 mg (5S)-8-chloro-7-(2,6-difluorophenyl)-2,5-dimethyl-9-(trifluoromethyl)-5H-pyrimido[1,2-a][1,4]benzodiazepin-3-one dissolved in 9 ml DMSO was added and the flasks incubated as described above for a further 192 h. 180 g of NaCl was added to the pooled reaction broth which was then extracted twice with an equal volume of ethyl acetate. The pooled organic phase was dried over anhydrous Na₂SO₄. The dried organic phase was evaporated under reduced pressure and the residue resuspended in 50 ml, 10% v/v acetonitrile in water. The solution was applied to a 10 g C18 cartridge which was eluted with a step gradient of acetonitrile in water. After evaporation of acetonitrile, product containing fractions were pooled and lyophilized. Final purification was by preparative SFC to give the enantiopure (-)-title compound (6.7 mg, 98 %) as a white powder after lyophilization. MS 470.1([M+H]+), ESI pos.

### Reference Compounds

### RE-A

### 9-chloro-7-(2-fluorophenyl)-5H-pyrimido[1,2-a][1,4]benzodiazepin-3-one

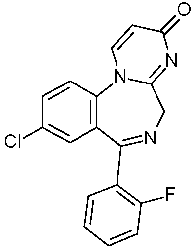

### a) 7-chloro-5-(2-fluorophenyl)-1,3-dihydro-1,4-benzodiazepine-2-thione

To a solution of 7-chloro-5-(2-fluorophenyl)-1,3-dihydro-1,4-benzodiazepin-2-one (Desalkylflurazepam, CAS# 2886-65-9, 3.00 g, 10.4 mmol) in toluene (30 mL) was added Lawesson's reagent (2.10 g, 5.19 mmol). The mixture was stirred at reflux until complete conversion as judged by LC-MS. To the mixture was added water (30 mL) followed by stirring for 2 h. The reaction mixture was filtered and concentrated *in vacuo* and the residue partitioned between toluene (25 mL) and water (25 mL). The phases were separated. The aqueous layer was extracted with toluene (2 × 20 mL). The combined organic layers were washed with brine, dried (MgSO₄), and concentrated *in vacuo.* The solid fractions were combined to give the title compound (3.62 g, quant.) as an off-white solid. MS: 304.0 ([{³⁵Cl}M+H]⁺), 306.0 ([³⁷Cl}M+H]⁺), ESI pos. The crude was used as such in the following step without further purification.

### b) 7-chloro-5-(2-fluorophenyl)-3H-1,4-benzodiazepin-2-amine

In analogy to experiment of example 1 b, 7-chloro-5-(2-fluorophenyl)-1,3-dihydro-1,4-benzodiazepine-2-thione was converted into the title compound (410 mg, 87 %) which was obtained as a light yellow solid. MS: 288.0 ([{³⁵Cl}M+H]⁺), 290.0 ([{³⁷Cl}M+H]⁺), ESI pos.

### c) 9-chloro-7-(2-fluorophenyl)-5H-pyrimido[1,2-a][1,4]benzodiazepin-3-one

In analogy to experiment of example 1 c, 7-chloro-5-(2-fluorophenyl)-3H-1,4-benzodiazepin-2-amine was converted into the title compound (31 mg, 18 %) which was obtained as an off-white solid. MS: 340.1 ([{³⁵Cl}M+H]⁺), 342.1 ([{³⁷Cl}M+H]⁺), ESI pos.

### Assay procedures

### Membrane preparation and binding assay for γ1-containing GABA_{A} subtypes

The affinity of compounds at GABA_{A} γ1 subunit-containing receptors was measured by competition for [³H]RO7239181 (67.3 Ci/mmol; Roche) binding to membranes from HEK293F cells (ThermoFisher R79007) expressing human (transiently transfected) receptors of composition α5β2γ1, α2β2γ1, α1β2γ1. For better protein expression of the α2 subunit-containing receptors, the 28 amino acid long signal peptide (Met1 to Ala28)of the human GABA_{A} α2 subunit was substituted by the 31 amino acid long signal peptide (Met1 to Ser31) of human GABA_{A} α5 subunit.

Harvested pellets from HEK293F cells expressing the different GABA_{A} receptor subtypes were resuspended in Mannitol Buffer pH 7.2-7.4 (Mannitol 0.29M, Triethylamine 10mM, Acetic acid 10mM, EDTA 1mM plus protease inhibitors (20 tablets Complete, Roche Diagnostics Cat. No. 05 056 489 001 per liter)), washed two times and then resuspended at 1:10 to 1:15 dilution in the same buffer. Cell disruption was performed by stirring the suspension in a Parr vessel #4637 at 435 psi for 15 minutes, and then the suspensions were centrifuged at 1000xg for 15 minutes at 4°C (Beckman Avanti J-HC; rotor JS-4.2). The supernatant (S1) was transferred in a 2l Schott flask and the pellet (P1) was resuspended with Mannitol Buffer up to 175ml. The resuspended pellet was transferred into a 250ml Corning centrifugal beaker and centrifuged at 1500xg for 10 minutes at 4°C (Beckman Avanti J-HC; rotor JS-4.2). The supernatant (S1) was then transferred in the 2l Schott flask and the pellet was discarded. The supernatants (S1) were centrifuged in 500ml Beckman polypropylene centrifugal beaker at 15'000xg for 30 minutes at 4°C (Beckman Avanti J-20 XP; rotor JLA-10.500). The pellet (P2) was resuspended with Mannitol Buffer 1:1 and frozen at -80°C. The supernatant (S2) was centrifuged in 100 ml Beckman polypropylene centrifugal tubes at 48000xg for 50 minutes at 4°C (Beckman Avanti J-20 XP; rotor JA-18). The supernatant (S3) was discarded and the pellet (P3) was resuspended with 1:1 Mannitol Buffer. The P2 and P3 protein concentration was determined with the BIORAD Standard assay method with bovine serum albumin as standard and measured on the NANO-Drop 1000. The membrane suspension was aliquots (500µl per tube) and stored at -80°C until required.

Membrane homogenates were resuspended and polytronised (Polytron PT1200E Kinematica AG) in Potassium Phosphate 10mM, KCl 100mM binding buffer at pH 7.4 to a final assay concentration determined with a previous experiment.

Radioligand binding assays were carried out in a volume of 200 µL (96-well plates) which contained 100 µL of cell membranes, [³H]RO7239181 at a concentration of 1.5 nM (α5β2γ1) or 20-30 nM (α1β2γ1, α2β2γ1) and the test compound in the range of [0.3-10000] × 10⁻⁹ M. Nonspecific binding was defined by 10 × 10⁻⁶ (α5β2γ1) and 30 × 10⁻⁶ M RO7239181 and typically represented less than 5% (α5β2γ1) and less than 20% (α1β2γ1, α2β2γ1) of the total binding. Assays were incubated to equilibrium for 1 hour at 4 °C and then, membranes were filtered onto unifilter (96-well white microplate with bonded GF/C filters preincubated 20-50 minutes in 0.3% Polyethylenimine) with a Filtermate 196 harvester (Packard BioScience) and washed 4 times with cold Potassium Phosphate 10mM pH 7.4, KCl 100mM binding buffer. After anhydrousing, filter-retained radioactivity was detected by liquid scintillation counting. Kᵢ values were calculated using Excel-Fit (Microsoft) and are the means of two determinations.

The compounds of the accompanying examples were tested in the above described assays, and the preferred compounds were found to possess a Kᵢ value for the displacement of [³H]RO7239181 from GABA_{A} γ1 subunit-containing receptors (e.g. α5β2γ1, α2β2γ1, α1β2γ1) of 100 nM or less. Most preferred are compounds with a Ki (nM) < 50. Representative test results, obtained by the above described assay measuring binding affinity to HEK293 cells expressing human (h) receptors, are shown in the Table 1.

### Preparation of [³H]RO7239181, 6-chloro-5-(2,6-difluorophenyl)-7-methyl-1-(tritritiomethyl)-3H-1,4-benzodiazepin-2-one

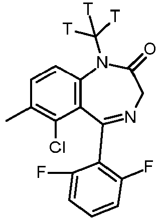

### a) 6-chloro-5-(2,6-difluorophenyl)-7-methyl-1,3-dihydro-1,4-benzodiazepin-2-one

A microwave tube was charged with 7-bromo-6-chloro-5-(2,6-difluorophenyl)-1,3-dihydro-1,4-benzodiazepin-2-one (building block A (see infra), 450 mg, 1.17 mmol), trimethylboroxine (205 mg, 228 µL, 1.63 mmol), potassium carbonate (242 mg, 1.75 mmol) and tetrakis(triphenylphosphine)palladium (0) (67.4 mg, 58.4 µmol). Degassed 1,4-dioxane (8.1 mL) and H₂O (2.7 ml) were added then the vial was capped. The suspension was reacted in microwave at 130 °C for 30 min to give complete conversion. The mixture was evaporated, treated with sat. aq. NaHCO₃ (20 mL) and extracted with EtOAc (2 x 20 mL). The organic layers were dried (Na₂SO₄), filtered and solvents were evaporated. The residue was purified by flash column chromatography (silica, 40 g, CH₂Cl₂/EtOAc in heptane 10% to 40% to 70%) to give the title compound (344 mg, 92%) as light yellow solid. MS (ESI): 321.1 ([M+H]⁺).

### b) 6-chloro-5-(2,6-difluorophenyl)-7-methyl-1-(tritritiomethyl)-3H-1,4-benzodiazepin-2-one

To a solution of [³H]methyl nosylate (1.85 GBq, 50 mCi, 0.61 µmol) in THF (200 µL) were added the *N*-desmethyl precursor 6-chloro-5-(2,6-difluorophenyl)-7-methyl-1,3-dihydro-1,4-benzodiazepin-2-one (0.43 mg, 1.34 µmol) dissolved in THF (200 µL) and 10 equivalents of sodium *tert*-butylate (0.5 M in THF, 13.4 µmol). After stirring for 4 h at room temperature the reaction mixture was treated with H₂O, evaporated, and the crude product was purified by HPLC (X-Terra Prep RP-18, 10 x 150 mm, MeCN/H₂O (containing 5% of MeCN) 40:60, 4 ml/min, 230 nm). The pure tritium-labeled compound was isolated by solid phase extraction (Sep-Pak Plus C18) and eluted from the cartridge as ethanolic solution to yield 1.6 GBq (43.2 mCi) of the target compound in > 99% radiochemical purity and a specific activity of 2.49 TBq/mmol (67.3 Ci/mmol) as determined by mass spectrometry (MS). The identity of the labeled compound was confirmed by HPLC (by co-injecting the unlabeled reference standard) and by MS.

MS: m/z = 335 [M(H)+H]⁺ (16%), 337 [M(³H)+H]⁺ (0%), 339 [M(³H₂)+H]⁺ (16%), 341 [M(³H₃)+H]⁺ (68%).

### Membrane preparation and binding assay for γ2-containing GABA_{A} subtypes

The affinity of compounds at GABA_{A} γ2 subunit-containing receptors was measured by competition for [³H]Flumazenil (81.1 Ci/mmol; Roche) binding to HEK293F cells expressing human (transiently transfected) receptors of composition α1β3γ2.

Harvested pellets from HEK293F cells expressing the different GABA_{A} γ2 receptor subtypes were resuspended in Mannitol Buffer pH 7,2 -7,4 and processed as described above for the cells expressing the GABA_{A} γ1 subunit-containing receptors.

Radioligand binding assays were carried out in a volume of 200 µL (96-well plates) which contained 100 µL of cell membranes, [³H]Flumazenil at a concentration of 1 nM and the test compound in the range of [0.1 · 10⁻³-10]×10⁻⁶ M. Nonspecific binding was defined by 10⁻⁵ M Diazepam and typically represented less than 5% of the total binding. Assays were incubated to equilibrium for 1 hour at 4 °C and harvested onto GF/C uni-filters (Packard) by filtration using a Packard harvester and washing with ice-cold wash buffer (50 mM Tris; pH 7.5). After anhydrousing, filter-retained radioactivity was detected by liquid scintillation counting. Kᵢ values were calculated using Excel-Fit (Microsoft) and are the means of two determinations.

The compounds of the accompanying examples were tested in the above described assay, and the preferred compounds were found to possess large Kᵢ value for displacement of [³H]Flumazenil from the α1β3γ2 subtype of the human GABA_{A} receptor of 100 nM or above. Most preferred are compounds with a Kᵢ α1β3γ2 (nM) > 300. In a preferred embodiment the compounds of the invention are binding selectively for the γ1 subunit-containing GABA_{A} receptors relative to γ2 subunit-containing GABA_{A} receptors. In particular, compounds of the present invention have γ2/γ1 selectivity ratio defined as "Kᵢ α1β3γ2 (nM) / Kᵢ α2β2γ1 (nM)" above 10-fold, or LogSel defined as "Log[Kᵢ α1β3γ2 (nM) / Kᵢ α2β2γ1 (nM)]" above 1. Representative test results, obtained by the above described assay measuring binding affinity to HEK293 cells expressing human (h) receptors, are shown in the Table 1 below.

**Table 1**

| **Example** | **Ki h-GABA_{A}** | **Ki h-GABA_{A}** | **Ki h-GABA_{A}** | **Ki h-GABA_{A}** | **γ2/γ1 Selectivity** | **LogSel** |
|---|---|---|---|---|---|---|
| | **α5β2γ1 (nM)** | **α2β2γ1 (nM)** | **α1β2γ1 (nM)** | **α1β3γ2 (nM)** | **Ratio** | |
| **1** | 3.8 | 57.9 | 63.4 | 716.2 | 12.4 | 1.09 |
| **2** | 1.1 | 9.7 | 8.6 | 461.8 | 47.7 | 1.68 |
| **3** | 10.3 | 111.7 | 182.8 | 2188.3 | 19.6 | 1.29 |
| **4** | 1.5 | 13.3 | 25.6 | 912.5 | 68.7 | 1.84 |
| **5** | 1.4 | 10.6 | ND | 2020.3 | 190.6 | 2.28 |
| **6** | 4.8 | 49.9 | ND | 1779.7 | 35.7 | 1.55 |
| **7** | 1.4 | 11.3 | ND | 694.6 | 61.2 | 1.79 |
| **8** | 2.0 | 37.7 | ND | 1235.2 | 32.7 | 1.51 |
| **9** | 3.1 | 28.4 | 98.1 | 5950.4 | 209.4 | 2.32 |
| **10** | 3.0 | 54.6 | 158.9 | 6122.9 | 112.1 | 2.05 |
| **11** | 4.1 | 40.1 | 166.9 | 6087.2 | 151.9 | 2.18 |
| **12** | 0.7 | 4.6 | ND | 322.4 | 70.5 | 1.85 |
| **13** | 1.0 | 11.4 | 17.6 | 607.4 | 53.3 | 1.73 |
| **14** | 2.3 | 15.6 | ND | 909.0 | 58.4 | 1.77 |
| **15** | 0.8 | 8.0 | ND | 1965.7 | 244.8 | 2.39 |
| **16** | 1.6 | 8.3 | ND | 1568.3 | 188.0 | 2.27 |
| **17** | 1.0 | 13.7 | ND | 507.4 | 36.9 | 1.57 |
| **18** | 9.7 | 60.6 | ND | 6335.8 | 104.6 | 2.02 |
| **19** | 4.9 | 24.1 | ND | 1167.7 | 48.4 | 1.68 |
| **20** | 6.6 | 100.8 | ND | 8883.1 | 88.1 | 1.95 |
| **21** | 3.4 | 23.3 | ND | 1435.8 | 61.6 | 1.79 |
| **22** | 2.1 | 15.3 | ND | 1127.2 | 73.8 | 1.87 |
| **23** | 6.8 | 74.1 | ND | 2998.3 | 40.5 | 1.61 |
| **24** | 3.0 | 30.3 | ND | 1821.1 | 60.1 | 1.78 |
| **25** | 9.4 | 47.9 | ND | 14677.8 | 306.4 | 2.49 |
| **26** | 21.6 | 75.0 | ND | >30000 | 400.1 | 2.60 |
| **27** | 6.2 | 30.8 | 67.8 | 9013.0 | 292.9 | 2.47 |
| **28** | 7.4 | 33.0 | ND | 11175.5 | 338.4 | 2.53 |
| **29** | 11.2 | 44.6 | 112.9 | 13061.5 | 292.6 | 2.47 |

### Functional expression of GABA_{A} receptors:

### Xenopus oocytes preparation

Xenopus laevis oocytes at maturation stages V-VI were used for the expression of cloned mRNA encoding GABA_{A} receptor subunits. Oocytes ready for RNA micro-injection were bought from Ecocyte, Castrop-Rauxel, Germany and stored in modified Barth's medium (composition in mM: NaCl 88, KCl 1, NaHCO₃ 2.4, HEPES 10, MgSO₄ 0.82, CaNO₃ 0.33, CaCl₂ 0.33, pH = 7.5) at 20 °C until the experiment.

### Xenopus oocytes microinjection

Oocytes were plated in 96-well plates for microinjection using the Roboinject automated instrument (MultiChannelSystems, Reutlingen, Germany). Approximately 50 nL of an aqueous solution containing the RNA transcripts for the subunits of the desired GABA_{A} receptor subtype was injected into each oocyte. RNA concentrations ranged between 20 and 200 pg/µL/subunit and were adjusted in pilot experiments to obtain GABA responses of a suitable size and a maximal effect of Flunitrazepam, Triazolam and Midazolam, reference benzodiazepine positive allosteric modulators (PAM) at the GABA_{A} receptor benzodiazepine (BZD) binding site. Oocytes were kept in modified Barth's medium (composition in mM: NaCl 88, KCl 1, NaHCO₃ 4, HEPES 10, MgSO₄ 0.82, CaNO₃ 0.33, CaCl₂ 0.33, pH = 7.5) at 20°C until the experiment.

### Electrophysiology

Electrophysiological experiments were performed using the Roboocyte instrument (MultiChannelSystems, Reutlingen, Germany) on days 3 to 5 after the micro-injection of mRNA. During the experiment the oocytes were constantly superfused by a solution containing (in mM) NaCl 90, KCl 1, HEPES 5, MgCl₂ 1, CaCl₂ 1 (pH 7.4). Oocytes were impaled by two glass microelectrodes (resistance: 0.5-0.8 MΩ) which were filled with a solution containing KCl 1M + K-acetate 1.5 M and voltage-clamped to -80 mV. The recordings were performed at room temperature using the Roboocyte two-electrode voltage clamp system (Multichannelsystem). After an initial equilibration period of 1.5 min GABA was added for 1.5 min at a concentration evoking approximately 20% of a maximal current response (EC₂₀). After another rest interval of 2.5 min GABA was again added evoking a response of similar amplitude and shape. 0.5 min after the onset of this second GABA application the test compound, at a concentration corresponding to approximatively 30-fold its Kᵢ α2β2γ1, was added while GABA was still present. Current traces were recorded at a digitization rate of 10 Hz during and shortly before and after the GABA application.

Each compound and concentration was tested on at least 3 oocytes. Different oocytes were used for different compound concentrations. The reference PAMs, Flunitrazepam, Triazolam and Midazolam, potentiated the GABA-induced current in α2β2γ1 GABA_{A} receptor subtype expressing oocytes by approximatively 60%.

### Data analysis

For the analysis, the digitized current traces of the first and second GABA response were superimposed and, if necessary, rescaled to equal maximal amplitudes. The ratio between the two responses during the time interval of test compound application was calculated point by point. The extremum of the resulting "ratio trace" was taken as the efficacy ("Fold increase") of the compound expressed as "% modulation of GABA EC₂₀" (100* (Fold increase-1)).

The results are shown in Table 2.

**Table 2**

| **Example** | **Ki h-GABA_{A} α2β2γ1 (nM)** | **Fold increase h-GABA-A α2β2γ1 oocyte @ 30-fold Ki** | **Efficacy (GABA)%** |
|---|---|---|---|
| **1** | 57.9 | 1.92 | 92 |
| **2** | 9.7 | 1.88 | 88 |
| **3** | 111.7 | 2.92 | 192 |
| **4** | 13.3 | 2.05 | 105 |
| **5** | 10.6 | 1.78 | 78 |
| **6** | 49.9 | 2.21 | 121 |
| **7** | 11.3 | 1.53 | 53 |
| **8** | 37.7 | 2.15 | 115 |
| **9** | 28.4 | 1.99 | 99 |
| **10** | 54.6 | 2.64 | 164 |
| **11** | 40.1 | 1.7 | 70 |
| **12** | 4.6 | 1.65 | 65 |
| **13** | 11.4 | 1.7 | 70 |
| **14** | 15.6 | 1.66 | 66 |
| **15** | 8.0 | 1.69 | 69 |
| **16** | 8.3 | 1.71 | 71 |
| **17** | 13.7 | 1.79 | 79 |
| **18** | 60.6 | 1.89 | 89 |
| **19** | 24.1 | 2.04 | 104 |
| **20** | 100.8 | 2.13 | 113 |
| **21** | 23.3 | 2.13 | 113 |
| **22** | 15.3 | 2.13 | 113 |
| **23** | 74.1 | 2.07 | 107 |
| **24** | 30.3 | 2.20 | 120 |
| **25** | 47.9 | 1.81 | 81 |
| **26** | 75.0 | 2.13 | 113 |
| **27** | 30.8 | 2.13 | 113 |
| **28** | 33.0 | 1.35 | 35 |
| **29** | 44.6 | ND | - |

### Reference compounds

Benzodiazepines reference compounds (classical marketed benzodiazepines) and reference pyrimido[1,2-a][1,4]benzodiazepin-3-ones listed below were tested for their affinity towards the GABA_{A} receptor α1β2γ1 and α2β2γ1subtypes as well as in the GABA_{A} receptor α1β3γ2 subtype. The results are shown in Table 3.

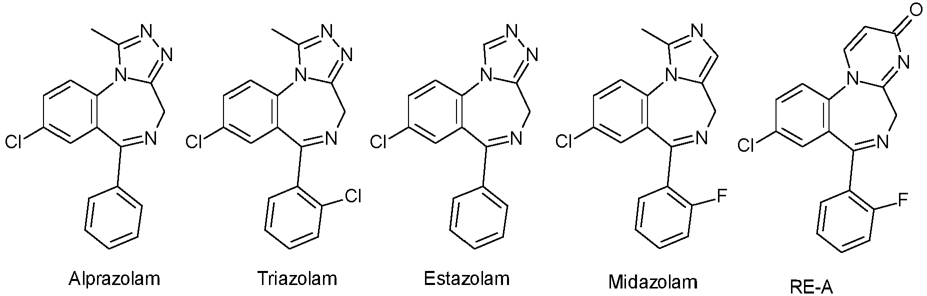

**Table 3**

| **Example** | **Ki h-GABA_{A}** | **Ki h-GABA_{A}** | **Ki h-GABA_{A}** | **γ2/γ1 Selectivity** | **LogSel** |
|---|---|---|---|---|---|
| | **α1β2γ1 (nM)** | **α2β2γ1 (nM)** | **α1β3γ2 (nM)** | **Ratio** | |
| **Alprazolam** | 5923 | 3945 | 19.6 | 0.0050 | -2.3 |
| **Triazolam** | 44.2 | 46.2 | 1.5 | 0.032 | -1.5 |
| **Midazolam** | 1153.2 | 737.7 | 5.0 | 0.0068 | -2.2 |
| **RE-A** | ND | 122.7 | 27.8 | 0.23 | -0.64 |

The preparation of reference example RE-A is described above.

### Preparation of pharmaceutical compositions comprising compounds of the invention

Tablets comprising compounds of formula (I) are manufactured as follows:

| **Ingredient** | **mg/tablet** | | | |
|---|---|---|---|---|
| | 5 | 25 | 100 | 500 |
| Compound of formula I | 5 | 25 | 100 | 500 |
| Lactose Anhydrous DTG | 125 | 105 | 30 | 150 |
| Sta-Rx 1500 | 6 | 6 | 6 | 60 |
| Microcrystalline Cellulose | 30 | 30 | 30 | 450 |
| Magnesium Stearate | 1 | 1 | 1 | 1 |
| Total | 167 | 167 | 167 | 831 |

### Manufacturing Procedure

1. Mix ingredients 1, 2, 3 and 4 and granulate with purified water.
2. Dry the granules at 50°C.
3. Pass the granules through suitable milling equipment.
4. Add ingredient 5 and mix for three minutes; compress on a suitable press.

Capsules comprising compounds of formula (I) are manufactured as follows:

| **Ingredient** | **mg/capsule** | | | |
|---|---|---|---|---|
| | 5 | 25 | 100 | 500 |
| Compound of formula I | 5 | 25 | 100 | 500 |
| Hydrous Lactose | 159 | 123 | 148 | - |
| Corn Starch | 25 | 35 | 40 | 70 |
| Talk | 10 | 15 | 10 | 25 |
| Magnesium Stearate | 1 | 2 | 2 | 5 |
| Total | 200 | 200 | 300 | 600 |

### Manufacturing Procedure

1. Mix ingredients 1, 2 and 3 in a suitable mixer for 30 minutes.
2. Add ingredients 4 and 5 and mix for 3 minutes.
3. Fill into a suitable capsule.

A compound of formula I lactose and corn starch are firstly mixed in a mixer and then in a comminuting machine. The mixture is returned to the mixer; the talc is added thereto and mixed thoapproximatively. The mixture is filled by machine into suitable capsules, e.g. hard gelatin capsules.

Injection solutions comprising compounds of formula (I) are manufactured as follows:

| **Ingredient** | **mg/injection solution.** |
|---|---|
| Compound of formula I | 3 |
| Polyethylene Glycol 400 | 150 |
| acetic acid | q.s. ad pH 5.0 |
| water for injection solutions | ad 1.0 ml |

## Claims

1. A compound of formula (I) 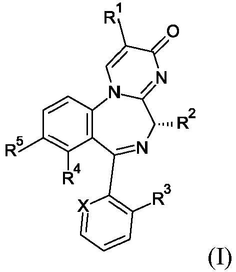 or a pharmaceutically acceptable salt thereof, wherein:
X is C-R⁶ or N;
R¹ is selected from hydrogen, halogen, cyano, C₁-C₆-alkyl, halo-C₁-C₆-alkyl, hydroxy-C₁-C₆-alkyl, amino-C₁-C₆-alkyl, C₁-C₆-alkoxy-C₁-C₆-alkyl, C₁-C₆-alkoxy-C₂-C₆-alkenyl, C₁-C₆-alkoxy, halo-C₁-C₆-alkoxy, carbamoyl, C₁-C₆-alkyl-NH-C(O)-, (C₁-C₆-alkyl)₂N-C(O)-, and C₃-C₁₀-cycloalkyl-NH-C(O)-;
R² is selected from hydrogen, C₁-C₆-alkyl, halo-C₁-C₆-alkyl, C₁-C₆-alkoxy, and halo-C₁-C₆-alkoxy;
R³ and R⁶ are each independently selected from hydrogen, C₁-C₆-alkyl, halo-C₁-C₆-alkyl, C₁-C₆-alkoxy, halo-C₁-C₆-alkoxy, halogen and cyano;
R⁴ is selected from chloro and bromo; and
R⁵ is selected from hydrogen, C₁-C₆-alkyl, halo-C₁-C₆-alkyl, C₁-C₆-alkoxy, halo-C₁-C₆-alkoxy, halogen and cyano.

2. The compound of formula (I) according to claim 1, or a pharmaceutically acceptable salt thereof, wherein:
R¹ is selected from hydrogen, halogen, C₁-C₆-alkyl, hydroxy-C₁-C₆-alkyl, C₁-C₆-alkoxy-C₁-C₆-alkyl, C₁-C₆-alkoxy-C₂-C₆-alkenyl, C₁-C₆-alkyl-NH-C(O)-, and C₃-C₁₀-cycloalkyl-NH-C(O)-;
R² is selected from hydrogen and C₁-C₆-alkyl;
R³ is halogen;
R⁵ is selected from halogen, C₁-C₆-alkyl and halo-C₁-C₆-alkyl; and
R⁶ is selected from hydrogen and halogen.

3. The compound of formula (I) according to claim 2, or a pharmaceutically acceptable salt thereof, wherein:
R¹ is selected from C₁-C₆-alkyl and C₁-C₆-alkyl-NH-C(O)-;
R² is C₁-C₆-alkyl;
R³ and R⁶ are both halogen;
R⁴ is chloro; and
R⁵ is halo-C₁-C₆-alkyl.

4. The compound of formula (I) according to claim 3, or a pharmaceutically acceptable salt thereof, wherein:
R¹ is selected from methyl and methyl-NH-C(O)-;
R² is methyl;
R³ and R⁶ are both fluoro;
R⁴ is chloro; and
R⁵ is CF₃.

5. The compound of formula (I) according to claim 1, or a pharmaceutically acceptable salt thereof, wherein said compound of formula (I) is selected from:
8,9-dichloro-7-(2-fluorophenyl)-5H-pyrimido[1,2-a][1,4]benzodiazepin-3-one;
8,9-dichloro-7-(2,6-difluorophenyl)-5H-pyrimido[1,2-a][1,4]benzodiazepin-3-one;
8,9-dichloro-7-(3-fluoro-2-pyridyl)-5H-pyrimido[1,2-a][1,4]benzodiazepin-3-one;
(5S)-8,9-dichloro-7-(2,6-difluorophenyl)-5-methyl-5H-pyrimido[1,2-a][1,4]benzodiazepin-3-one;
2-bromo-8,9-dichloro-7-(2,6-difluorophenyl)-5H-pyrimido[1,2-a][1,4]benzodiazepin-3-one;
(5*S*)-8-bromo-9-chloro-7-(2,6-difluorophenyl)-5-methyl-5H-pyrimido[1,2-a][1,4]benzodiazepin-3-one;
(5*S*)-8,9-dichloro-7-(2,6-difluorophenyl)-2,5-dimethyl-5H-pyrimido[1,2-a][1,4]benzodiazepin-3-one;
(5*S*)-8-chloro-7-(2,6-difluorophenyl)-2,5,9-trimethyl-5H-pyrimido[1,2-a][1,4]benzodiazepin-3-one;
(5*S*)-8-chloro-7-(2,6-difluorophenyl)-2,5-dimethyl-9-(trifluoromethyl)-5H-pyrimido[1,2-a][1,4]benzodiazepin-3-one;
(5*S*)-8-chloro-7-(2,6-difluorophenyl)-5-methyl-9-(trifluoromethyl)-5H-pyrimido[1,2-a][1,4]benzodiazepin-3-one;
(5*S*)-8-chloro-7-(2,6-difluorophenyl)-2-ethyl-5-methyl-9-(trifluoromethyl)-5H-pyrimido[1,2-a][1,4]benzodiazepin-3-one;
9-bromo-8-chloro-7-(2,6-difluorophenyl)-5H-pyrimido[1,2-a][1,4]benzodiazepin-3-one;
(5*S*)-9-bromo-8-chloro-7-(2,6-difluorophenyl)-5-methyl-5H-pyrimido[1,2-a][1,4]benzodiazepin-3-one;
(5*S*)-8,9-dichloro-7-(2,6-difluorophenyl)-2-ethyl-5-methyl-5H-pyrimido[1,2-a][1,4]benzodiazepin-3-one;
2,9-dibromo-8-chloro-7-(2,6-difluorophenyl)-5H-pyrimido[1,2-a][1,4]benzodiazepin-3-one;
(5*S*)-2,9-dibromo-8-chloro-7-(2,6-difluorophenyl)-5-methyl-5H-pyrimido[1,2-a][1,4]benzodiazepin-3-one;
8-bromo-9-chloro-7-(2,6-difluorophenyl)-5H-pyrimido[1,2-a][1,4]benzodiazepin-3-one;
(5*S*)-8-chloro-7-(2,6-difluorophenyl)-2-[(*E*)-2-ethoxyvinyl]-5-methyl-9-(trifluoromethyl)-5H-pyrimido[1,2-a][1,4]benzodiazepin-3-one;
(5S)-8,9-dichloro-7-(2,6-difluorophenyl)-2-[(*E*)-2-ethoxyvinyl]-5-methyl-5H-pyrimido[1,2-a][1,4]benzodiazepin-3-one;
(5*S*)-8-chloro-7-(2,6-difluorophenyl)-2-(2-ethoxyethyl)-5-methyl-9-(trifluoromethyl)-5H-pyrimido[1,2-a][1,4]benzodiazepin-3-one;
(5*S*)-8,9-dichloro-7-(2,6-difluorophenyl)-2-(2-ethoxyethyl)-5-methyl-5H-pyrimido[1,2-a][1,4]benzodiazepin-3-one;
8,9-dichloro-7-(2,6-difluorophenyl)-2-[(*E*)-2-ethoxyvinyl]-5H-pyrimido[1,2-a][1,4]benzodiazepin-3-one;
(5*S*)-2,8-dibromo-9-chloro-7-(2,6-difluorophenyl)-5-methyl-5H-pyrimido[1,2-a][1,4]benzodiazepin-3-one;
8,9-dichloro-7-(2,6-difluorophenyl)-2-(2-ethoxyethyl)-5H-pyrimido[1,2-a][1,4]benzodiazepin-3-one;
8-chloro-7-(2,6-difluorophenyl)-2-[(*E*)-2-ethoxyvinyl]-9-(trifluoromethyl)-5H-pyrimido[1,2-a][1,4]benzodiazepin-3-one;
8-chloro-7-(2,6-difluorophenyl)-2-(2-ethoxyethyl)-9-(trifluoromethyl)-5H-pyrimido[1,2-a][1,4]benzodiazepin-3-one;
(5*S*)-8-chloro-7-(2,6-difluorophenyl)-N,5-dimethyl-3-oxo-9-(trifluoromethyl)-5H-pyrimido[1,2-a][1,4]benzodiazepine-2-carboxamide;
(5*S*)-8-chloro-N-cyclopropyl-7-(2,6-difluorophenyl)-5-methyl-3-oxo-9-(trifluoromethyl)-5H-pyrimido[1,2-a][1,4]benzodiazepine-2-carboxamide; and
(5*S*)-8-chloro-7-(2,6-difluorophenyl)-2-(hydroxymethyl)-5-methyl-9-(trifluoromethyl)-5H-pyrimido[1,2-a][1,4]benzodiazepin-3-one.

6. The compound of formula (I) according to claim 5, or a pharmaceutically acceptable salt thereof, wherein said compound of formula (I) is selected from:
(5*S*)-8-chloro-7-(2,6-difluorophenyl)-2,5-dimethyl-9-(trifluoromethyl)-5H-pyrimido[1,2-a][1,4]benzodiazepin-3-one; and
(5*S*)-8-chloro-7-(2,6-difluorophenyl)-*N*,5-dimethyl-3-oxo-9-(trifluoromethyl)-5H-pyrimido[1,2-a][1,4]benzodiazepine-2-carboxamide.

7. A compound of formula (I) according to any one of claims 1 to 6, or a pharmaceutically acceptable salt thereof, for use as therapeutically active substance.

8. A pharmaceutical composition comprising a compound of formula (I) according to any one of claims 1 to 6, or a pharmaceutically acceptable salt thereof, and a therapeutically inert carrier.

9. A compound of formula (I) according to any of claims 1 to 6, or a pharmaceutically acceptable salt thereof, or a pharmaceutical composition according to claim 8, for use in treating or preventing acute neurological disorders, chronic neurological disorders and/or cognitive disorders.

## Patentansprüche

1. Verbindung der Formel (I): 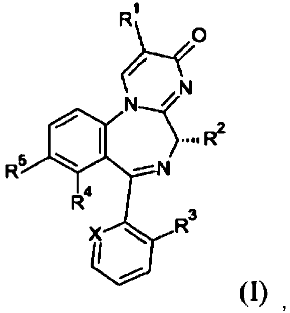 oder ein pharmazeutisch unbedenkliches Salz davon, wobei:
X C-R⁶ oder N ist;
R¹ aus Wasserstoff, Halogen, Cyano, C₁-C₆-Alkyl, Halo-C₁-C₆-alkyl, Hydroxy-C₁-C₆-alkyl, Amino-C₁-C₆-alkyl, C₁-C₆-Alkoxy-C₁-C₆-alkyl, C₁-C₆-Alkoxy-C₂-C₆-alkenyl, C₁-C₆-Alkoxy, Halo-C₁-C₆-alkoxy, Carbamoyl, C₁-C₆-Alkyl-NH-C(O)-, (C₁-C₆-Alkyl)₂N-C(O)- und C₃-C₁₀-Cycloalkyl-NH-C(O)- ausgewählt ist;
R² aus Wasserstoff, C₁-C₆-Alkyl, Halo-C₁-C₆-alkyl, C₁-C₆-Alkoxy und Halo-C₁-C₆-alkoxy ausgewählt ist;
R³ und R⁶ jeweils unabhängig aus Wasserstoff, C₁-C₆-Alkyl, Halo-C₁-C₆-alkyl, C₁-C₆-Alkoxy, Halo-C₁-C₆-alkoxy, Halogen und Cyano ausgewählt sind;
R⁴ aus Chlor und Brom ausgewählt ist; und
R⁵ aus Wasserstoff, C₁-C₆-Alkyl, Halo-C₁-C₆-alkyl, C₁-C₆-Alkoxy, Halo-C₁-C₆-alkoxy, Halogen und Cyano ausgewählt ist.

2. Verbindung der Formel (I) nach Anspruch 1 oder ein pharmazeutisch unbedenkliches Salz davon, wobei:
R¹ aus Wasserstoff, Halogen, Cyano, C₁-C₆-Alkyl, Hydroxy-C₁-C₆-alkyl, C₁-C₆-Alkoxy-C₁-C₆-alkyl, C₁-C₆-Alkoxy-C₂-C₆-alkenyl, C₁-C₆-Alkyl-NH-C(O)- und C₃-C₁₀-Cycloalkyl-NH-C(O)- ausgewählt ist;
R² aus Wasserstoff und C₁-C₆-Alkyl ausgewählt ist;
R³ Halogen ist;
R⁵ aus Halogen, C₁-C₆-Alkyl und Halo-C₁-C₆-alkyl ausgewählt ist; und
R⁶ aus Wasserstoff und Halogen ausgewählt ist.

3. Verbindung der Formel (I) nach Anspruch 2 oder ein pharmazeutisch unbedenkliches Salz davon, wobei:
R¹ aus C₁-C₆-Alkyl und C₁-C₆-Alkyl-NH-C(O)- ausgewählt ist;
R² C₁-C₆-Alkyl ist;
R³ und R⁶ beide Halogen sind;
R⁴ Chlor ist; und
R⁵ Halo-C₁-C₆-alkyl ist.

4. Verbindung der Formel (I) nach Anspruch 3 oder ein pharmazeutisch unbedenkliches Salz davon, wobei:
R¹ aus Methyl und Methyl-NH-C(O)- ausgewählt ist;
R² Methyl ist;
R³ und R⁶ beide Fluor sind;
R⁴ Chlor ist; und
R⁵ CF₃ ist.

5. Verbindung der Formel (I) nach Anspruch 1 oder ein pharmazeutisch unbedenkliches Salz davon, wobei die Verbindung der Formel (I) ausgewählt ist aus:
8,9-Dichlor-7-(2-fluorphenyl)-5H-pyrimido[1,2-a][1,4]benzodiazepin-3-on;
8,9-Dichlor-7-(2,6-difluorphenyl)-5H-pyrimido[1,2-a][1,4]benzodiazepin-3-on;
8,9-Dichlor-7-(3-fluor-2-pyridyl)-5H-pyrimido[1,2-a][1,4]benzodiazepin-3-on;
(5*S*)-8,9-Dichlor-7-(2,6-difluorphenyl)-5-methyl-5H-pyrimido[1,2-a][1,4]benzodiazepin-3-on;
2-Brom-8,9-dichlor-7-(2,6-difluorphenyl)-5H-pyrimido[1,2-a][1,4]benzodiazepin-3-on;
(5*S*)-8-Brom-9-chlor-7-(2,6-difluorphenyl)-5-methyl-5H-pyrimido[1,2-a][1,4]benzodiazepin-3-on;
(5*S*)-8,9-Dichlor-7-(2,6-difluorphenyl)-2,5-dimethyl-5H-pyrimido[1,2-a][1,4]benzodiazepin-3-on;
(5*S*)-8-Chlor-7-(2,6-difluorphenyl) -2,5,9-trimethyl-5H-pyrimido[1,2-a][1,4]benzodiazepin-3-on;
(5*S*)-8-Chlor-7-(2,6-difluorphenyl)-2,5-dimethyl-9-(trifluormethyl)-5H-pyrimido[1,2-a][1,4]benzodiazepin-3-on;
(5*S*)-8-Chlor-7-(2,6-difluorphenyl)-5-methyl-9-(trifluormethyl)-5H-pyrimido[1,2-a][1,4]benzodiazepin-3-on;
(5*S*)-8-Chlor-7-(2,6-difluorphenyl)-2-ethyl-5-methyl-9-(trifluormethyl)-5H-pyrimido[1,2-a][1,4]benzodiazepin-3-on;
9-Brom-8-chlor-7-(2,6-difluorphenyl)-5H-pyrimido[1,2-a][1,4]benzodiazepin-3-on;
(5*S*)-9-Brom-8-chlor-7-(2,6-difluorphenyl)-5-methyl-5H-pyrimido[1,2-a][1,4]benzodiazepin-3-on;
(5*S*)-8,9-Dichlor-7-(2,6-difluorphenyl)-2-ethyl-5-methyl-5H-pyrimido[1,2-a][1,4]benzodiazepin-3-on;
2,9-Dibrom-8-chlor-7-(2,6-difluorphenyl)-5H-pyrimido[1,2-a][1,4]benzodiazepin-3-on;
(5S)-2,9-Dibrom-8-chlor-7-(2,6-difluorphenyl)-5-methyl-5H-pyrimido[1,2-a][1,4]benzodiazepin-3-on;
8-Brom-9-chlor-7-(2,6-difluorphenyl)-5H-pyrimido[1,2-a][1,4]benzodiazepin-3-on;
(5*S*)-8-Chlor-7-(2,6-difluorphenyl)-2-[(*E*)-2-ethoxyvinyl]-5-methyl-9-(trifluormethyl)-5H-pyrimido[1,2-a][1,4]benzodiazepin-3-on;
(5*S*)-8,9-Dichlor-7-(2,6-difluorphenyl)-2-[(*E*)-2-ethoxyvinyl]-5-methyl-5H-pyrimido[1,2-a][1,4]benzodiazepin-3-on;
(5*S*)-8-Chlor-7-(2,6-difluorphenyl)-2-(2-ethoxyethyl)-5-methyl-9-(trifluormethyl)-5H-pyrimido[1,2-a][1,4]benzodiazepin-3-on;
(5*S*)-8,9-Dichlor-7-(2,6-difluorphenyl)-2-(2-ethoxyethyl)-5-methyl-5H-pyrimido[1,2-a][1,4]benzodiazepin-3-on;
8,9-Dichlor-7-(2,6-difluorphenyl)-2-[(*E*)-2-ethoxyvinyl]-5H-pyrimido[1,2-a][1,4]benzodiazepin-3-on;
(5*S*)-2,8-Dibrom-9-chlor-7-(2,6-difluorphenyl)-5-methyl-5H-pyrimido[1,2-a][1,4]benzodiazepin-3-on;
8,9-Dichlor-7-(2,6-difluorphenyl)-2-(2-ethoxyethyl)-5H-pyrimido[1,2-a][1,4]benzodiazepin-3-on;
8-Chlor-7-(2,6-difluorphenyl)-2-[(*E*)-2-ethoxyvinyl]-9-(trifluormethyl)-5H-pyrimido[1,2-a][1,4]benzodiazepin-3-on;
8-Chlor-7-(2,6-difluorphenyl)-2-(2-ethoxyethyl)-9-(trifluormethyl)-5H-pyrimido[1,2-a][1,4]benzodiazepin-3-on;
(5*S*)-8-Chlor-7-(2,6-difluorphenyl)-N,5-dimethyl-3-oxo-9-(trifluormethyl)-5H-pyrimido[1,2-a][1,4]benzodiazepin-2-carboxamid;
(5*S*)-8-Chlor-N-cyclopropyl-7-(2,6-difluorphenyl)-5-methyl-3-oxo-9-(trifluormethyl)-5H-pyrimido[1,2-a][1,4]benzodiazepin-2-carboxamid; und
(5*S*)-8-Chlor-7-(2,6-difluorphenyl)-2-(hydroxymethyl)-5-methyl-9-(trifluormethyl)-5H-pyrimido[1,2-a][1,4]benzodiazepin-3-on.

6. Verbindung der Formel (I) nach Anspruch 5 oder ein pharmazeutisch unbedenkliches Salz davon, wobei die Verbindung der Formel (I) ausgewählt ist aus:
(5*S*)-8-Chlor-7-(2,6-difluorphenyl)-2,5-dimethyl-9-(trifluormethyl)-5H-pyrimido[1,2-a][1,4]benzodiazepin-3-on; und
(5*S*)-8-Chlor-7-(2,6-difluorphenyl)-N,5-dimethyl-3-oxo-9-(trifluormethyl)-5H-pyrimido[1,2-a][1,4]benzodiazepin-2-carboxamid.

7. Verbindung der Formel (I) nach einem der Ansprüche 1 bis 6 oder ein pharmazeutisch unbedenkliches Salz davon zur Verwendung als eine therapeutisch aktive Substanz.

8. Pharmazeutische Zusammensetzung, umfassend eine Verbindung der Formel (I) nach einem der Ansprüche 1 bis 6 oder ein pharmazeutisch unbedenkliches Salz davon und einen therapeutisch inerten Trägerstoff.

9. Verbindung der Formel (I) nach einem der Ansprüche 1 bis 6 oder ein pharmazeutisch unbedenkliches Salz davon oder pharmazeutische Zusammensetzung nach Anspruch 8 zur Verwendung bei der Behandlung oder Prävention von akuten neurologischen Störungen, chronischen neurologischen Störungen und/oder kognitiven Störungen.

## Revendications

1. Composé de formule (I) 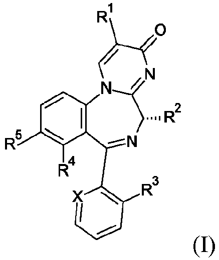 ou sel pharmaceutiquement acceptable de celui-ci, dans lequel :
X représente C-R⁶ ou N ;
R¹ est choisi parmi hydrogène, halogène, cyano, alkyle en C₁-C₆, haloalkyle en C₁-C₆, hydroxyalkyle en C₁-C₆, aminoalkyle en C₁-C₆, alcoxy en C₁-C₆-alkyle en C₁-C₆, alcoxy en C₁-C₆-alcényle en C₂-C₆, alcoxy en C₁-C₆, haloalcoxy en C₁-C₆, carbamoyle, alkyle en C₁-C₆-NH-C(O)-, (alkyle en C₁-C₆)₂N-C(O)- et cycloalkyle en C₃-C₁₀-NH-C(O)- ;
R² est choisi parmi hydrogène, alkyle en C₁-C₆, haloalkyle en C₁-C₆, alcoxy en C₁-C₆ et haloalcoxy en C₁-C₆ ;
R³ et R⁶ sont chacun indépendamment choisis parmi hydrogène, alkyle en C₁-C₆, haloalkyle en C₁-C₆, alcoxy en C₁-C₆, haloalcoxy en C₁-C₆, halogène et cyano ;
R⁴ est choisi parmi chloro et bromo ; et
R⁵ est choisi parmi hydrogène, alkyle en C₁-C₆, haloalkyle en C₁-C₆, alcoxy en C₁-C₆, haloalcoxy en C₁-C₆, halogène et cyano.

2. Composé de formule (I) selon la revendication 1, ou sel pharmaceutiquement acceptable de celui-ci, dans lequel :
R¹ est choisi parmi hydrogène, halogène, alkyle en C₁-C₆, hydroxyalkyle en C₁-C₆, alcoxy en C₁-C₆-alkyle en C₁-C₆, alcoxy en C₁-C₆-alcényle en C₂-C₆, alkyle en C₁-C₆-NH-C(O)- et cycloalkyle en C₃-C₁₀-NH-C(O)- ;
R² est choisi parmi hydrogène et alkyle en C₁-C₆ ;
R³ représente halogène ;
R⁵ est choisi parmi halogène, alkyle en C₁-C₆ et haloalkyle en C₁-C_{6 ;} et
R⁶ est choisi parmi hydrogène et halogène.

3. Composé de formule (I) selon la revendication 2, ou sel pharmaceutiquement acceptable de celui-ci, dans lequel :
R¹ est choisi parmi alkyle en C₁-C₆ et alkyle en C₁-C₆-NH-C(O)- ;
R² représente alkyle en C₁-C_{6 ;}
R³ et R⁶ représentent tous deux halogène ;
R⁴ représente chloro ; et
R⁵ représente haloalkyle en C₁-C₆.

4. Composé de formule (I) selon la revendication 3, ou sel pharmaceutiquement acceptable de celui-ci, dans lequel :
R¹ est choisi parmi méthyle et méthyle-NH-C(O)- ;
R² représente méthyle ;
R³ et R⁶ représentent tous deux fluoro ;
R⁴ représente chloro ; et
R⁵ représente CF₃.

5. Composé de formule (I) selon la revendication 1, ou sel pharmaceutiquement acceptable de celui-ci, dans lequel ledit composé de formule (I) est choisi parmi :
8,9-dichloro-7-(2-fluorophényl)-5H-pyrimido[1,2-a][1,4]benzodiazépin-3-one ;
8,9-dichloro-7-(2,6-difluorophényl)-5H-pyrimido[1,2-a][1,4]benzodiazépin-3-one ;
8,9-dichloro-7-(3-fluoro-2-pyridyl)-5H-pyrimido[1,2-a][1,4]benzodiazépin-3-one ;
(5*S*)-8,9-dichloro-7-(2,6-difluorophényl)-5-méthyl-5H-pyrimido[1,2-a][1,4]benzodiazépin-3-one ;
2-bromo-8,9-dichloro-7-(2,6-difluorophényl)-5H-pyrimido[1,2-a][1,4]benzodiazépin-3-one ;
(5*S*)-8-bromo-9-chloro-7-(2,6-difluorophényl)-5-méthyl-5H-pyrimido[1,2-a][1,4]benzodiazépin-3-one ;
(5*S*)-8,9-dichloro-7-(2,6-difluorophényl)-2,5-diméthyl-5H-pyrimido[1,2-a][1,4]benzodiazépin-3-one ;
(5*S*)-8-chloro-7-(2,6-difluorophényl)-2,5,9-triméthyl-5H-pyrimido[1,2-a][1,4]benzodiazépin-3-one ;
(5*S*)-8-chloro-7-(2,6-difluorophényl)-2,5-diméthyl-9-(trifluorométhyl)-5H-pyrimido[1,2-a][1,4]benzodiazépin-3-one ;
(5*S*)-8-chloro-7-(2,6-difluorophényl)-5-méthyl-9-(trifluorométhyl)-5H-pyrimido[1,2-a][1,4]benzodiazépin-3-one ;
(5*S*)-8-chloro-7-(2,6-difluorophényl)-2-éthyl-5-méthyl-9-(trifluorométhyl)-5H-pyrimido[1,2-a][1,4]benzodiazépin-3-one ;
9-bromo-8-chloro-7-(2,6-difluorophényl)-5H-pyrimido[1,2-a][1,4]benzodiazépin-3-one ;
(5*S*)-9-bromo-8-chloro-7-(2,6-difluorophényl)-5-méthyl-5H-pyrimido[1,2-a][1,4]benzodiazépin-3-one ;
(5*S*)-8,9-dichloro-7-(2,6-difluorophényl)-2-éthyl-5-méthyl-5H-pyrimido[1,2-a][1,4]benzodiazépin-3-one ;
2,9-dibromo-8-chloro-7-(2,6-difluorophényl)-5H-pyrimido[1,2-a][1,4]benzodiazépin-3-one ;
(5*S*)-2,9-dibromo-8-chloro-7-(2,6-difluorophényl)-5-méthyl-5H-pyrimido[1,2-a][1,4]benzodiazépin-3-one ;
8-bromo-9-chloro-7-(2,6-difluorophényl)-5H-pyrimido[1,2-a][1,4]benzodiazépin-3-one ;
(5*S*)-8-chloro-7-(2,6-difluorophényl)-2-[(*E*)-2-éthoxyvinyl]-5-méthyl-9-(trifluorométhyl)-5H-pyrimido[1,2-a][1,4]benzodiazépin-3-one ;
(5*S*)-8,9-dichloro-7-(2,6-difluorophényl)-2-[(*E*)-2-éthoxyvinyl]-5-méthyl-5H-pyrimido[1,2-a][1,4]benzodiazépin-3-one ;
(5*S*)-8-chloro-7-(2,6-difluorophényl)-2-(2-éthoxyéthyl)-5-méthyl-9-(trifluorométhyl)-5H-pyrimido[1,2-a][1,4]benzodiazépin-3-one ;
(5*S*)-8,9-dichloro-7-(2,6-difluorophényl)-2-(2-éthoxyéthyl)-5-méthyl-5H-pyrimido[1,2-a][1,4]benzodiazépin-3-one ;
8,9-dichloro-7-(2,6-difluorophényl)-2-[(*E*)-2-éthoxyvinyl]-5H-pyrimido[1,2-a][1,4]benzodiazépin-3-one ;
(5*S*)-2,8-dibromo-9-chloro-7-(2,6-difluorophényl)-5-méthyl-5H-pyrimido[1,2-a][1,4]benzodiazépin-3-one ;
8,9-dichloro-7-(2,6-difluorophényl)-2-(2-éthoxyéthyl)-5H-pyrimido[1,2-a][1,4]benzodiazépin-3-one ;
8-chloro-7-(2,6-difluorophényl)-2-[(*E*)-2-éthoxyvinyl]-9-(trifluorométhyl)-5H-pyrimido[1,2-a][1,4]benzodiazépin-3-one ; et
8-chloro-7-(2,6-difluorophényl)-2-(2-éthoxyéthyl)-9-(trifluorométhyl)-5H-pyrimido[1,2-a][1,4]benzodiazépin-3-one ;
(5*S*)-8-chloro-7-(2,6-difluorophényl)-N,5-diméthyl-3-oxo-9-(trifluorométhyl)-5H-pyrimido[1,2-a][1,4]benzodiazépine-2-carboxamide ;
(5*S*)-8-chloro-N-cyclopropyl-7-(2,6-difluorophényl)-5-méthyl-3-oxo-9-(trifluorométhyl)-5H-pyrimido[1,2-a][1,4]benzodiazépine-2-carboxamide ; et
(5*S*)-8-chloro-7-(2,6-difluorophényl)-2-(hydroxyméthyl)-5-méthyl-9-(trifluorométhyl)-5H-pyrimido[1,2-a][1,4]benzodiazépin-3-one.

6. Composé de formule (I) selon la revendication 5, ou sel pharmaceutiquement acceptable de celui-ci, dans lequel ledit composé de formule (I) est choisi parmi :
(5*S*)-8-chloro-7-(2,6-difluorophényl)-2,5-diméthyl-9-(trifluorométhyl)-5H-pyrimido[1,2-a][1,4]benzodiazépin-3-one ; et
(5*S*)-8-chloro-7-(2,6-difluorophényl)-*N*,5-diméthyl-3-oxo-9-(trifluorométhyl)-5H-pyrimido[1,2-a][1,4]benzodiazépine-2-carboxamide.

7. Composé de formule (I) selon l'une quelconque des revendications 1 à 6, ou sel pharmaceutiquement acceptable de celui-ci, pour utilisation en tant que substance thérapeutiquement active.

8. Composition pharmaceutique comprenant un composé de formule (I) selon l'une quelconque des revendications 1 à 6, ou un sel pharmaceutiquement acceptable de celui-ci, et un véhicule thérapeutiquement inerte.

9. Composé de formule (I) selon l'une quelconque des revendications 1 à 6, ou sel pharmaceutiquement acceptable de celui-ci, ou composition pharmaceutique selon la revendication 8, pour utilisation dans le traitement ou la prévention de troubles neurologiques aigus, de troubles neurologiques chroniques et/ou de troubles cognitifs.
